# EUROPEAN PATENT APPLICATION

(11) **EP 3 865 486 A1**
(43) Date of publication of application: **18.08.2021**
(21) Application number: 20157271.6
(22) Date of filing: 13.02.2020
(51) Int. Cl.: C07D 487/22, C09K 11/06, H05B 33/14

(54) **HETEROCYCLIC COMPOUND AND AN ORGANIC ELECTROLUMINESCENCE DEVICE COMPRISING THE HETEROCYCLIC COMPOUND**

(71) Applicant: IDEMITSU KOSAN CO., LTD., Tokyo 100-8321 (JP)
(72) Inventor: Schäfer, Thomas, 4410 Liestal (CH); Boufflet, Pierre, 4055 Basel (CH); Chebotareva, Natalia, 68220 Hagenthal le Bas (FR)
(74) Representative: Hollah, Dorothee

(57) **Abstract**

The present invention relates to specific heterocyclic compounds of formula (I), a material, preferably an emitter material, for an organic electroluminescence device comprising said specific heterocyclic compounds, an organic electroluminescence device comprising said specific heterocyclic compounds, an electronic equipment comprising said organic electroluminescence device, a light emitting layer comprising at least one host and at least one dopant, wherein the dopant comprises at least one of said specific heterocyclic compounds, and the use of said heterocyclic compounds in an organic electroluminescence device.

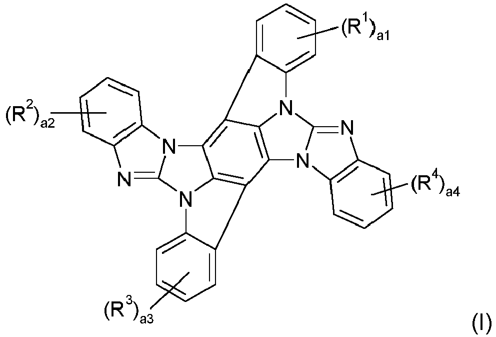

## Description

The present invention relates to specific heterocyclic compounds, a material, preferably an emitter material, for an organic electroluminescence device comprising said specific heterocyclic compounds, an organic electroluminescence device comprising said specific heterocyclic compounds, an electronic equipment comprising said organic electroluminescence device, a light emitting layer comprising at least one host and at least one dopant, wherein the dopant comprises at least one of said specific heterocyclic compounds, and the use of said heterocyclic compounds in an organic electroluminescence device.

When a voltage is applied to an organic electroluminescence device (hereinafter may be referred to as an organic EL device), holes are injected to an emitting layer from an anode and electrons are injected to an emitting layer from a cathode. In the emitting layer, injected holes and electrons are re-combined and excitons are formed.

An organic EL device comprises an emitting layer between the anode and the cathode. Further, there may be a case where it has a stacked layer structure comprising an organic layer such as a hole-injecting layer, a hole-transporting layer, an electron-injecting layer, an electron-transporting layer, etc.

WO 2017/093958 relates to compounds of formula (I) and their use in electronic devices, especially electroluminescent devices. It is mentioned WO 2017/093958 that the compounds may be used as charge transport material, charge blocker material and/or host material in electroluminescent devices. 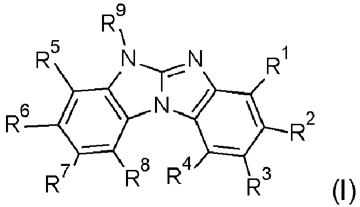 wherein
at least two of the substituents R¹ and R², R² and R³, R³ and R⁴, R⁵ and R⁶, R⁶ and R⁷, R⁷ and R⁸ form together one of the following ring systems

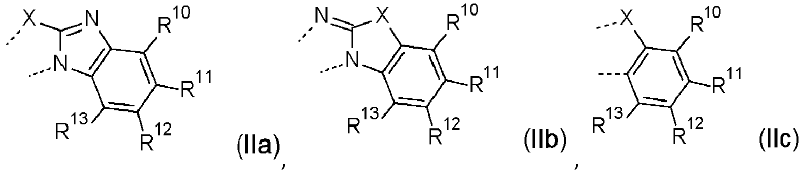

Compounds of formula (I), wherein R⁶ and R⁷ together form a ring system (IIa), wherein X is connected at the position R⁷ and N is connected at the position R⁶ of formula (I) are not exemplified.

US 2013/0092922 relates to an electronic device comprising anode, cathode and at least one organic layer, wherein the organic layer may comprise in one embodiment a compound of formula (IX-2).

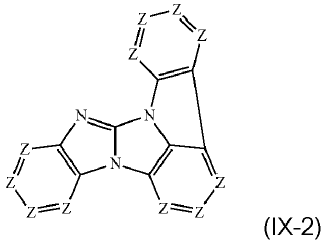

There is no specific example for a compound of formula (IX-2) mentioned in US 2013/009292. According to the examples, the compounds disclosed in US 2013/0092922 are employed as host (= matrix) materials or as hole conductors.

US 2010/0051928 relates to electroluminescence device, comprising:
a pair of electrodes; and
at least one organic layer including a light emitting layer, the light emitting layer being provided between the pair of electrodes,
wherein at least one layer of the at least one organic layer may comprise in one embodiment a carbazole based compound of formula (10):

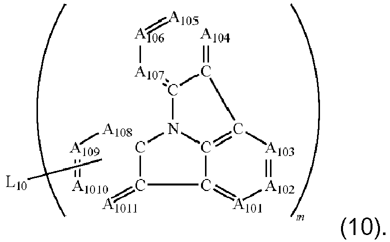

Benzimidazole based compounds are not disclosed in US 2010/0051928. According to the examples, the compounds disclosed in US 2010/0051928 are employed as host materials, hole transport materials or electron transport materials.

WO 2013/084805 relates to a nitrogen-containing heterocyclic compound represented by the general formula (1), an organic semiconductor material containing the nitrogen-containing heterocyclic compound and an organic semiconductor element using the nitrogen-containing heterocyclic compound.

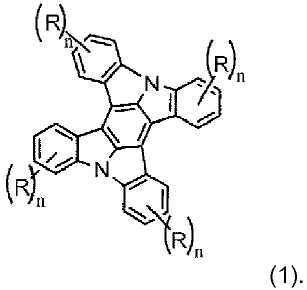

The organic electronic device is according to WO 2013/084805 any of a light emitting element, a thin film transistor, or a photovoltaic element.

However, the specific structure and substitution pattern of polycyclic compounds has a significant impact on the performance of the polycyclic compounds in organic electronic devices.

Notwithstanding the developments described above, there remains a need for organic electroluminescence devices comprising new materials, especially dopant (= emitter) materials, to provide improved performance of electroluminescence devices.

Accordingly, it is an object of the present invention, with respect to the aforementioned related art, to provide materials suitable for providing organic electroluminescence devices which ensure good performance of the organic electroluminescence devices, especially good EQEs and/or a long lifetime. More particularly, it should be possible to provide dopant (= emitter) materials, especially blue light emitting dopant materials having a narrow spectrum (smaller FWHM), i.e good color purity when used as dopant in organic electroluminescence devices.

Said object is according to one aspect of the present invention solved by a heterocyclic compound represented by formula (I): 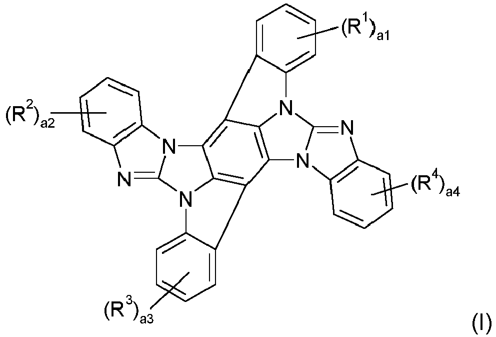 wherein
R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; a cycloalkyl group having from ring 3 to 20 carbon atoms which is unsubstituted or substituted; an alkenyl group having from 2 to 20 carbon atoms which is unsubstituted or substituted; an alkynyl group having from 2 to 20 carbon atoms which is unsubstituted or substituted; CN; N(R⁵)₂; OR⁶; SR⁷; Si(R⁸)₃; or halogen;
or
   two adjacent residues R¹, R², R³ and/or R⁴ together form a ring structure which is unsubstituted or substituted;
R⁵, R⁶, R⁷ and R⁸ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; or a cycloalkyl group having from 3 to 20 ring carbon atoms which is unsubstituted or substituted;
a1, a2, a3 and a4 each independently represents 0, 1, 2, 3 or 4;
when a1, a2, a3 and a4 are 2 to 4, each of the residues R¹, R², R³ as well as R⁴ are allowed to be the same or different.

The compounds of formula (I) can in principal be used in any layer of an EL device. Preferably, the compound of formula (I) is a dopant (= emitter) in organic EL elements, especially in the light-emitting layer, more preferably a fluorescent dopant. Particularly, the compounds of formula (I) are used as fluorescent dopants in organic EL devices, especially in the light-emitting layer.

The term organic EL device (organic electroluminescence device) is used interchangeably with the term organic light-emitting diode (OLED) in the present application.

It has been found that the specific compounds of formula (I) show a narrow emission characteristic, preferably a narrow fluorescence, more preferably a narrow blue fluorescence. Such a narrow emission characteristic is suitable to prevent energy losses by outcoupling. The compounds of formula (I) according to the present invention preferably have a Full width at half maximum (FWHM) of lower than 50 nm, more preferably lower than 40 nm, even more preferably lower than 35 nm, most preferably lower than 30 nm. Further most preferably from lower than 30 nm.

It has further been found that organic EL devices comprising the compounds of the present invention are generally characterized by high external quantum efficiencies (EQE) and long lifetimes, especially when the specific compounds of formula (I) are used as dopants (light emitting material), especially fluorescent dopants in organic electroluminescence devices.

The terms hydrogen atom, halogen, a substituted or unsubstituted alkyl group having from 1 to 20 carbon atoms, a substituted or unsubstituted alkenyl group having from 2 to 20 carbon atoms, a substituted or unsubstituted alkynyl group having from 2 to 20 carbon atoms, a substituted or unsubstituted cycloalkyl group having from 3 to 20 ring carbon atoms, a substituted or unsubstituted alkoxy group having 1 to 25 carbon atoms, a substituted or unsubstituted aryl group having from 6 to 18 ring carbon atoms, a substituted or unsubstituted aralkyl group having 6 to 30 ring carbon atoms, a substituted or unsubstituted heteroaryl group having from 5 to 18 ring atoms, N(R⁵)₂, OR⁶, SR⁷ and Si(R⁸)₃,

are known in the art and generally have the following meaning, if said groups are not further specified in specific embodiments mentioned below:
In the invention, hydrogen atom includes isomers differing in the number of neutrons, i.e. protium, deuterium and tritium.

The substituted or unsubstituted aryl group having from 6 to 18 ring carbon atoms, preferably from 6 to 13 ring carbon atoms, may be a non-condensed aryl group or a condensed aryl group. Specific examples thereof include phenyl group, naphthyl group, phenanthryl group, biphenyl group, terphenyl group, fluoranthenyl group, triphenylenyl group, phenanthrenyl group, fluorenyl group, anthracenyl, chrysenyl, spirofluorenyl group, , benzo[c]phenanthrenyl group, with phenyl group, naphthyl group, biphenyl group, terphenyl group, phenanthryl group, triphenylenyl group, fluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, fluorene-2-yl group, especially a 9,9-di-C₁₋₂₀alkylfluorene-2-yl group, like a 9,9-dimethylfluorene-2-yl group, a 9,9-di-C₆₋₁₈arylfluorene-2-yl group, like a 9,9-diphenylfluorene-2-yl group, or a 9,9-di-C₅₋₁₈heteroarylfluorene-2-yl group, fluoranthene-3-yl group, fluoranthene-2-yl group, fluoranthene-8-yl group being more preferred.

The heteroaryl group having from 5 to 18 ring atoms, preferably from 6 to 13 ring atoms, may be a non-condensed heteroaryl group or a condensed heteroaryl group. Specific examples thereof include the residues of pyrrole ring, isoindole ring, benzofuran ring, isobenzofuran ring, benzothiophene, dibenzothiophene ring, isoquinoline ring, quinoxaline ring, quinazoline, phenanthridine ring, phenanthroline ring, pyridine ring, pyrazine ring, pyrimidine ring, pyridazine ring, indole ring, quinoline ring, acridine ring, carbazole ring, furan ring, thiophene ring, benzoxazole ring, benzothiazole ring, benzimidazole ring, dibenzofuran ring, triazine ring, oxazole ring, oxadiazole ring, thiazole ring, thiadiazole ring, triazole ring, imidazole ring, indolidine ring, imidazopyridine ring, 4-imidazo[1,2-a]benzimidazoyl, 5-benzimidazo[1,2-a]benzimidazoyl, and benzimidazolo[2,1-b][1,3]benzothiazolyl, with the residues of dibenzofuran ring, carbazole ring, and dibenzothiophene ring being preferred, and the residues of dibenzofuran-1-yl group, dibenzofuran-3-yl group, dibenzofuran-2-yl group, dibenzofuran-4-yl group, 9-phenylcarbazole-3-yl group, 9-phenylcarbazole-2-yl group, 9-phenylcarbazole-4-yl group, dibenzothiophene-2-yl group, and dibenzothiophene-4-yl, dibenzothiophene-1-yl group, and dibenzothiophene-3-yl group being more preferred.

Examples of the alkyl group having from 1 to 20 carbon atoms include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, with methyl group, ethyl group, n-propyl group, isopropyl group, n-butyl group, s-butyl group, isobutyl group, t-butyl group being preferred. Preferred are alkyl groups having 1 to 8 carbon atoms, more preferably 1 to 4 carbon atoms. Suitable examples for alkyl groups having 1 to 8 carbon atoms respectively 1 to 4 carbon atoms are mentioned before.

Examples of the alkenyl group having from 2 to 20 carbon atoms include those disclosed as alkyl groups having 2 to 20 carbon atoms but comprising at least one double bond, preferably one, or where possible, two or three double bonds.

Examples of the alkynyl group having 2 to 20 carbon atoms include those disclosed as alkyl groups having 2 to 20 carbon atoms but comprising at least one triple bond, preferably one, or where possible, two or three triple bonds.

Examples of the cycloalkyl group having 3 to 20 ring carbon atoms include cyclopropyl group, cyclobutyl group, cyclopentyl group, cyclohexyl group, cyclooctyl group, and adamantyl group, with cyclopentyl group, and cyclohexyl group being preferred. Preferred are cycloalkyl groups having 3 to 6 carbon atoms. Suitable examples for cycloalkyl groups having 3 to 6 carbon atoms are mentioned before.

The group Si(R⁸)₃ is preferably an C₁₋₂₀alkyl and/or C₆₋₁₈aryl substituted silyl group. Preferred examples of C₁₋₂₀alkyl and/or C₆₋₁₈aryl substituted silyl groups include alkylsilyl groups having 1 to 10 carbon atoms in each alkyl residue, preferably 1 to 5 carbon atoms, including trimethylsilyl group, triethylsilyl group, tributylsilyl group, dimethylethylsilyl group, t-butyldimethylsilyl group, propyldimethylsilyl group, dimethylisopropylsilyl group, dimethylpropylsilyl group, dimethylbutylsilyl group, dimethyltertiarybutylsilyl group, diethylisopropylsilyl group, and arylsilyl groups having 6 to 18 ring carbon atoms in each aryl residue, including phenyldimethylsilyl group, diphenylmethylsilyl group, diphenyltertiarybutylsilyl group, and triphenylsilyl group, with diphenyltertiarybutylsilyl group and t-butyldimethylsilyl group being preferred.

Examples of halogen atoms include fluorine, chlorine, bromine, and iodine, with fluorine being preferred.

The group OR⁶ is preferably a C₁₋₂₀alkoxy group or a C₆₋₁₈aryloxy group. Examples of an alkoxy group having 1 to 20 carbon atoms, preferably 1 to 8 carbon atoms, include those having an alkyl portion selected from the alkyl groups mentioned above. Examples of an aryloxy group having 6 to 18 ring carbon atoms include those having an aryl portion selected from the aryl groups mentioned above.

The group SR⁷ is preferably a C₁₋₂₀alkylthio group or a C₆₋₁₈arylthio group. Examples of an alkylthio group having 1 to 20 carbon atoms include those having an alkyl portion selected from the alkyl groups mentioned above. Examples of an arylthio group having 6 to 18 ring carbon atoms include those having an aryl portion selected from the aryl groups mentioned above.

The group N(R⁵)₂ is preferably an C₁₋₂₀alkyl and/or C₆₋₁₈aryl and/or heteroaryl (having 5 to 18 ring atoms) substituted amino group. Examples of an alkylamino group (alkyl substituted amino group) having 1 to 20 ring carbon atoms include those having an alkyl portion selected from the alkyl groups mentioned above. Examples of an arylamino group (aryl substituted amino group) having 6 to 18 ring carbon atoms include those having an aryl portion selected from the aryl groups mentioned above. Examples of a heteroarylamino group (heteroaryl substituted amino group), preferably a heteroarylamino group having 5 to 18 ring atoms include those having an aryl portion selected from the heteroaryl groups mentioned above.

Examples of the optional substituent(s) indicated by "substituted or unsubstituted" and "may be substituted" referred to above or hereinafter include a halogen atom (fluorine, chlorine, bromine, iodine), a cyano group, an alkyl group having 1 to 20, preferably 1 to 6 carbon atoms, a cycloalkyl group having 3 to 20, preferably 5 to 12 carbon atoms, an alkoxy group having 1 to 20, preferably 1 to 5 carbon atoms, a fluoroalkyl group having 1 to 20, preferably 1 to 5 carbon atoms, an alkylamino group having 1 to 20 carbon atoms, preferably 1 to 5 carbon atoms, a carboxyalkyl group having 1 to 20 carbon atoms, preferably 1 to 5 carbon atoms, a carboxamidalkyl group having 1 to 20 carbon atoms, preferably 1 to 5 carbon atoms, a silyl group (Si(R⁸)₃), an aryl group having 6 to 18 ring carbon atoms, an aryloxy group having 6 to 18 ring carbon atoms, an alkylthio group having 1 to 20, preferably 1 to 5 carbon atoms, an arylthio group having 6 to 18 ring carbon atoms, an arylamino group having 6 to 18 ring carbon atoms in each aryl residue, a carboxyaryl group having 6 to 18 ring carbon atoms in the aryl residue, a carboxamidaryl group having 6 to 18 ring carbon atoms in the aryl residue and a heteroaryl group having 5 to 18 ring atoms.

Examples of a carboxyalkyl group having 1 to 20 carbon atoms, preferably 1 to 5 carbon atoms, those having an alkyl portion selected from the alkyl groups mentioned above.

Examples of a fluoroalkyl group having 1 to 20 carbon atoms include the alkyl groups mentioned above wherein the hydrogen atoms thereof are partly or entirely substituted by fluor atoms.

Examples of a carboxamidalkyl group (alkyl substituted amide group) having 1 to 20 carbon atoms, preferably 1 to 5 carbon atoms include those having an alkyl portion selected from the alkyl groups mentioned above.

Examples of a carboxamidaryl group (aryl substituted amide group) having 6 to 18 carbon atoms, preferably 6 to 18 carbon atoms, include those having an aryl portion selected from the aryl groups mentioned above.

The optional substituent is preferably a fluorine atom, a cyano group, an alkyl group having 1 to 20 carbon atoms, an aryl group having 6 to 18 ring carbon atoms, a cycloalkyl group having 5 to 12 carbon atoms, an arylsilyl group having 6 to 18 ring carbon atoms in each aryl residue, and a heteroaryl group having 5 to 18 ring atoms; more preferably a cyano group, a phenyl group, a naphthyl group, a biphenyl group, a terphenyl group, a phenanthryl group, a triphenylenyl group, a 9,9-disubstituted fluorenyl group, a spirobifluorenyl group, a fluoranthenyl group, a residue based on a dibenzofuran ring, a residue based on a carbazole ring, and a residue based on a dibenzothiophene ring, a methyl group, an ethyl group, an n-propyl group, an isopropyl group, an n-butyl group, an s-butyl group, an isobutyl group, a t-butyl group, a cyclopentyl group, an arylsilyl group having 6 to 18 ring carbon atoms in each aryl residue, preferably SiMe₃ and SiPh₃, and a cyclohexyl group.

The optional substituent mentioned above may be further substituted by one or more of the optional substituents mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

The "carbon number of a to b" in the expression of "substituted or unsubstituted X group having a to b carbon atoms" is the carbon number of the unsubstituted X group and does not include the carbon atom(s) of an optional substituent.

The term "unsubstituted" referred to by "unsubstituted or substituted" means that a hydrogen atom is not substituted by one the groups mentioned above.

An index of 0 in the definition in any formula mentioned above and below means that a hydrogen atom is present at the position defined by said index.

### The compounds of formula (I)

In the heterocyclic compounds represented by formula (I) 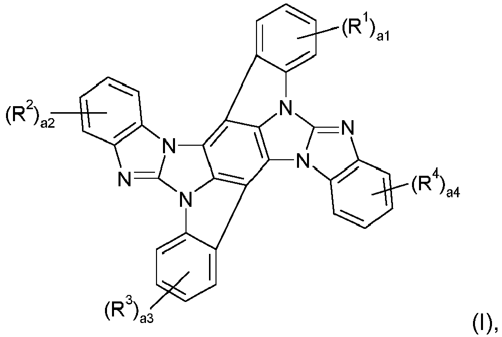 the residues have the following meanings:
R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; a cycloalkyl group having from ring 3 to 20 carbon atoms which is unsubstituted or substituted; an alkenyl group having from 2 to 20 carbon atoms which is unsubstituted or substituted; an alkynyl group having from 2 to 20 carbon atoms which is unsubstituted or substituted; CN; N(R⁵)₂; OR⁶; SR⁷; Si(R⁸)₃; or halogen;
or
   two adjacent residues R¹, R², R³ and/or R⁴ together form a ring structure which is unsubstituted or substituted.

Examples for ring structures formed by two adjacent residues R¹, R², R³ and/or R⁴ are shown below: 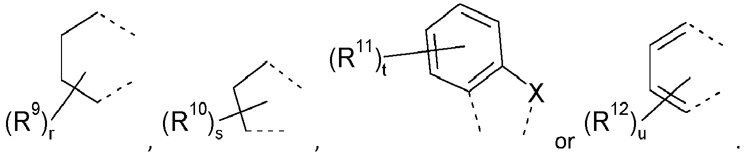 wherein
R⁹, R¹⁰, R¹¹ and R¹² each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; or a cycloalkyl group having from 3 to 20 ring carbon atoms which is unsubstituted or substituted; preferably an aryl group having from 6 to 13 ring carbon atoms which is unsubstituted or substituted; a heteroaryl group having from 5 to 13 ring atoms which is unsubstituted or substituted; an alkyl group having from 1 to 4 carbon atoms which is unsubstituted or substituted; or a cycloalkyl group having from 5 to 6 ring carbon atoms which is unsubstituted or substituted;
r, s, t and u each independently represents 0, 1, 2, 3 or 4, preferably 0, 1 or 2, more preferably 0;
when r, s, t and u are 2 to 4, each of the residues R⁹, R¹⁰, R¹¹ as well as R¹² are allowed to be the same or different; and
X is O, S or C(R¹³R¹⁴); NR¹⁵ and
R¹³, R¹⁴ and R¹⁵ each independently represents an alkyl group having from 1 to 20 carbon atoms which is unsubstituted or substituted, a substituted or unsubstituted aryl group having from 6 to 18 carbon atoms or a substituted or unsubstituted heterocyclic group having 5 to 18 ring atoms;
wherein the dotted lines are bonding sites.

Preferably, R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; a cycloalkyl group having from 3 to 20 ring carbon atoms which is unsubstituted or substituted; an alkenyl group having from 2 to 20 carbon atoms which is unsubstituted or substituted; an alkynyl group having from 2 to 20 carbon atoms which is unsubstituted or substituted; CN; N(R⁵)₂; OR⁶; SR⁷; Si(R⁸)₃; or halogen.

More preferably, R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; CN; N(R⁵)₂, Si(R⁸)₃; or halogen; or a cycloalkyl group having from 3 to 20 ring carbon atoms which is unsubstituted or substituted.

Most preferably, R¹, R², R³ and R⁴ each independently represents an alkyl group having 1 to 4 carbon atoms which is unsubstituted or substituted; an aryl group having 6 to 13 ring carbon atoms which is unsubstituted or substituted; or a heteroaryl group having from 5 to 13 ring atoms which is unsubstituted or substituted.

In one embodiment of the present invention, R¹ and R³ are identical and/or R² and R⁴ are identical.

R⁵, R⁶, R⁷ and R⁸ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; or a cycloalkyl group having from 3 to 20 ring carbon atoms which is unsubstituted or substituted.

Preferably, R⁵, R⁶, R⁷ and R⁸ each independently represents an aryl group having from 6 to 13 ring carbon atoms which is unsubstituted or substituted; or an alkyl group having from 1 to 20 carbon atoms which is unsubstituted or substituted.

More preferably, R⁵, R⁶, R⁷ and R⁸ each independently represents a phenyl which is unsubstituted or substituted; or an alkyl group having from 1 to 4 carbon atoms which is unsubstituted or substituted.

a1, a2, a3 and a4 each independently represents 0, 1, 2, 3 or 4;
when a1, a2, a3 and a4 are 2 to 4, each of the residues R¹, R², R³ as well as R⁴ are allowed to be the same or different. Preferably, the sum of a1, a2, a3 and a4 is at least 1, preferably at least 2. More preferably, a1 and a3 each independently represents 1; and a2 and a4 each independently represents 0, 1 or 2.

In a preferred embodiment the heterocyclic compound according to the present invention is represented by the following formula (la) 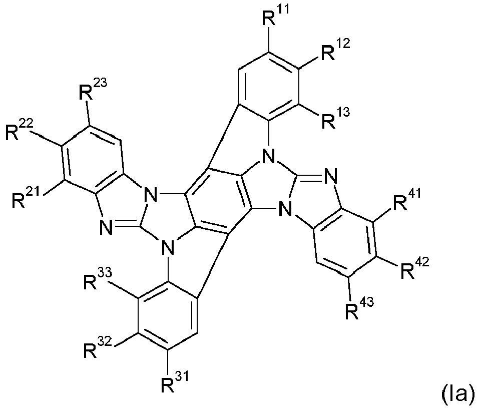 wherein
R¹¹, R¹², R¹³, R²¹, R²², R²³, R³¹, R³², R³³, R⁴¹, R⁴² and R⁴³ each independently represents hydrogen, or any of the definitions given for R¹, R², R³ and R⁴ as mentioned above.

Preferably, at least 1 of R¹¹, R¹², R¹³, R²¹, R²², R²³, R³¹, R³², R³³, R⁴¹, R⁴² and R⁴³ is not hydrogen, more preferably at least 2 of R¹¹, R¹², R¹³, R²¹, R²², R²³, R³¹, R³², R³³, R⁴¹, R⁴² and R⁴³ is are not hydrogen. Most preferably, 1 of R¹¹, R¹², R¹³ and 1 of R³¹, R³², R³³ is not hydrogen and 0, 1 or 2 of R²¹, R²², R²³ and 0 or 1 of R⁴¹, R⁴² and R⁴³ are not hydrogen. The remaining residues R¹¹, R¹², R¹³, R²¹, R²², R²³, R³¹, R³², R³³, R⁴¹, R⁴² and R⁴³ are most preferably hydrogen.

In a further preferred embodiment the heterocyclic compound according to the present invention is represented by the following formula (Iaa) 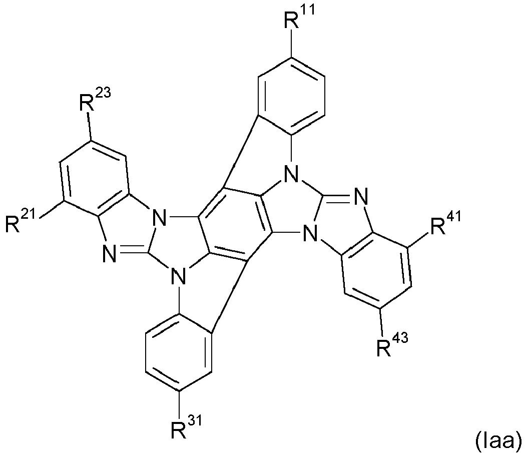 wherein
R¹¹, R²¹, R²³, R³¹, R⁴¹ and R⁴³ each independently represents hydrogen, or any of the definitions given for R¹, R², R³ and R⁴ as mentioned above.

Below, examples for compounds of formula (I) are given:

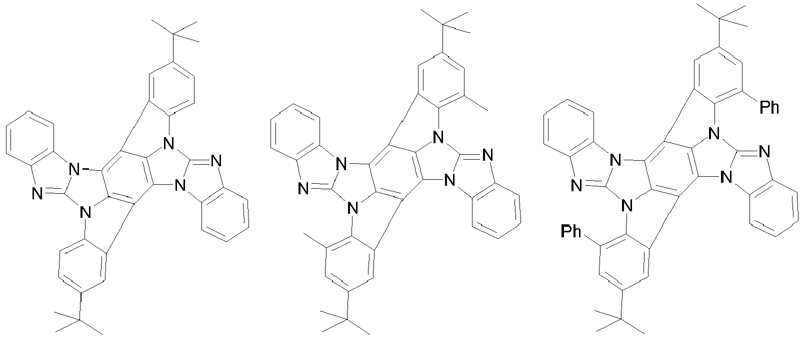

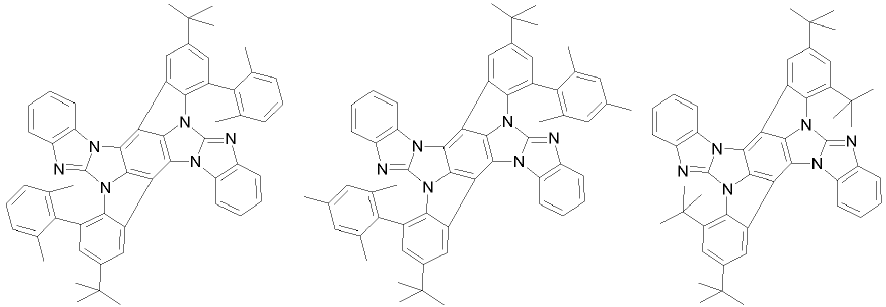

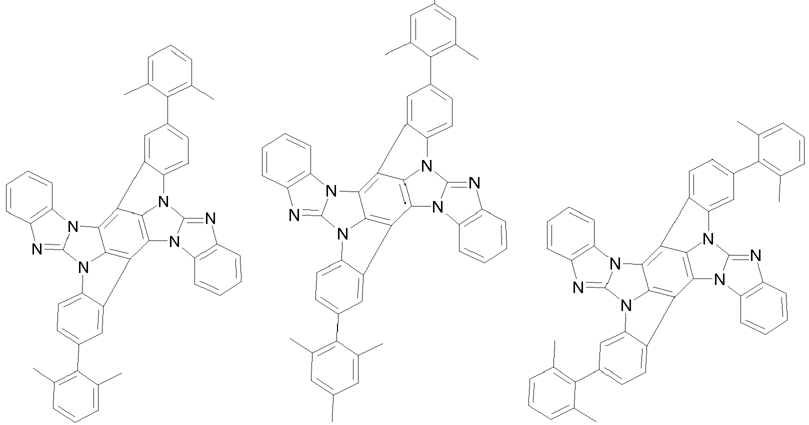

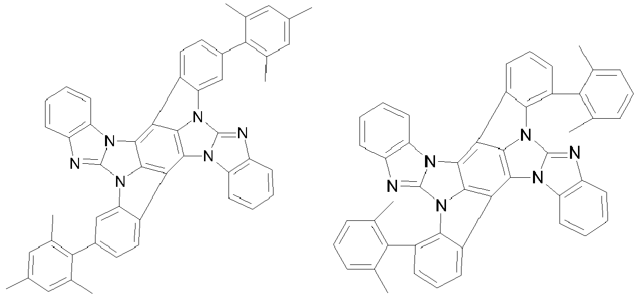

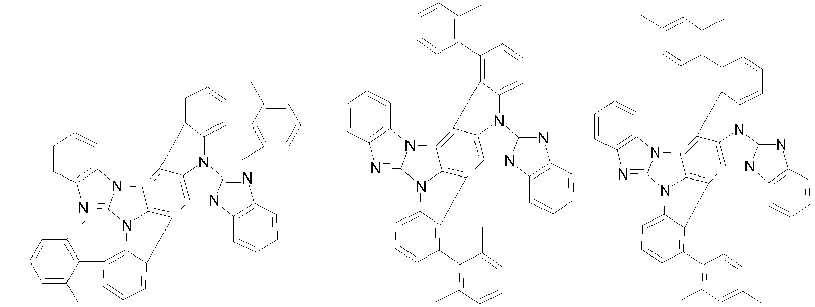

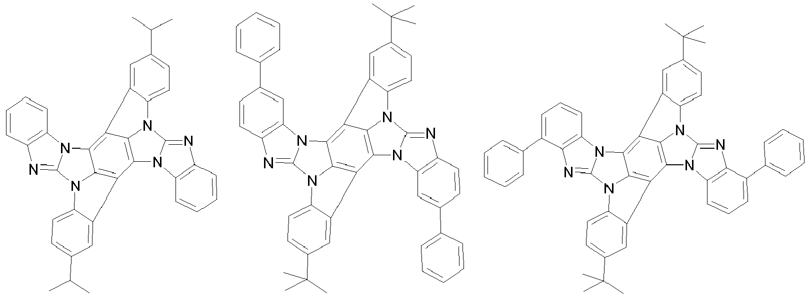

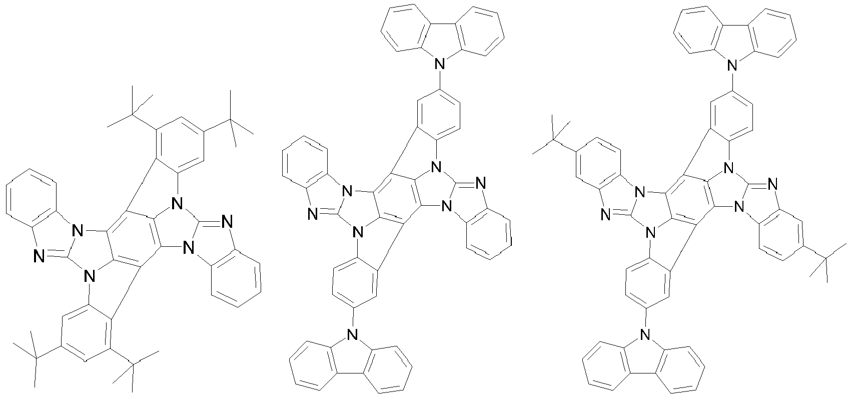

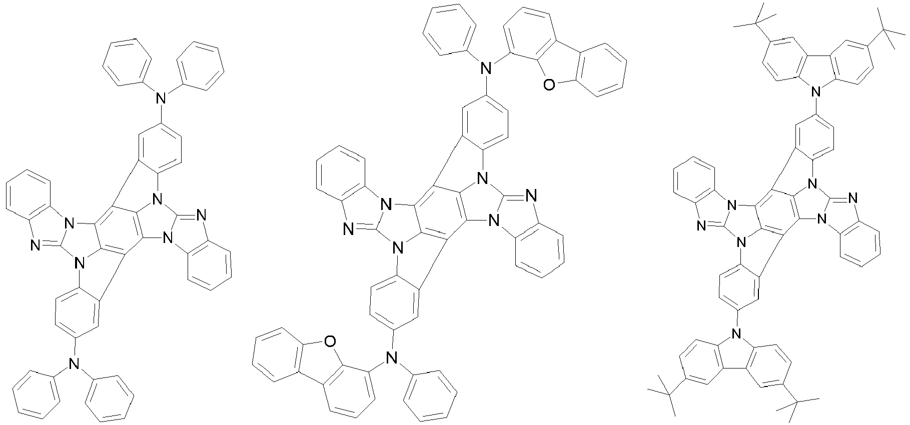

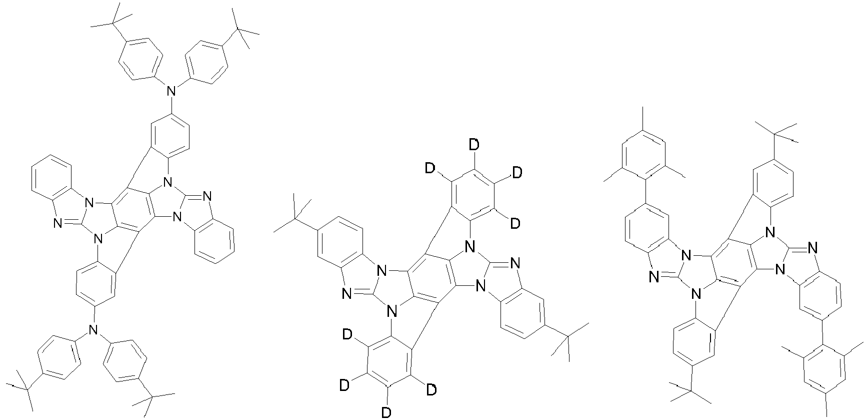

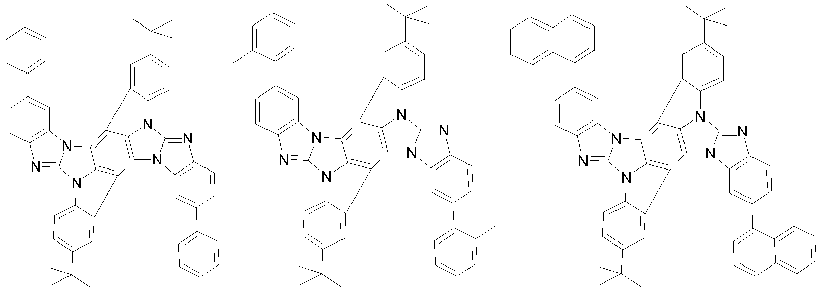

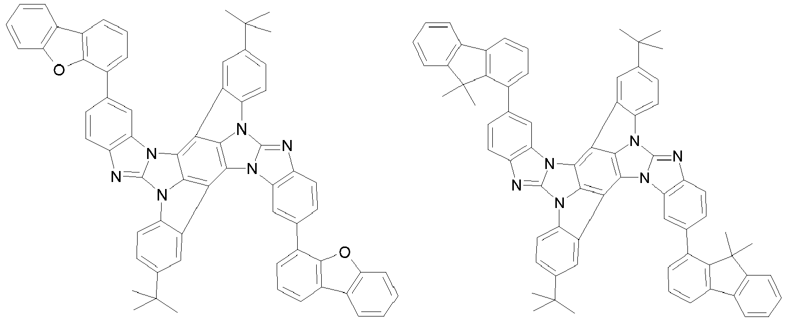

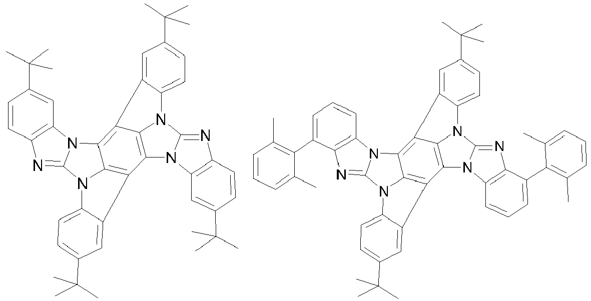

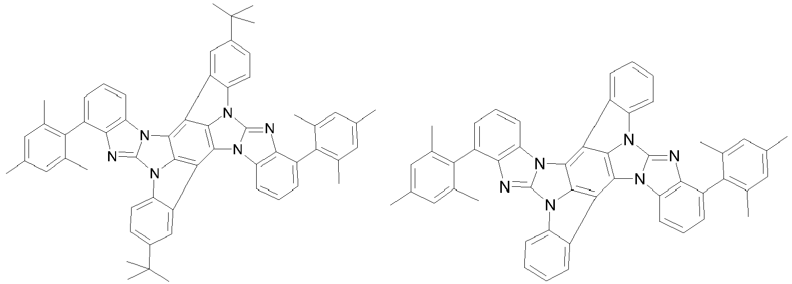

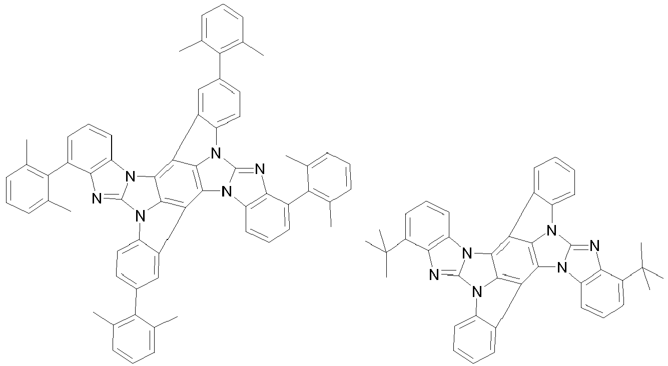

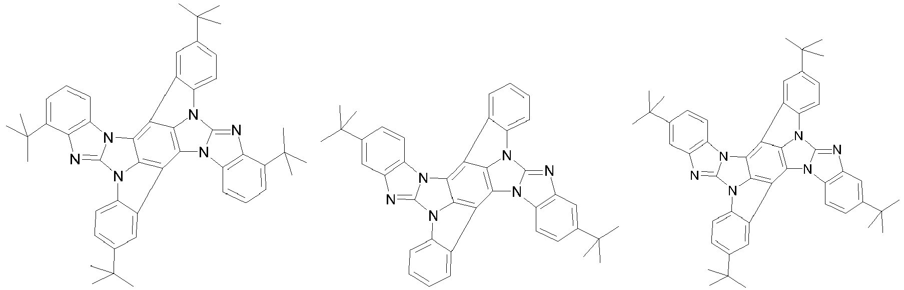

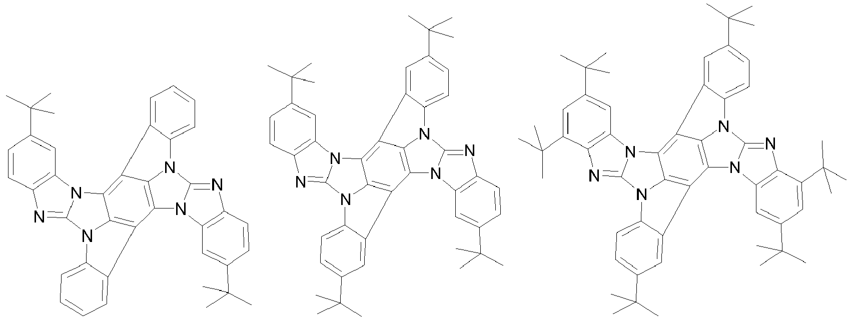

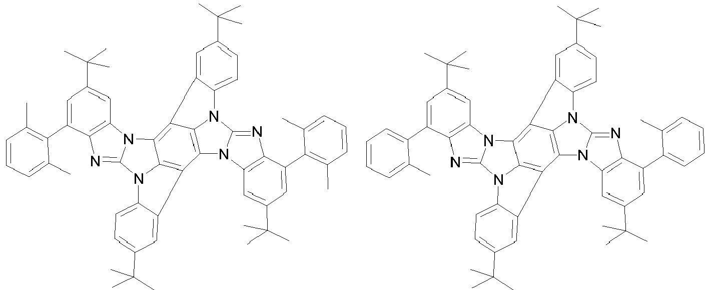

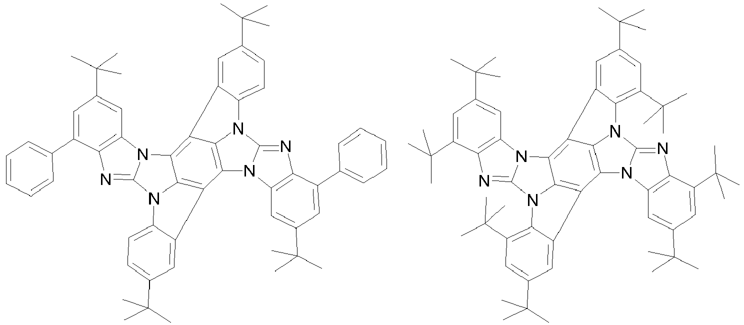

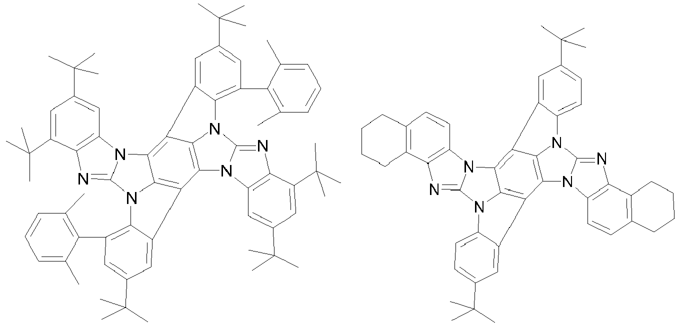

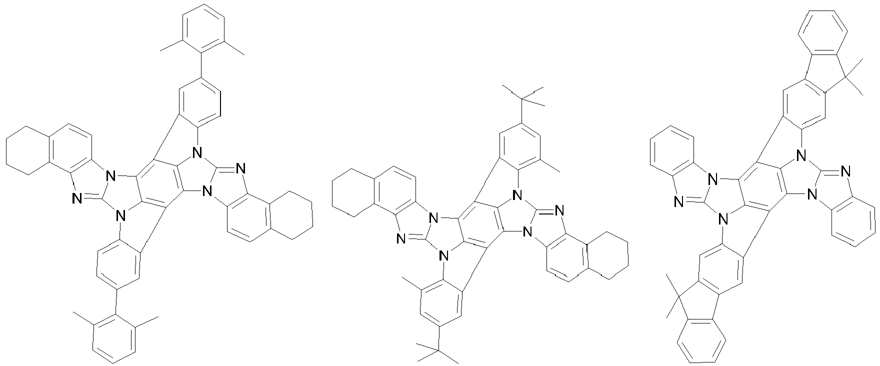

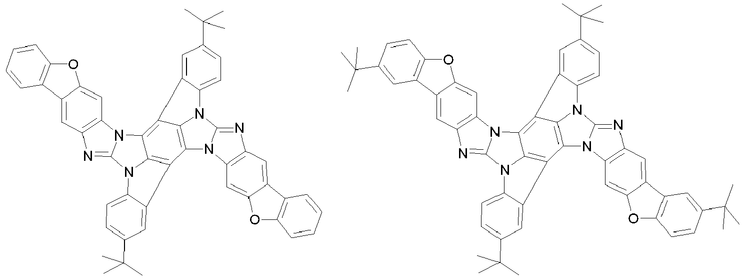

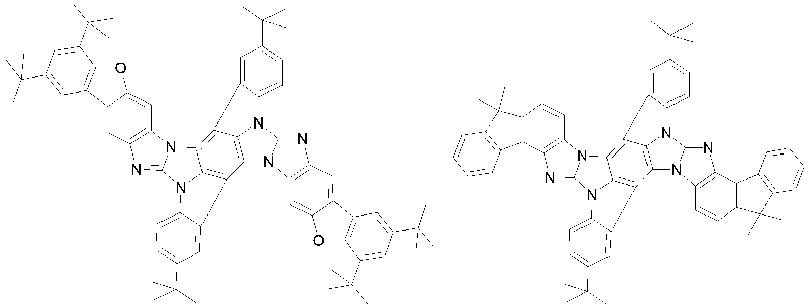

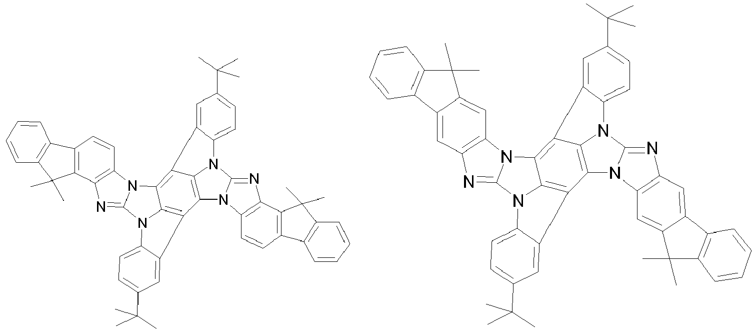

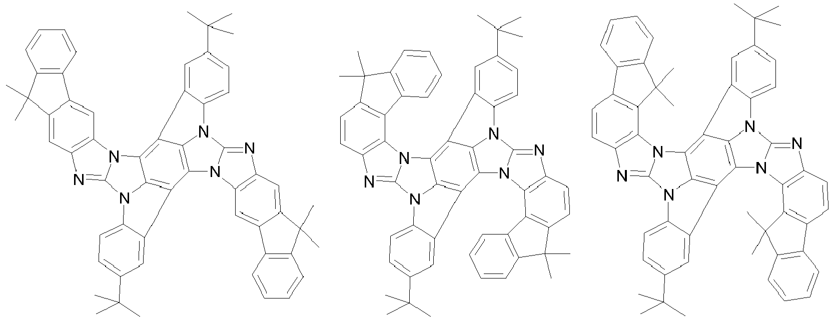

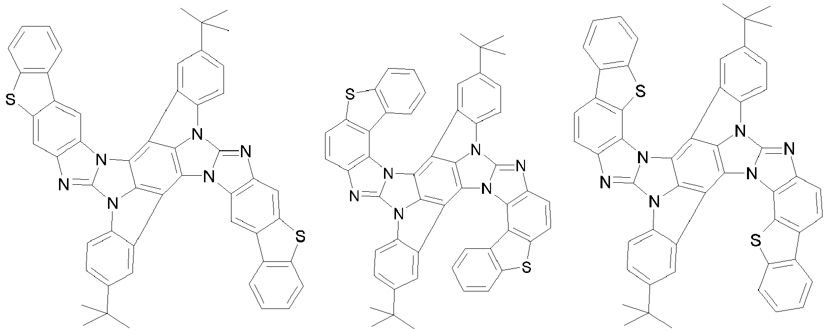

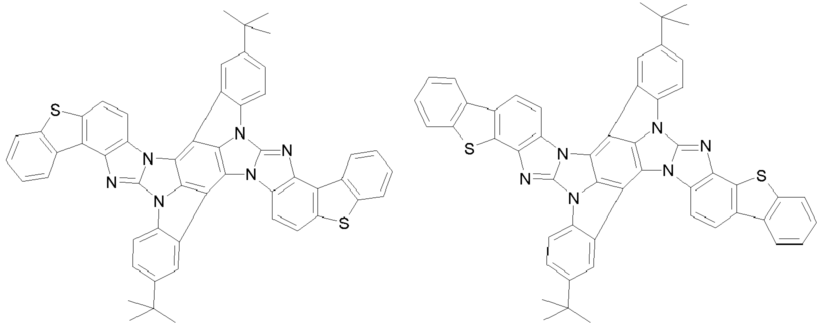

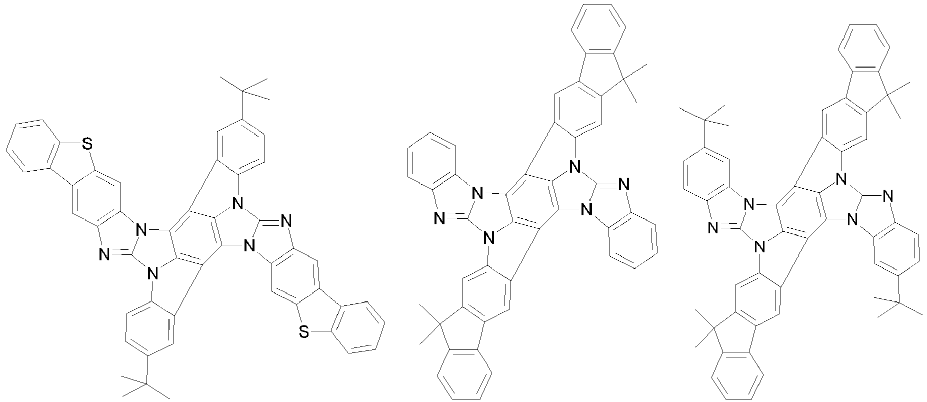

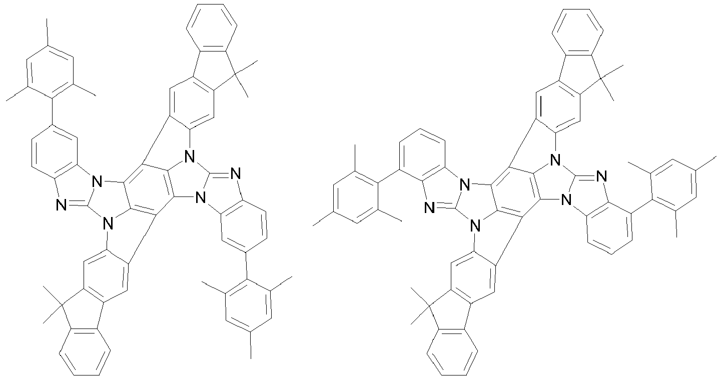

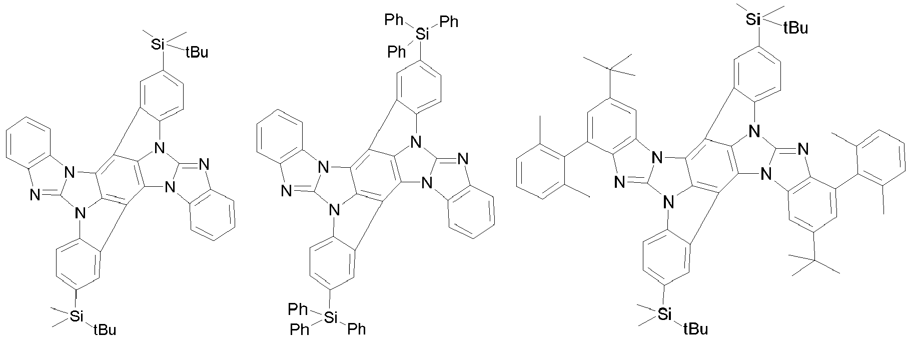

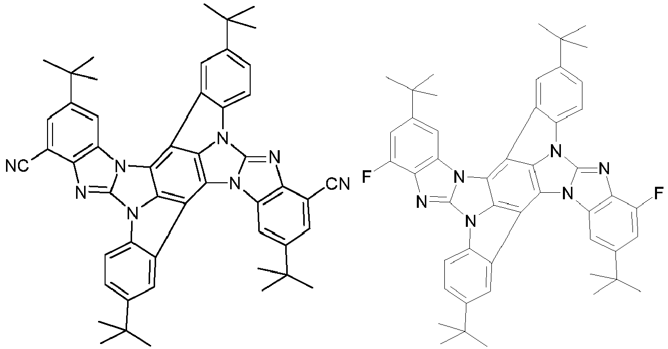

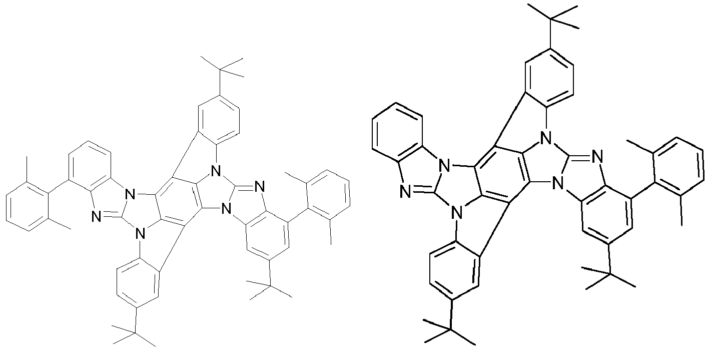

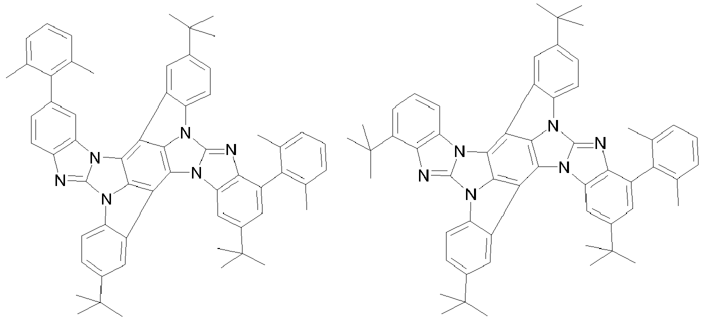

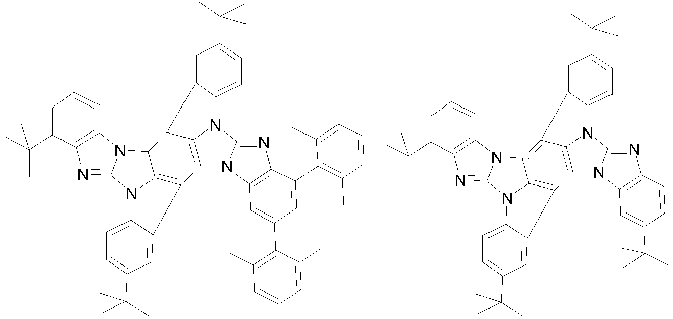

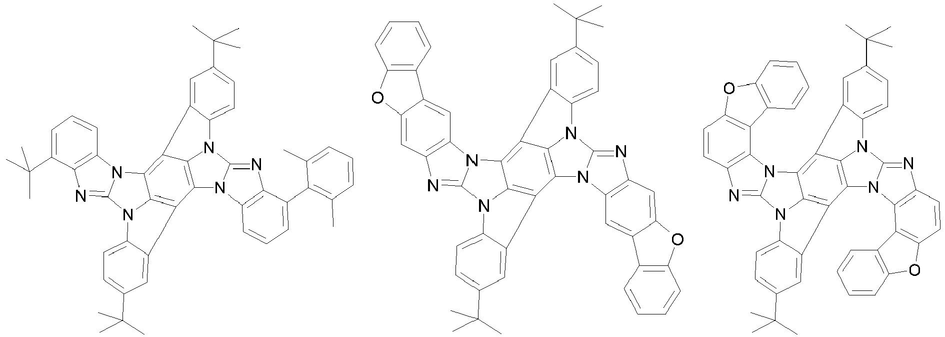

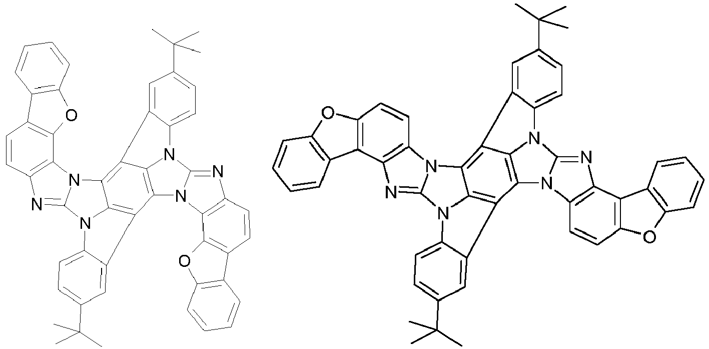

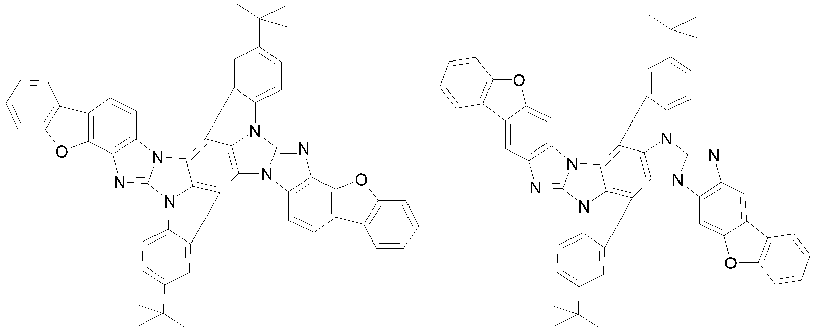

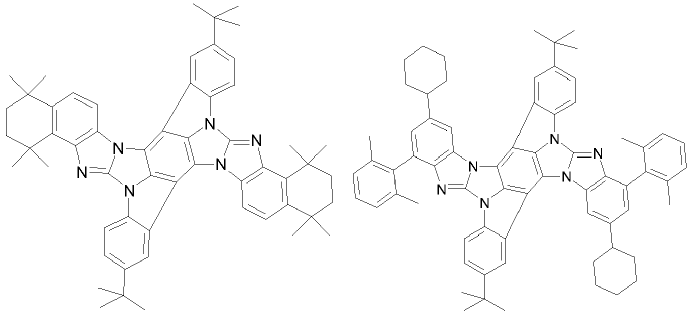

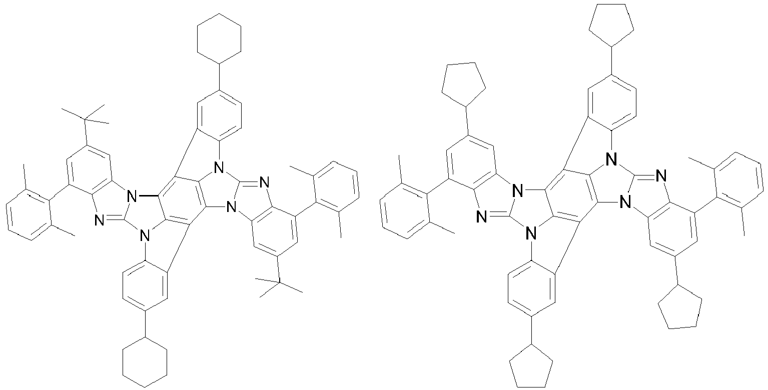

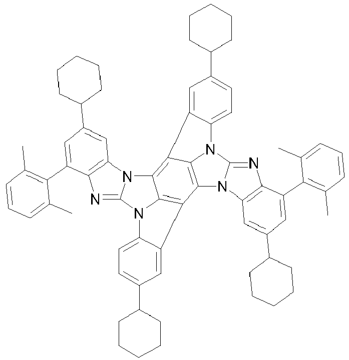

### Preparation of the compounds of formula (I)

The compound represented by formula (I) can be synthesized in accordance with the reactions conducted in the Examples of the present application, and by using alternative reactions or raw materials suited to an intended product, in analogy to reactions and raw materials known in the art.

The compounds of formula (I) are for example prepared by the following steps:
(i) Coupling two intermediates (II) and (II') with a compound of formula (III), wherein intermediate (IV) is obtained: 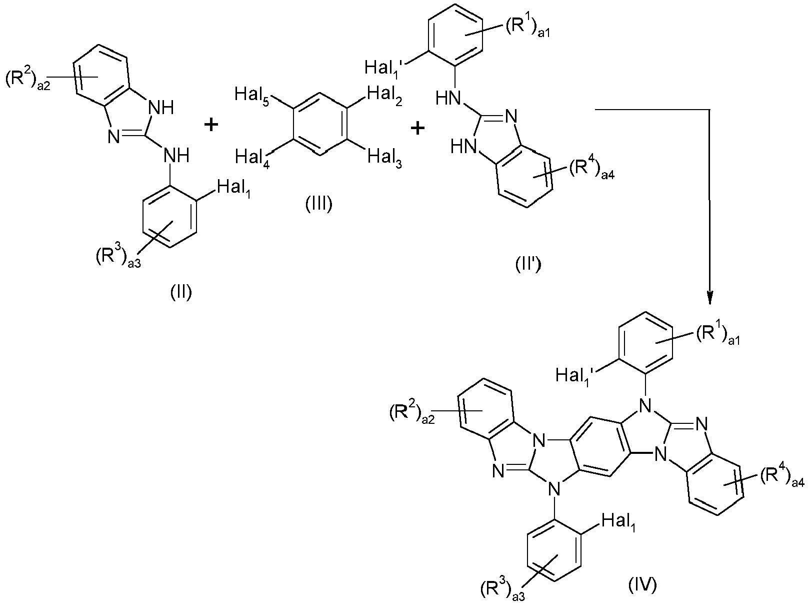 wherein
   Hal₁, Hal_{1'}, Hal₂, Hal₃, Hal₄ and Hal₅ each independently represent halogen, preferably, Hal₁ and Hal_{1'} each independently represent I, Br and Cl, more preferably Br and Cl; Hal₂ and Hal₄ each independently represent F or Cl, more preferably F; and Hal₃ and Hal₅ each independently represent F, Cl, Br or I, more preferably Cl or Br and most preferably Br; and
   all other residues and indices are as defined before;
(ii) Ring closure at intermediate (IV), whereby the compound of formula (I) is formed:

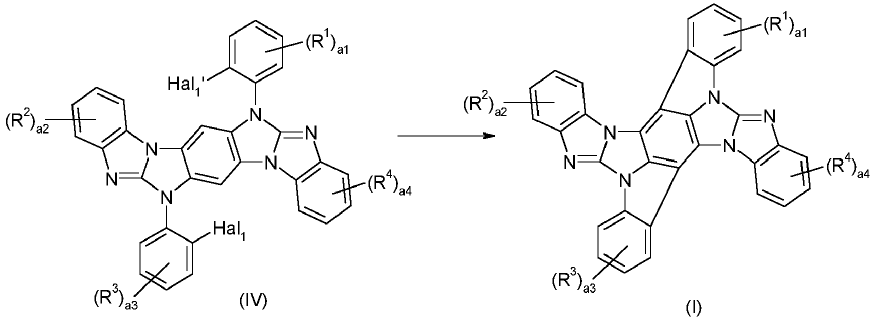

Suitable reaction conditions are mentioned in the examples of the present application.

The intermediates (II) and (II') can for example be prepared by coupling a benzimidazole halogenated in 2-position (V) with an aniline halogenated in the 2-position (VI):

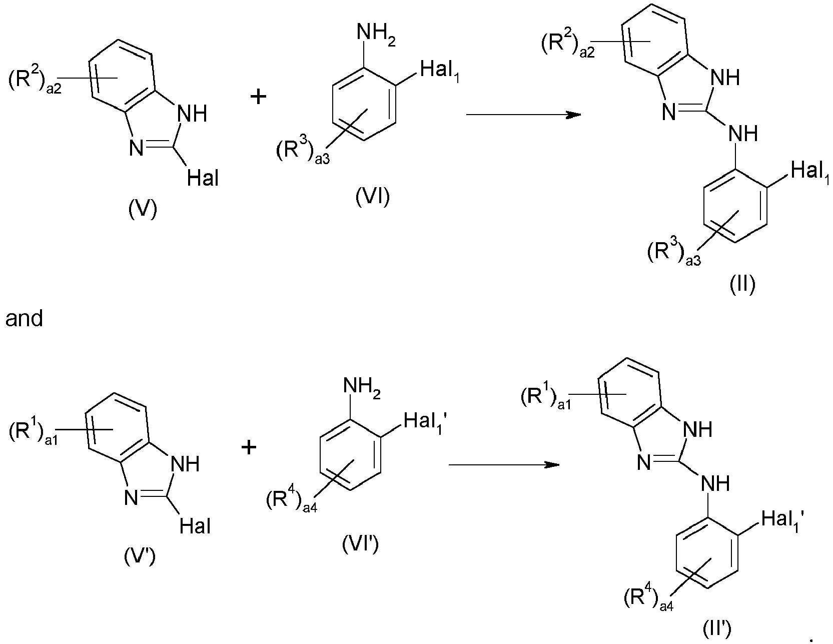

Examples for suitable preparation processes are mentioned below.

### Organic electroluminescence device

According to one aspect of the present invention a material for an organic electroluminescence device comprising at least one compound of formula (I) is provided.

According to another aspect of the present invention, an organic electroluminescence device comprising at least one compound of formula (I) is provided.

According to another aspect of the invention, the following organic electroluminescence device is provided: An organic electroluminescence device comprising a cathode, an anode, and one or more organic thin film layers comprising a light emitting layer disposed between the cathode and the anode, wherein at least one layer of the organic thin film layers comprises at least one compound of formula (I).

According to another aspect of the invention an organic electroluminescence device is provided, wherein the light emitting layer comprises at least one compound of formula (I).

According to another aspect of the invention an organic electroluminescence device is provided, wherein the light emitting layer comprises at least one compound of formula (I) as a dopant material and an anthracene compound as a host material.

According to another aspect of the invention an electronic equipment provided with the organic electroluminescence device according to the present invention is provided.

According to another aspect of the invention an emitter material is provided comprising at least one compound of formula (I).

According to another aspect of the invention a light emitting layer is provided comprising at least one host and at least one dopant, wherein the dopant comprises at least one compound of formula (I).

According to another aspect of the invention the use of a compound of formula (I) according to the present invention in an organic electroluminescence device is provided.

In one embodiment, the organic EL device has a hole-transporting layer between the anode and the emitting layer.

In one embodiment, the organic EL device has an electron-transporting layer between the cathode and the emitting layer.

In the present specification, regarding the "one or more organic thin film layers between the emitting layer and the anode", if only one organic layer is present between the emitting layer and the anode, it means that layer, and if plural organic layers are present, it means at least one layer thereof. For example, if two or more organic layers are present between the emitting layer and the anode, an organic layer nearer to the emitting layer is called the "hole-transporting layer", and an organic layer nearer to the anode is called the "hole-injecting layer". Each of the "hole-transporting layer" and the "hole-injecting layer" may be a single layer or may be formed of two or more layers. One of these layers may be a single layer and the other may be formed of two or more layers.

Similarly, regarding the "one or more organic thin film layers between the emitting layer and the cathode", if only one organic layer is present between the emitting layer and the cathode, it means that layer, and if plural organic layers are present, it means at least one layer thereof. For example, if two or more organic layers are present between the emitting layer and the cathode, an organic layer nearer to the emitting layer is called the "electron-transporting layer", and an organic layer nearer to the cathode is called the "electron-injecting layer". Each of the "electron-transporting layer" and the "electron-injecting layer" may be a single layer or may be formed of two or more layers. One of these layers may be a single layer and the other may be formed of two or more layers.

The "one or more organic thin film layers comprising an emitting layer" mentioned above, preferably the emitting layer, comprises a compound represented by formula (I). The compound represented by formula (I) preferably functions as an emitter material, more preferably as a fluorescent emitter material, most preferably as a blue fluorescent emitter material. By the presence of a compound of formula (I) in the organic EL device, preferably in the emitting layer, organic EL devices characterized by high external quantum efficiencies (EQE) and long lifetimes are provided.

According to another aspect of the invention, an emitting layer of the organic electroluminescence device is provided which comprises at least one compound of formula (I).

Preferably, the emitting layer comprises at least one emitting material (dopant material) and at least one host material, wherein the emitting material is at least one compound of formula (I).

Preferred host materials are substituted or unsubstituted polyaromatic hydrocarbon (PAH) compounds, substituted or unsubstituted polyheteroaromatic compounds, substituted or unsubstituted anthracene compounds, or substituted or unsubstituted pyrene compounds.

More preferably, the organic electroluminescence device according to the present invention comprises in the emitting layer at least one compound of formula (I) as a dopant material and at least one host material selected from the group consisting of substituted or unsubstituted polyaromatic hydrocarbon (PAH) compounds, substituted or unsubstituted polyheteroaromatic compounds, substituted or unsubstituted anthracene compounds, and substituted or unsubstituted pyrene compounds. Preferably, the at least one host is at least one substituted or unsubstituted anthracene compound.

According to another aspect of the invention, an emitting layer of the organic electroluminescence device is provided which comprises at least one compound of formula (I) as a dopant material and an anthracene compound as a host material.

Suitable anthracene compounds are represented by the following formula (10): 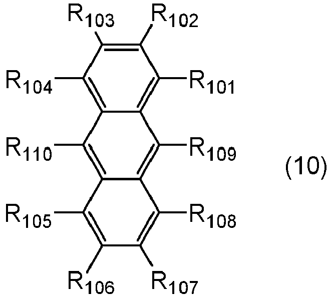 wherein
one or more pairs of two or more adjacent R₁₀₁ to R₁₁₀ may form a substituted or unsubstituted, saturated or unsaturated ring;
R₁₀₁ to R₁₁₀ that do not form the substituted or unsubstituted, saturated or unsaturated ring are independently a hydrogen atom, a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylene group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₁₂₁)(R₁₂₂)(R₁₂₃), -C(=O)R₁₂₄, -COOR₁₂₅, -N(R₁₂₆)(R₁₂₇), a halogen atom, a cyano group, a nitro group, a substituted or unsubstituted aryl group including 6 to 50 ring carbon atoms, a substituted or unsubstituted monovalent heterocyclic group including 5 to 50 ring atoms, or a group represented by the following formula (31);
R₁₂₁ to R₁₂₇ are independently a hydrogen atom, a substituted or unsubstituted alkyl group including 1 to 50 carbon atoms, a substituted or unsubstituted cycloalkyl group including 3 to 50 ring carbon atoms, a substituted or unsubstituted aryl group including 6 to 50 ring carbon atoms or a substituted or unsubstituted monovalent heterocyclic group including 5 to 50 ring atoms; when each of R₁₂₁ to R₁₂₇ is present in plural, each of the plural R₁₂₁ to R₁₂₇ may be the same or different;
provided that at least one of R₁₀₁ to R₁₁₀ that do not form the substituted or unsubstituted, saturated or unsaturated ring is a group represented by the following formula (31). If two or more groups represented by the formula (31) are present, each of these groups may be the same or different;

   -L₁₀₁-Ar₁₀₁ (31)

   wherein in the formula (31),
   L₁₀₁ is a single bond, a substituted or unsubstituted arylene group including 6 to 30 ring carbon atoms or a substituted or unsubstituted divalent heterocyclic group including 5 to 30 ring atoms;
   Ar₁₀₁ is a substituted or unsubstituted aryl group including 6 to 50 ring carbon atoms or a substituted or unsubstituted monovalent heterocyclic group including 5 to 50 ring atoms.

Specific examples of each substituent, substituents for "substituted or unsubstituted" and the halogen atom in the compound (10) are the same as those mentioned above.

An explanation will be given on "one or more pairs of two or more adjacent R₁₀₁ to R₁₁₀ may form a substituted or unsubstituted, saturated or unsaturated ring".
The "one pair of two or more adjacent R₁₀₁ to R₁₁₀" is a combination of R₁₀₁ and R₁₀₂, R₁₀₂ and R₁₀₃, R₁₀₃ and R₁₀₄, R₁₀₅ and R₁₀₆, R₁₀₆ and R₁₀₇, R₁₀₇ and R₁₀₈, R₁₀₈ and R₁₀₉, R₁₀₁ and R₁₀₂ and R₁₀₃ or the like, for example.
The substituent in "substituted" in the "substituted or unsubstituted" for the saturated or unsaturated ring is the same as those for "substituted or unsubstituted" mentioned in the formula (10).

The "saturated or unsaturated ring" means, when R₁₀₁ and R₁₀₂ form a ring, for example, a ring formed by a carbon atom with which R₁₀₁ is bonded, a carbon atom with which R₁₀₂ is bonded and one or more arbitrary elements. Specifically, when a ring is formed by R₁₀₁ and R₁₀₂, when an unsaturated ring is formed by a carbon atom with which R₁₀₁ is bonded, a carbon atom with R₁₀₂ is bonded and four carbon atoms, the ring formed by R₁₀₁ and R₁₀₂ is a benzene ring.

The "arbitrary element" is preferably a C element, a N element, an O element or a S element. In the arbitrary element (C element or N element, for example), atomic bondings that do not form a ring may be terminated by a hydrogen atom, or the like.
The "one or more arbitrary element" is preferably 2 or more and 15 or less, more preferably 3 or more and 12 or less, and further preferably 3 or more and 5 or less arbitrary elements.

For example, R₁₀₁ and R₁₀₂ may form a ring, and simultaneously, R₁₀₅ and R₁₀₆ may form a ring. In this case, the compound represented by the formula (10) is a compound represented by the following formula (10A), for example:

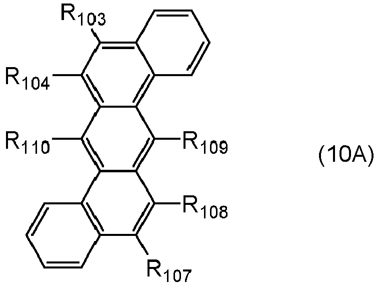

In one embodiment, R₁₀₁ to R₁₁₀ are independently a hydrogen atom, a substituted or unsubstituted alkyl group including 1 to 50 carbon atoms, a substituted or unsubstituted aryl group including 6 to 50 ring carbon atoms, a substituted or unsubstituted heterocyclic group including 5 to 50 ring atoms or a group represented by the formula (31).

Preferably, R₁₀₁ to R₁₁₀ are independently a hydrogen atom, a substituted or unsubstituted aryl group including 6 to 50 ring carbon atoms, a substituted or unsubstituted heterocyclic group including 5 to 50 ring atoms or a group represented by the formula (31).

More preferably, R₁₀₁ to R₁₁₀ are independently a hydrogen atom, a substituted or unsubstituted aryl group including 6 to 18 ring carbon atoms, a substituted or unsubstituted heterocyclic group including 5 to 18 ring atoms or a group represented by the formula (31).

Most preferably, at least one of R₁₀₉ and R₁₁₀ is a group represented by the formula (31).

Further most preferably, R₁₀₉ and R₁₁₀ are independently a group represented by the formula (31).

In one embodiment, the compound (10) is a compound represented by the following formula (10-1): 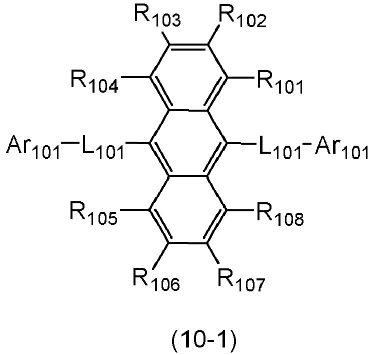 wherein in the formula (10-1), R₁₀₁ to R₁₀₈, L₁₀₁ and Ar₁₀₁ are as defined in the formula (10).

In one embodiment, the compound (10) is a compound represented by the following formula (10-2): 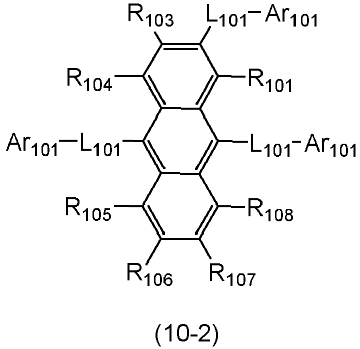 wherein in the formula (10-2), R₁₀₁, R₁₀₃ to R₁₀₈, L₁₀₁ and Ar₁₀₁ are as defined in the formula (10).

In one embodiment, the compound (10) is a compound represented by the following formula (10-3): 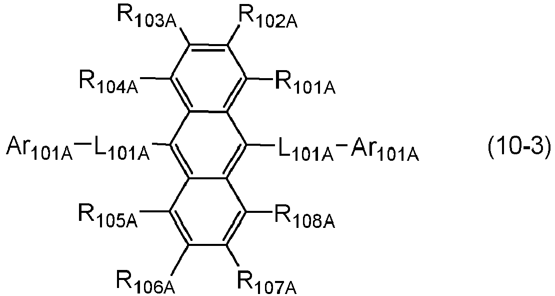 wherein in the formula (10-3),
R_{101A} to R_{108A} are independently a hydrogen atom or a substituted or unsubstituted aryl group including 6 to 50 ring carbon atoms;
L_{101A} is a single bond or a substituted or unsubstituted arylene group including 6 to 30 ring carbon atoms, and the two L_{101A}s may be the same or different;
Ar_{101A} is a substituted or unsubstituted aryl group including 6 to 50 ring carbon atoms, and the two Ar_{101A}s may be the same or different.

In one embodiment, the compound (10) is a compound represented by the following formula (10-4): 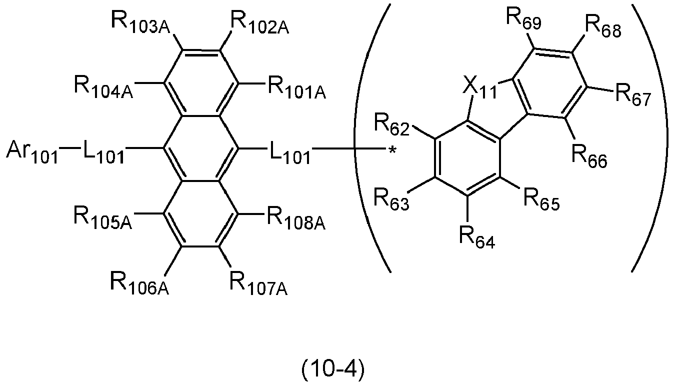 wherein in the formula (10-4),
L₁₀₁ and Ar₁₀₁ are as defined in the formula (10);
R_{101A} to R_{108A} are independently a hydrogen atom or a substituted or unsubstituted aryl group including 6 to 50 ring carbon atoms;
X₁₁ is O, S, or N(R₆₁);
R₆₁ is a hydrogen atom, a substituted or unsubstituted alkyl group including 1 to 50 carbon atoms or a substituted or unsubstituted aryl group including 6 to 50 ring carbon atoms;
one of R₆₂ to R₆₉ is an atomic bonding that is bonded with L₁₀₁;
one or more pairs of adjacent R₆₂ to R₆₉ that are not bonded with L₁₀₁ may be bonded with each other to form a substituted or unsubstituted, saturated or unsaturated ring; and R₆₂ to R₆₉ that are not bonded with L₁₀₁ and do not form the substituted or unsubstituted, saturated or unsaturated ring are independently a hydrogen atom, a substituted or unsubstituted alkyl group including 1 to 50 carbon atoms or a substituted or unsubstituted aryl group including 6 to 50 ring carbon atoms.

In one embodiment, the compound (10) is a compound represented by the following formula (10-4A): 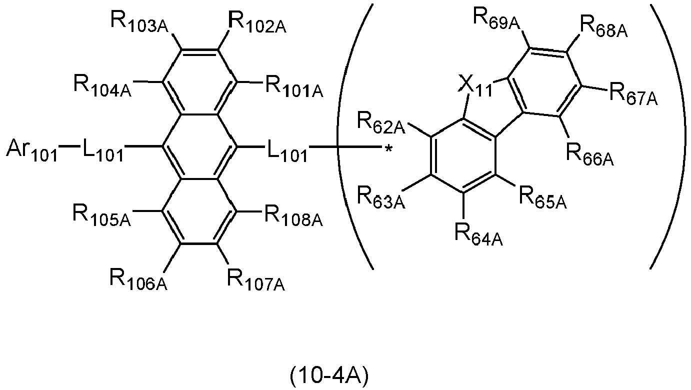 wherein in the formula (10-4A),
L₁₀₁ and Ar₁₀₁ are as defined in the formula (10);
R_{101A} to R_{108A} are independently a hydrogen atom or a substituted or unsubstituted aryl group including 6 to 50 ring carbon atoms;
X₁₁ is O, S or N(R₆₁);
R₆₁ is a hydrogen atom, a substituted or unsubstituted alkyl group including 1 to 50 carbon atoms or a substituted or unsubstituted aryl group including 6 to 50 ring carbon atoms;
one or more pairs of adjacent two or more of R_{62A} to R_{69A} may form a substituted or unsubstituted, saturated or unsaturated ring, and adjacent two of R_{62A} to R_{69A} form a ring represented by the following formula (10-4A-1); and
R_{62A} to R_{69A} that do not form a substituted or unsubstituted, saturated or unsaturated ring are independently a hydrogen atom, a substituted or unsubstituted alkyl group including 1 to 50 carbon atoms or a substituted or unsubstituted aryl group including 6 to 50 ring carbon atoms. 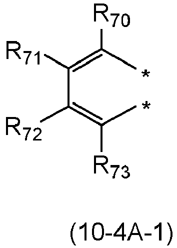
wherein in the formula (10-4A-1),
each of the two atomic bondings * is bonded with adjacent two of R_{62A} to R_{69A};
one of R₇₀ to R₇₃ is an atomic bonding that is bonded with L₁₀₁; and
R₇₀ to R₇₃ that are not bonded with L₁₀₁ are independently a hydrogen atom, a substituted or unsubstituted alkyl group including 1 to 50 carbon atoms or a substituted or unsubstituted aryl group including 6 to 50 ring carbon atoms.

In one embodiment, the compound (10) is a compound represented by the following formula (10-6): 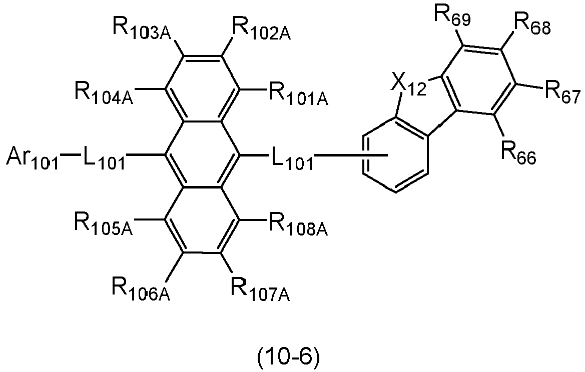 wherein in the formula (10-6),
L₁₀₁ and Ar₁₀₁ are as defined in the formula (10);
R_{101A} to R_{108A} are as defined in the formula (10-4);
R₆₆ to R₆₉ are as defined in the formula (10-4); and
X₁₂ is O or S.

In one embodiment, the compound represented by the formula (10-6) is a compound represented by the following formula (10-6H): 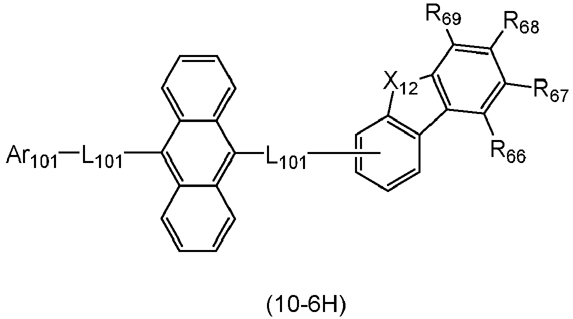 wherein in the formula (10-6H),
L₁₀₁ and Ar₁₀₁ are as defined in the formula (10);
R₆₆ to R₆₉ are as defined in the formula (10-4); and
X₁₂ is O or S.

In one embodiment, the compound represented by the formulas (10-6) and (10-6H) is a compound represented by the following formula (10-6Ha): 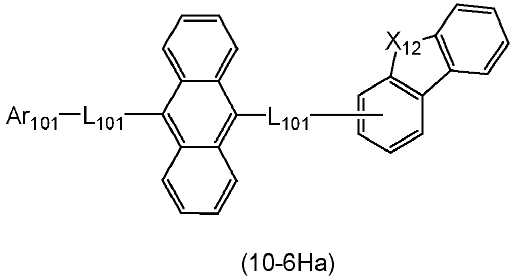 wherein in the formula (10-6Ha),
L₁₀₁ and Ar₁₀₁ are as defined in the formula (10); and
X₁₂ is O or S.

In one embodiment, the compound represented by the formulas (10-6), (10-6H) and (10-6Ha) is a compound represented by the following formula (10-6Ha-1) or (10-6Ha-2): 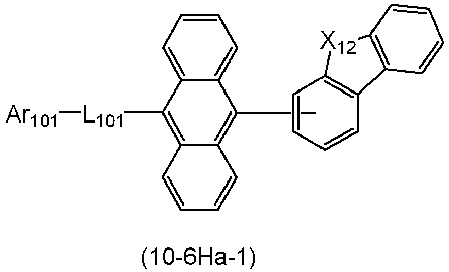 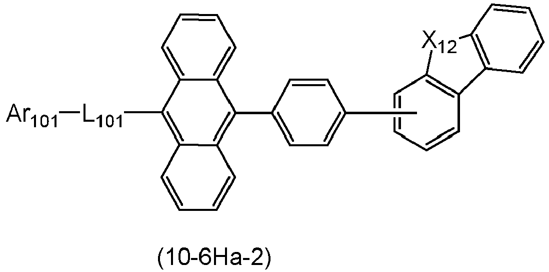 wherein in the formula (10-6Ha-1) and (10-6Ha-2),
L₁₀₁ and Ar₁₀₁ are as defined in the formula (10); and
X₁₂ is O or S.

In one embodiment, the compound (10) is a compound represented by the following formula (10-7): 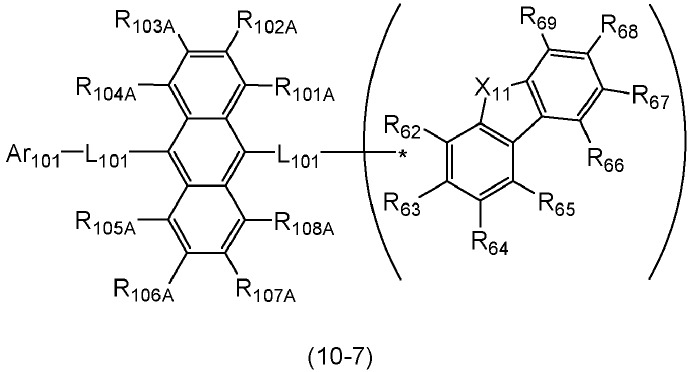 wherein in the formula (10-7),
L₁₀₁ and Ar₁₀₁ are as defined in the formula (10);
R_{101A} to R_{108A} are as defined in the formula (10-4);
X₁₁ is as defined in the formula (10-4); and
R₆₂ to R₆₉ are as defined in the formula (10-4), provided that any one pair of R₆₆ and R₆₇, R₆₇ and R₆₈, and R₆₈ and R₆₉ are bonded with each other to form a substituted or unsubstituted, saturated or unsaturated ring.

In one embodiment, the compound (10) is a compound represented by the following formula (10-7H): 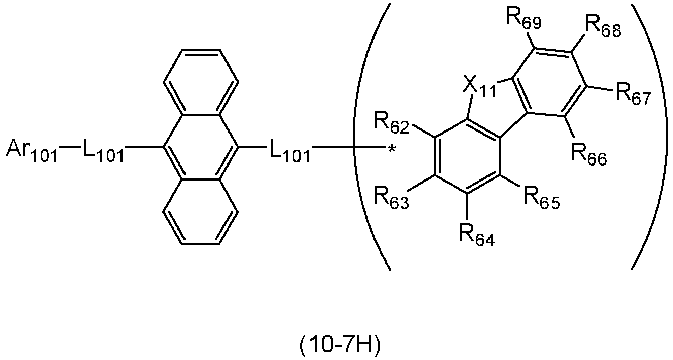 wherein in the formula (10-7H),
L₁₀₁ and Ar₁₀₁ are as defined in the formula (10);
X₁₁ is as defined in the formula (10-4); and
R₆₂ to R₆₉ are as defined in the formula (10-4), provided that any one pair of R₆₆ and R₆₇, R₆₇ and R₆₈, and R₆₈ and R₆₉ are bonded with each other to form a substituted or unsubstituted, saturated or unsaturated ring.

In one embodiment, the compound (10) is a compound represented by the following formula (10-8): 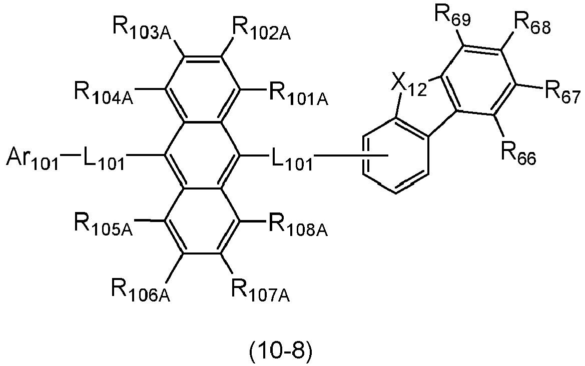 wherein in the formula (10-8),
L₁₀₁ and Ar₁₀₁ are as defined in the formula (10);
R_{101A} to R_{108A} are as defined in the formula (10-4);
X₁₂ is O or S; and
R₆₆ to R₆₉ are as defined in the formula (10-4), provided that any one pair of R₆₆ and R₆₇, R₆₇ and R₆₈, as well as R₆₈ and R₆₉ are bonded with each other to form a substituted or unsubstituted, saturated or unsaturated ring.

In one embodiment, the compound represented by the formula (10-8) is a compound represented by the following formula (10-8H):

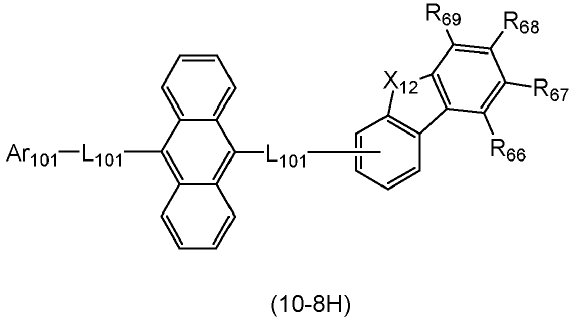

In the formula (10-8H), L₁₀₁ and Ar₁₀₁ are as defined in the formula (10).
R₆₆ to R₆₉ are as defined in the formula (10-4), provided that any one pair of R₆₆ and R₆₇, R₆₇ and R₆₈, as well as R₆₈ and R₆₉ are bonded with each other to form a substituted or unsubstituted, saturated or unsaturated ring. Any one pair of R₆₆ and R₆₇, R₆₇ and R₆₈, as well as R₆₈ and R₆₉ may preferably be bonded with each other to form an unsubstituted benzene ring; and
X₁₂ is O or S.

In one embodiment, as for the compound represented by the formula (10-7), (10-8) or (10-8H), any one pair of R₆₆ and R₆₇, R₆₇ and R₆₈, as well as R₆₈ and R₆₉ are bonded with each other to form a ring represented by the following formula (10-8-1) or (10-8-2), and R₆₆ to R₆₉ that do not form the ring represented by the formula (10-8-1) or (10-8-2) do not form a substituted or unsubstituted, saturated or unsaturated ring. 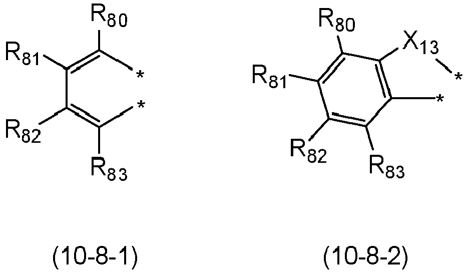 wherein in the formulas (10-8-1) and (10-8-2),
the two atomic bondings * are independently bonded with one pair of R₆₆ and R₆₇, R₆₇ and R₆₈, or R₆₈ and R₆₉;
R₈₀ to R₈₃ are independently a hydrogen atom, a substituted or unsubstituted alkyl group including 1 to 50 carbon atoms or a substituted or unsubstituted aryl group including 6 to 50 ring carbon atoms; and
X₁₃ is O or S.

In one embodiment, the compound (10) is a compound represented by the following formula (10-9): 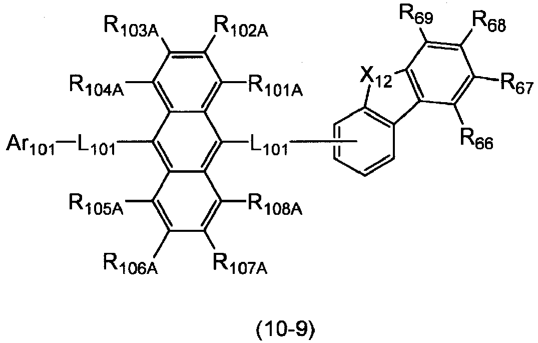 wherein in the formula (10-9),
L₁₀₁ and Ar₁₀₁ are as defined in the formula (10);
R_{101A} to R_{108A} are as defined in the formula (10-4);
R₆₆ to R₆₉ are as defined in the formula (10-4), provided that R₆₆ and R₆₇, R₆₇ and R₆₈, as well as R₆₈ and R₆₉ are not bonded with each other and do not form a substituted or unsubstituted, saturated or unsaturated ring; and
X₁₂ is O or S.

In one embodiment, the compound (10) is selected from the group consisting of compounds represented by the following formulas (10-10-1) to (10-10-4).

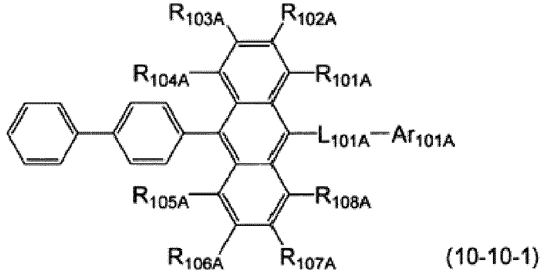

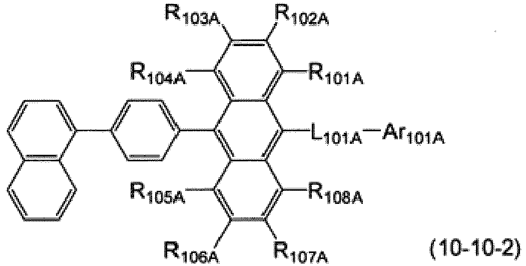

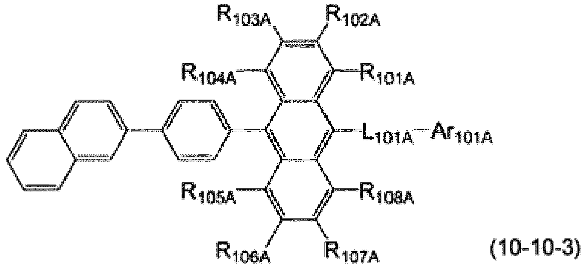

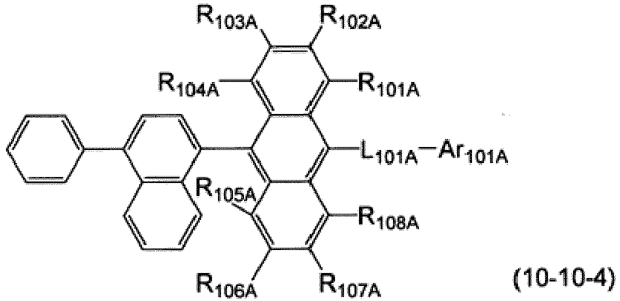

In the formulae (10-10-1H) to (10-10-4H), L_{101A} and Ar_{101A} are as defined in the formula (10-3).

As for the compound represented by the formula (10), the following compounds can be given as specific examples.

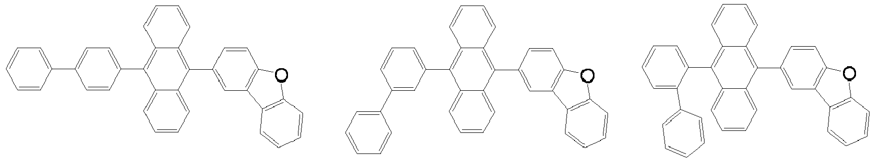

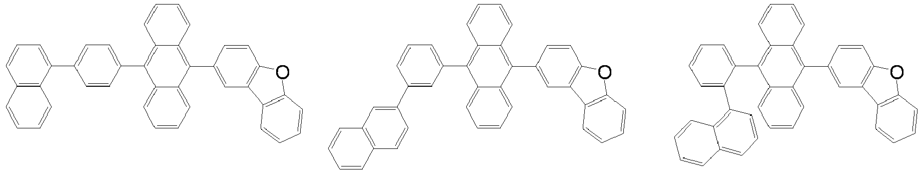

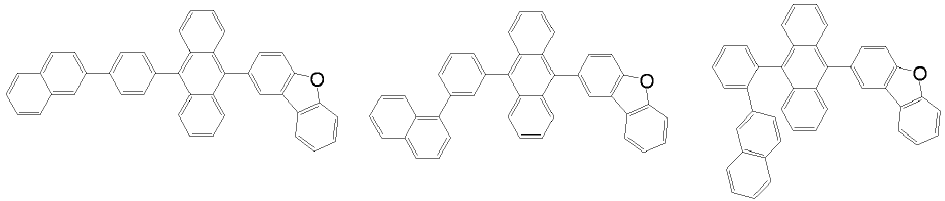

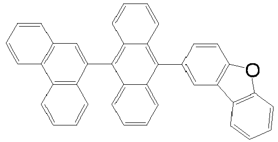

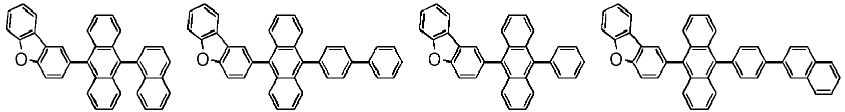

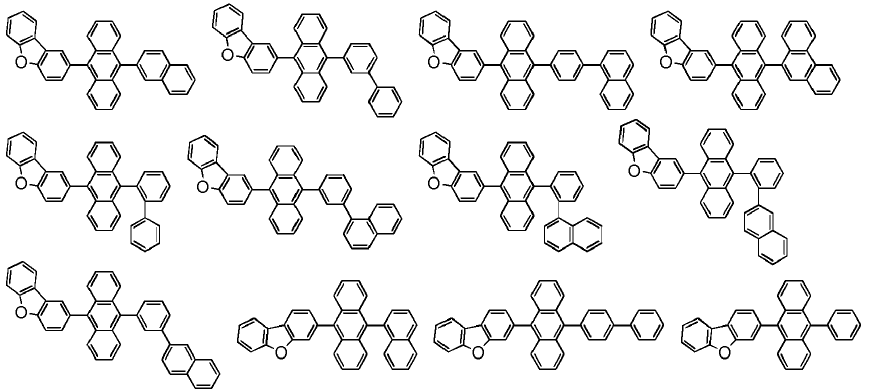

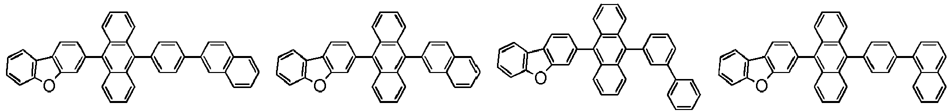

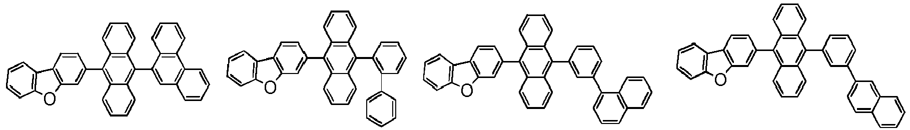

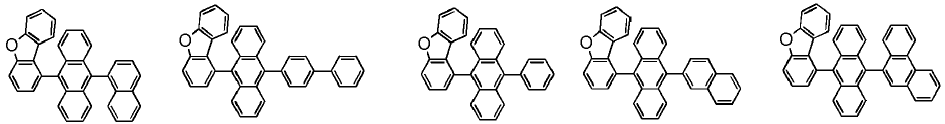

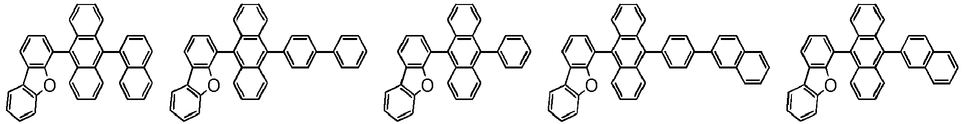

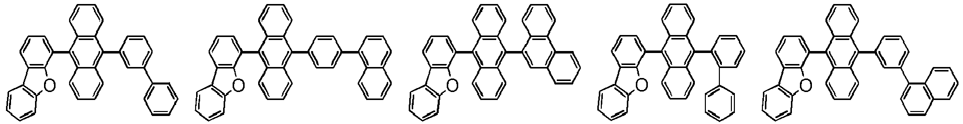

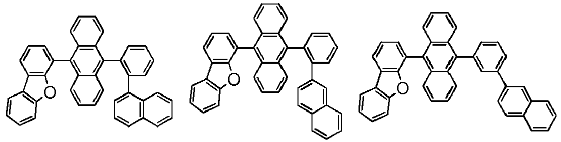

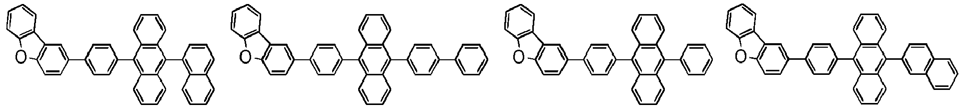

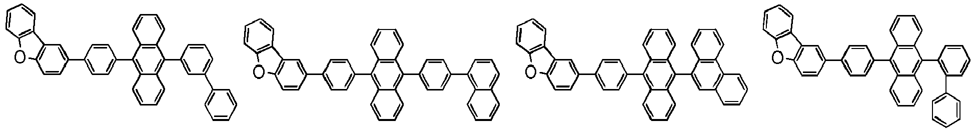

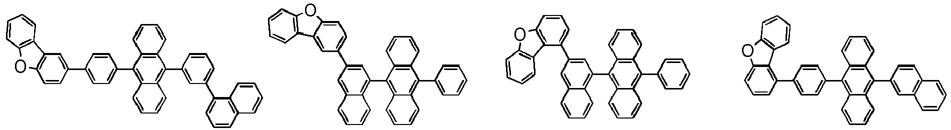

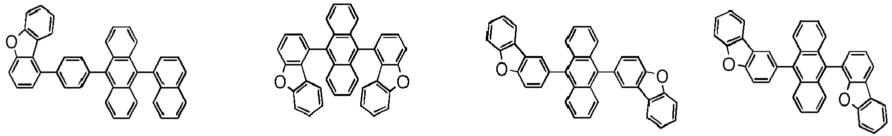

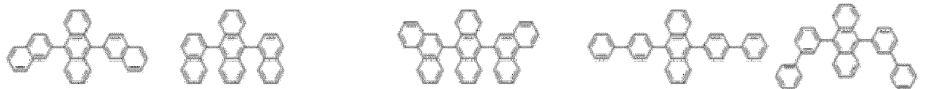

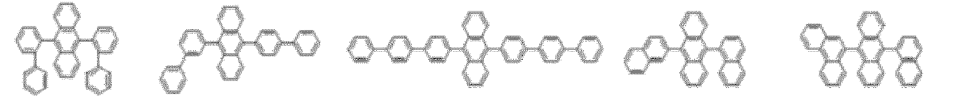

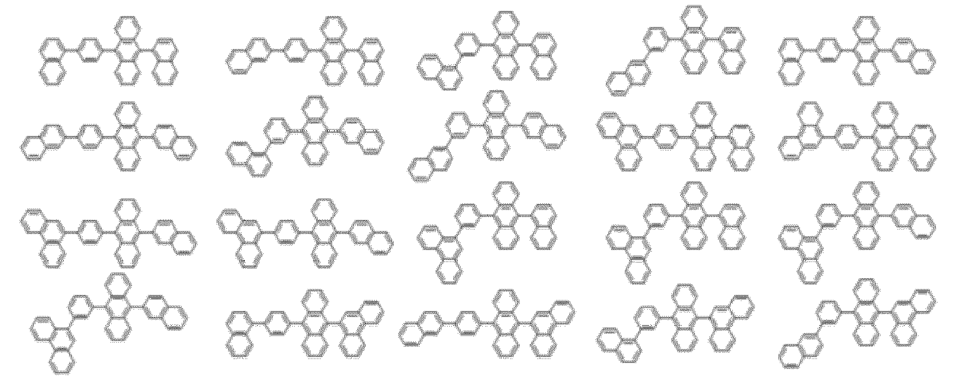

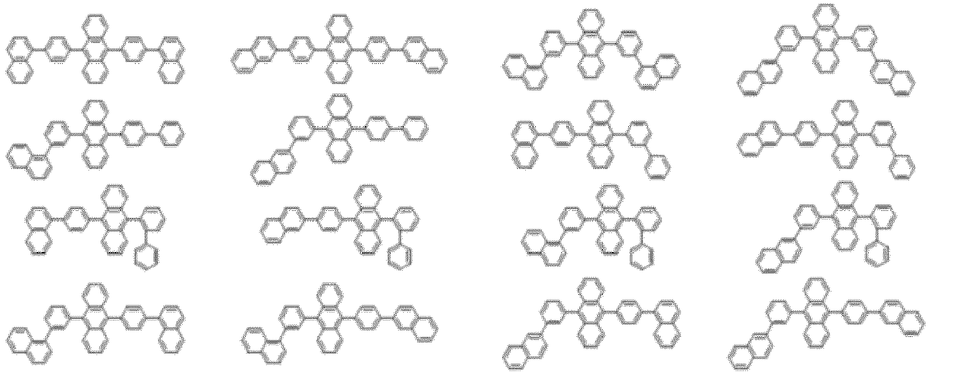

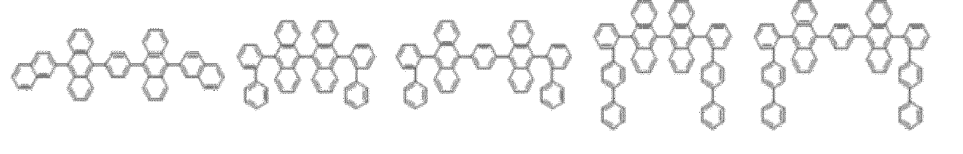

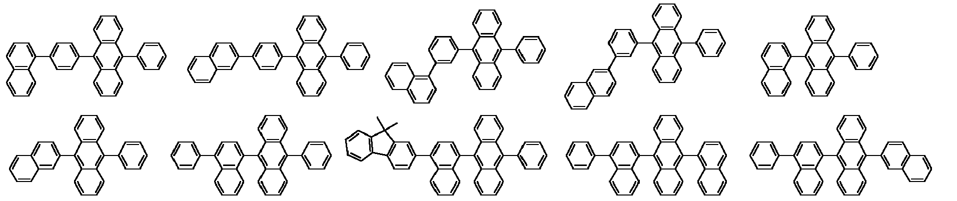

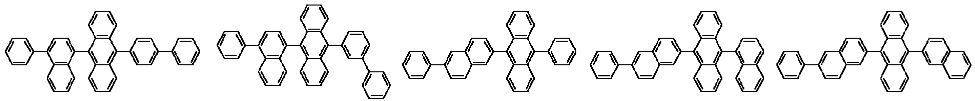

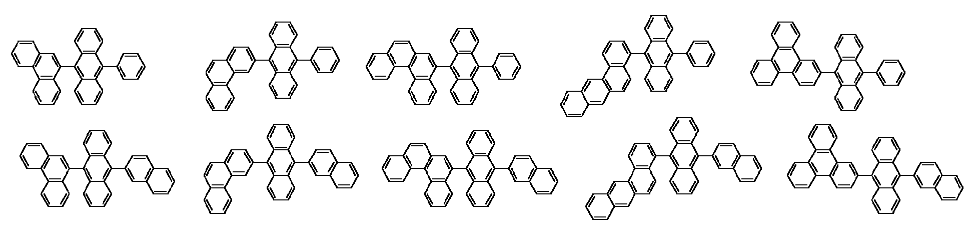

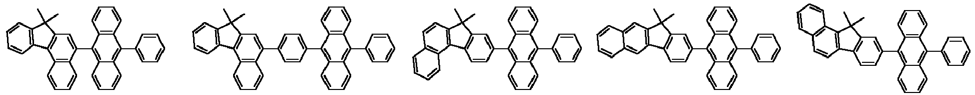

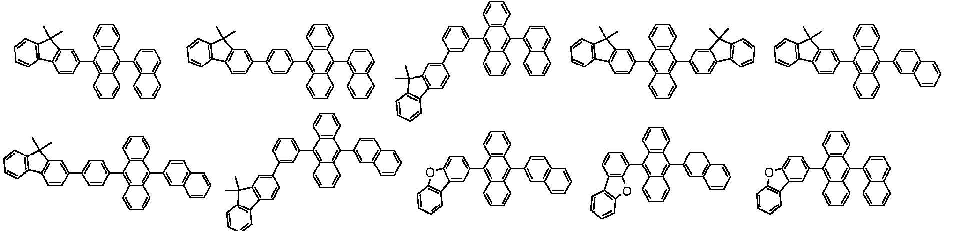

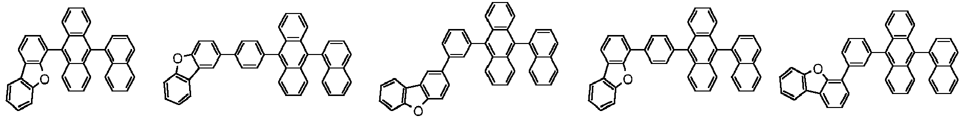

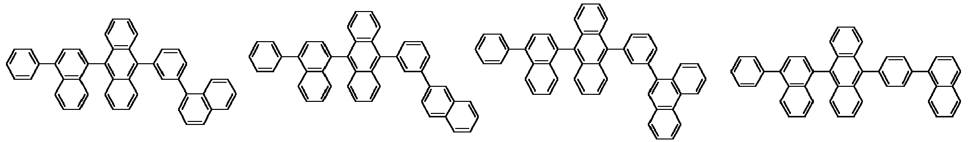

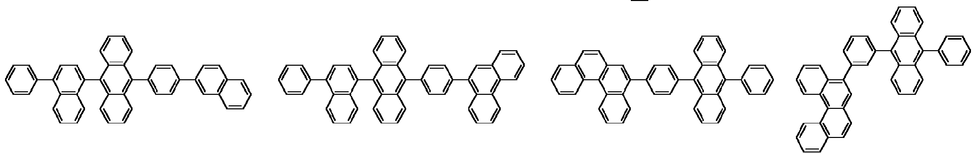

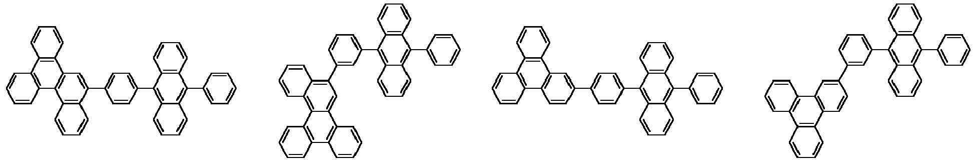

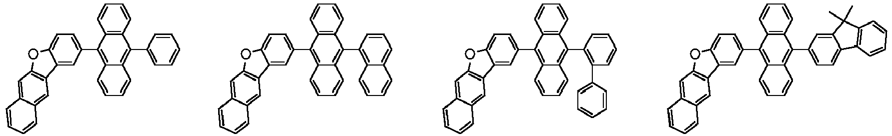

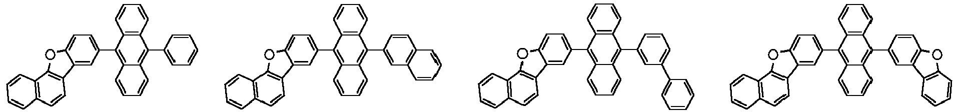

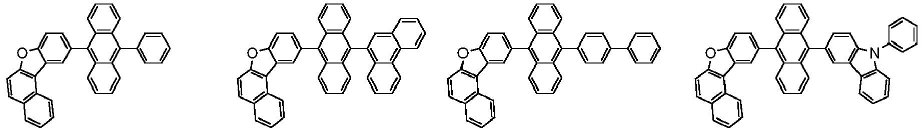

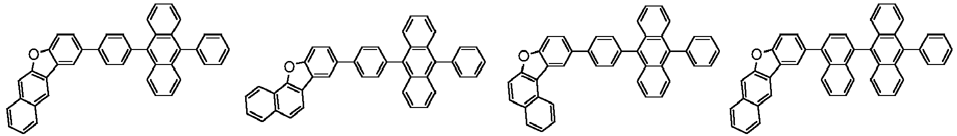

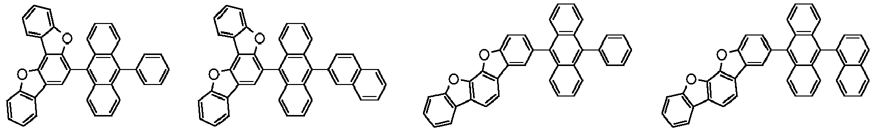

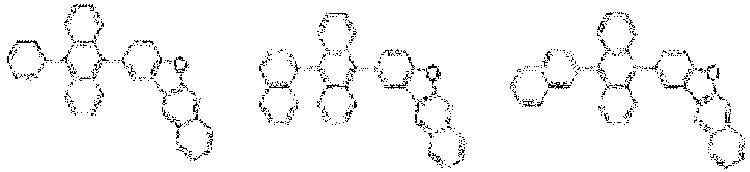

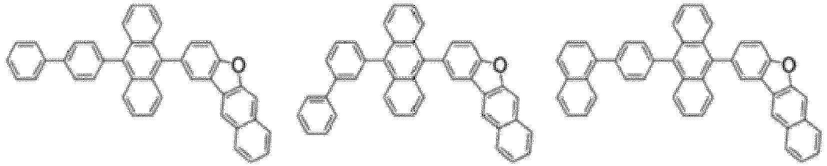

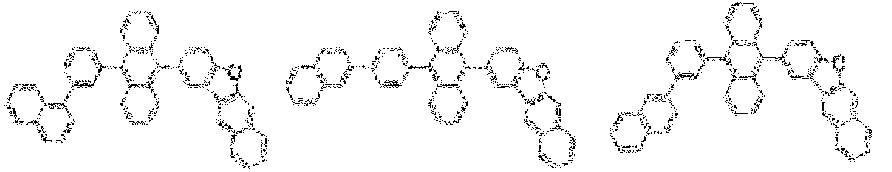

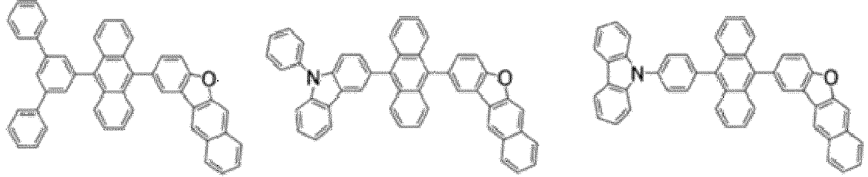

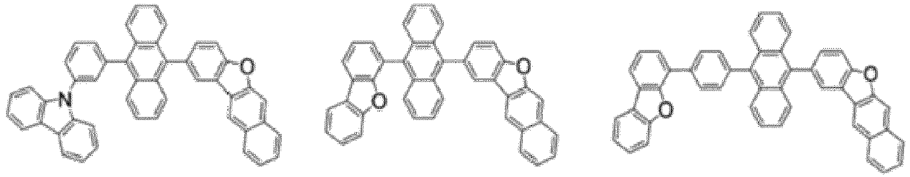

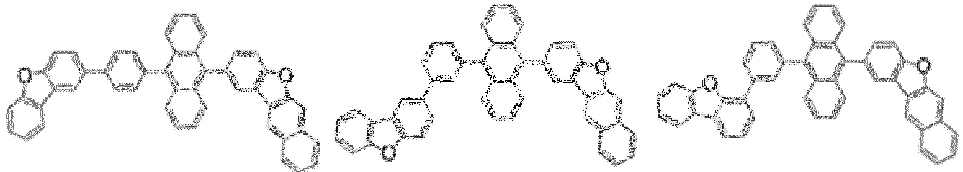

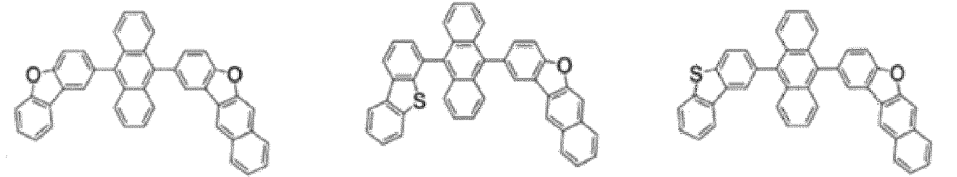

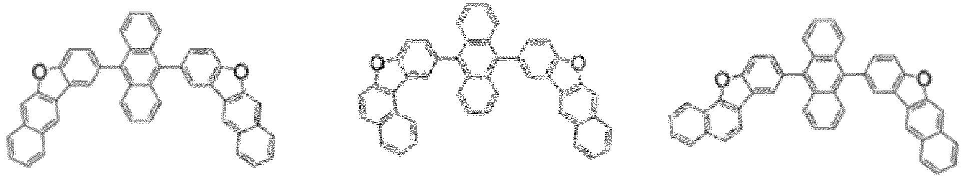

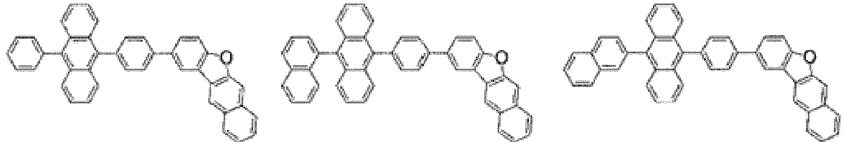

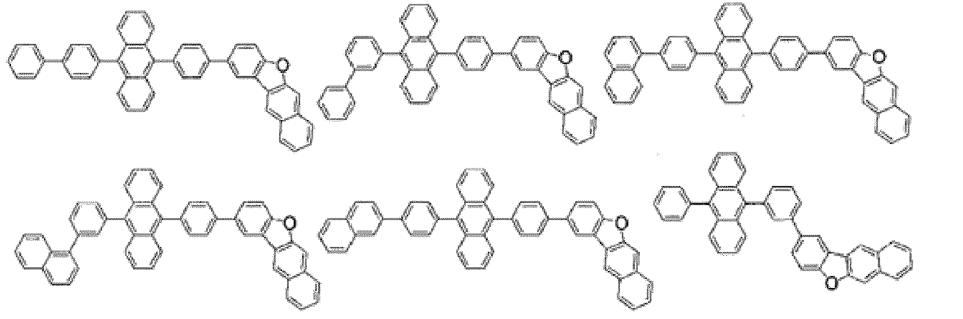

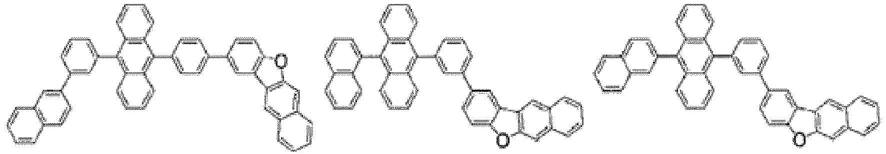

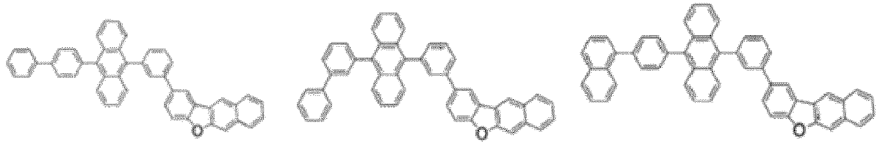

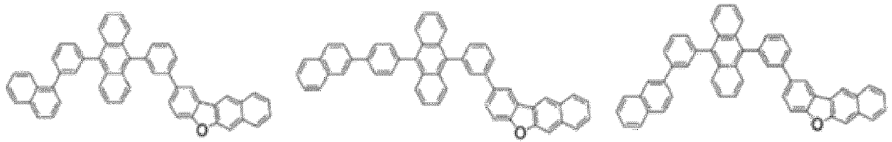

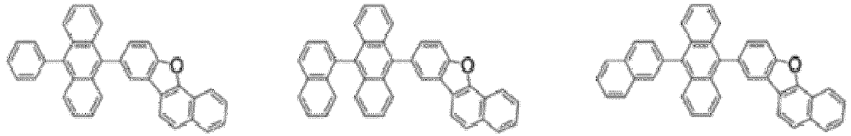

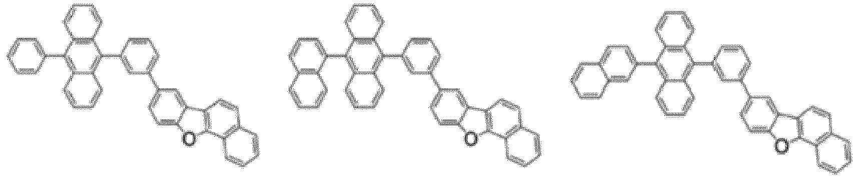

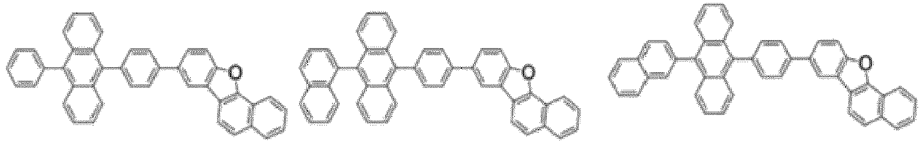

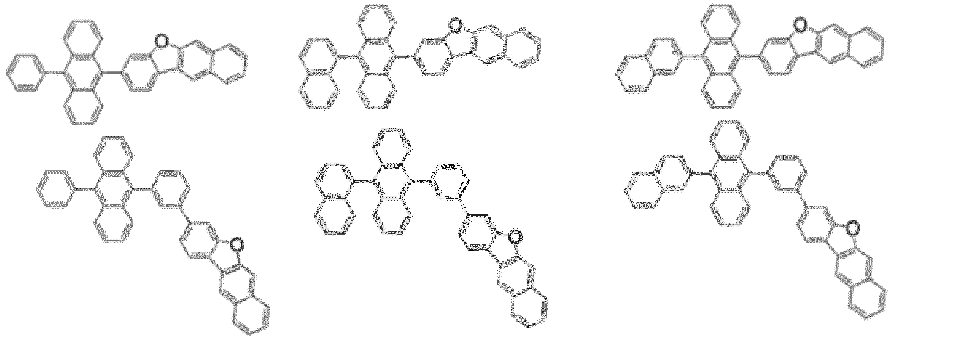

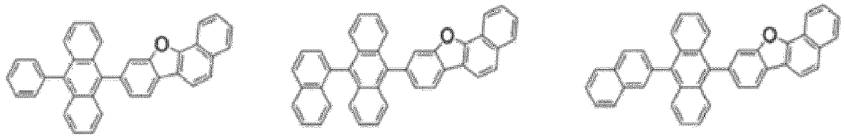

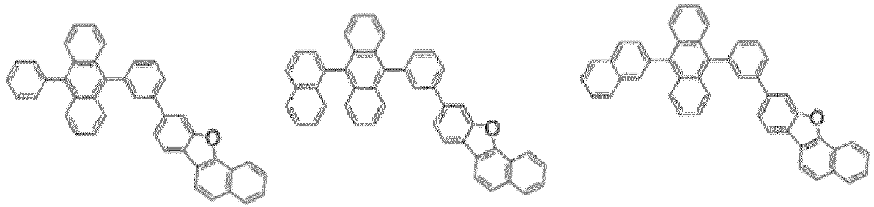

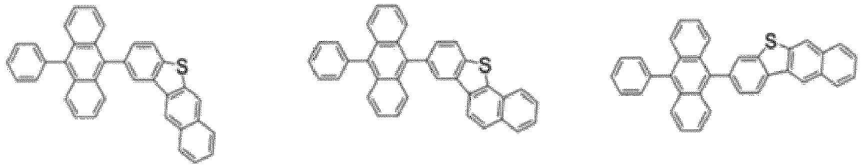

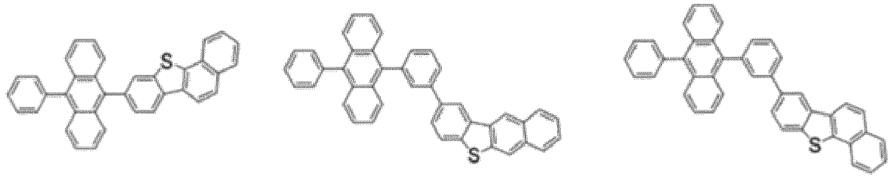

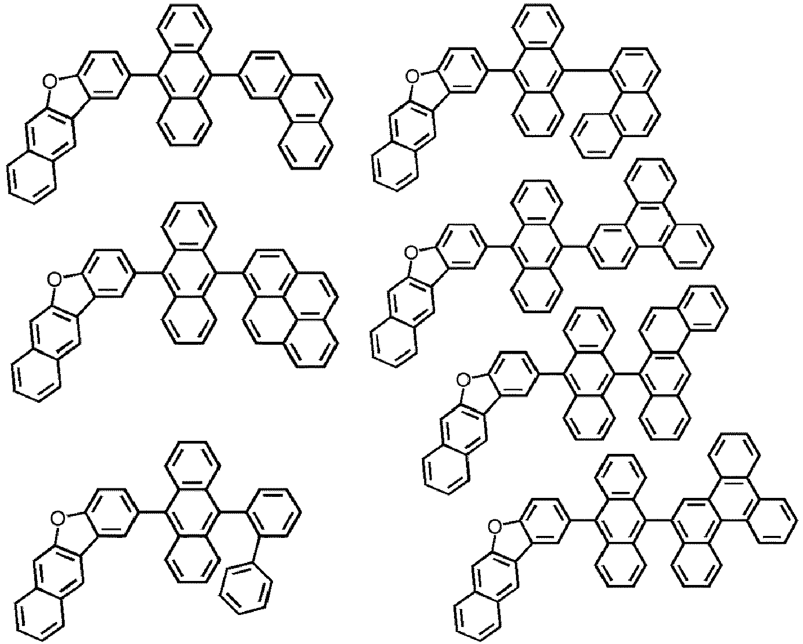

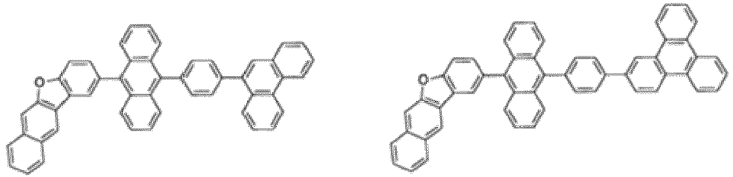

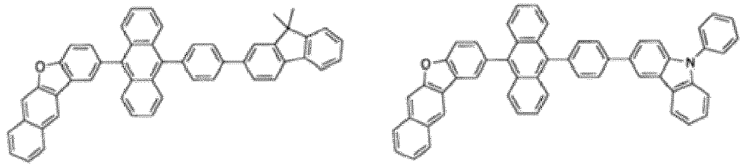

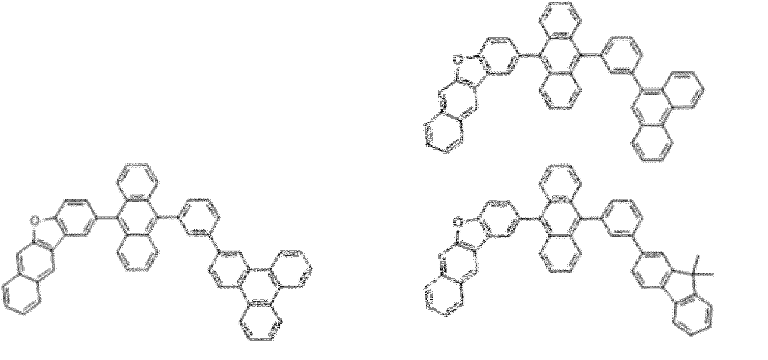

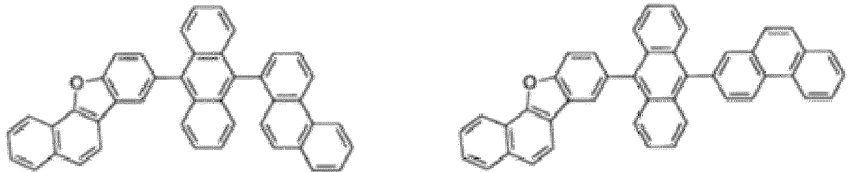

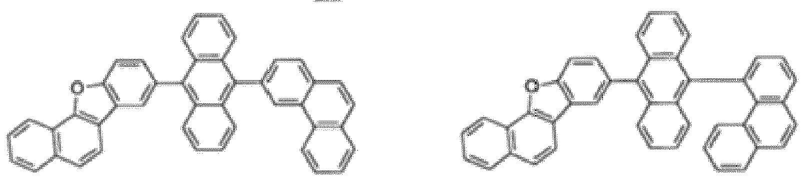

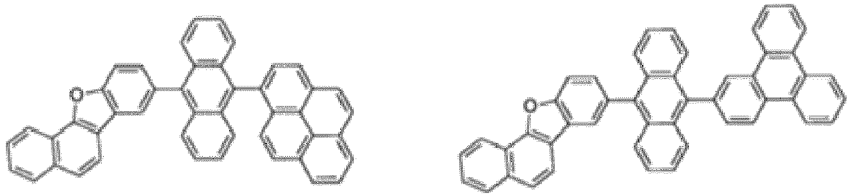

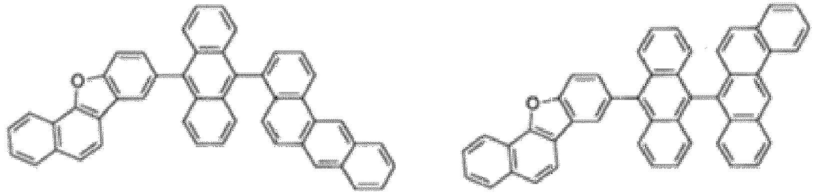

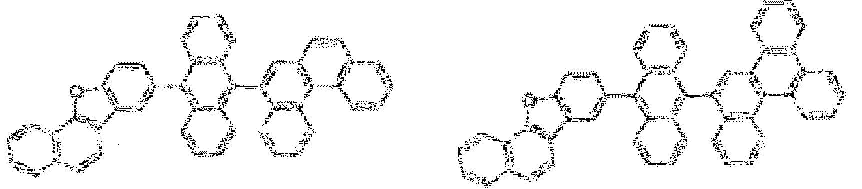

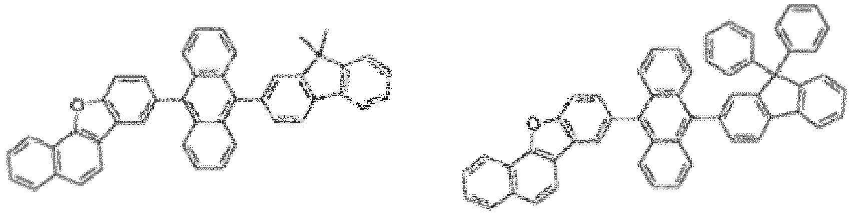

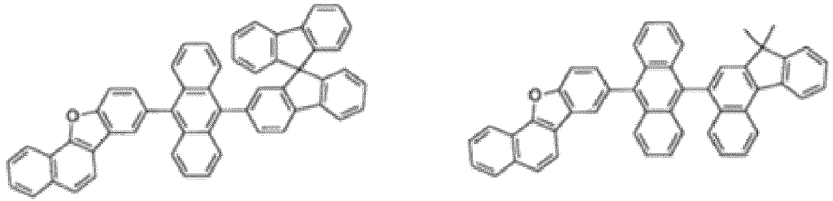

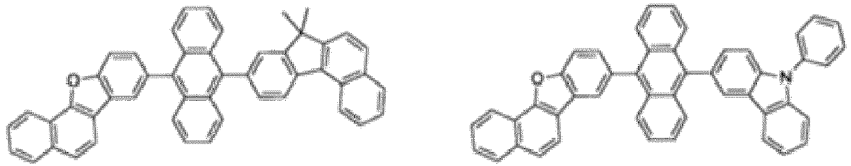

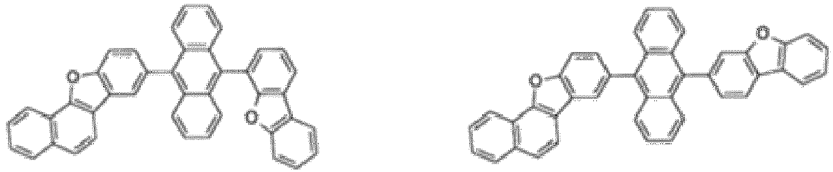

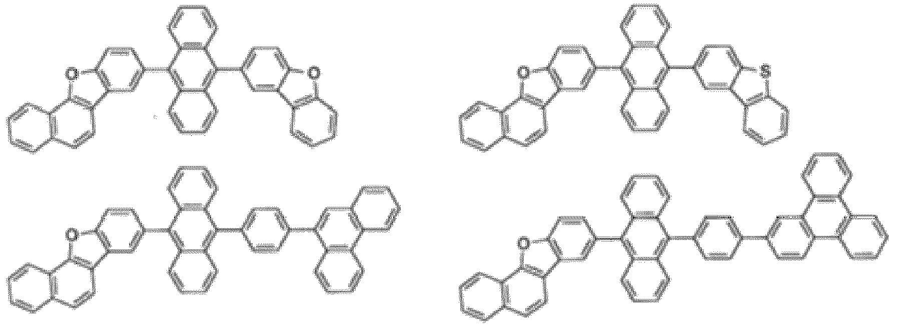

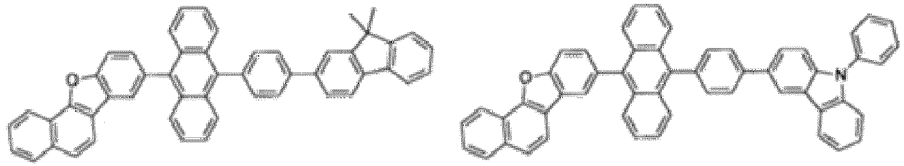

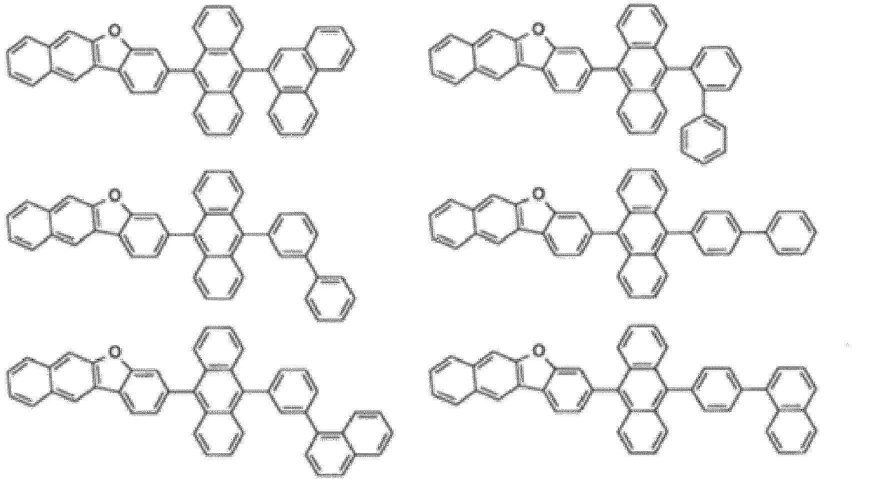

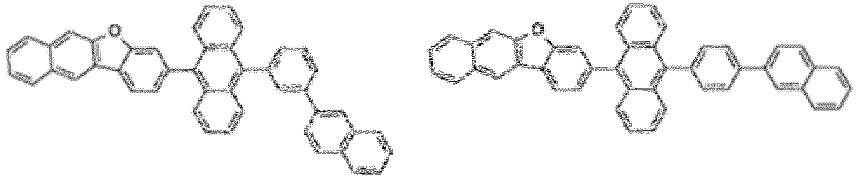

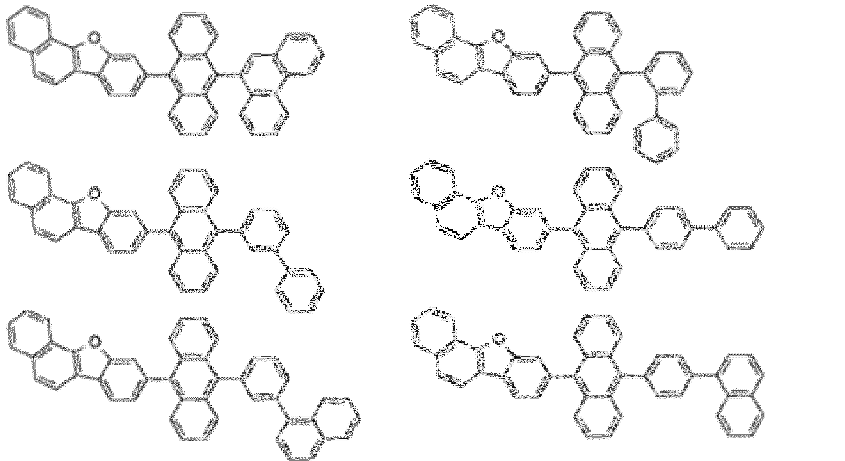

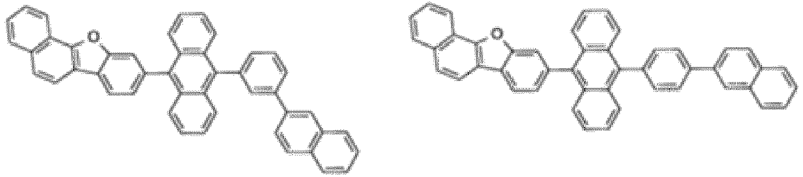

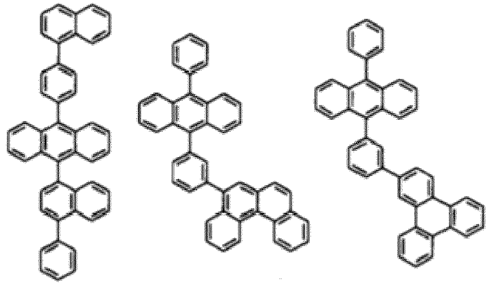

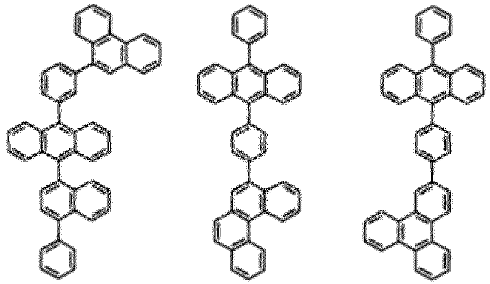

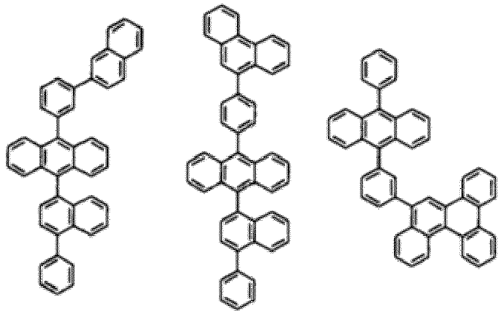

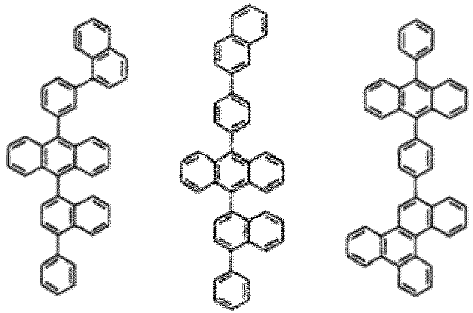

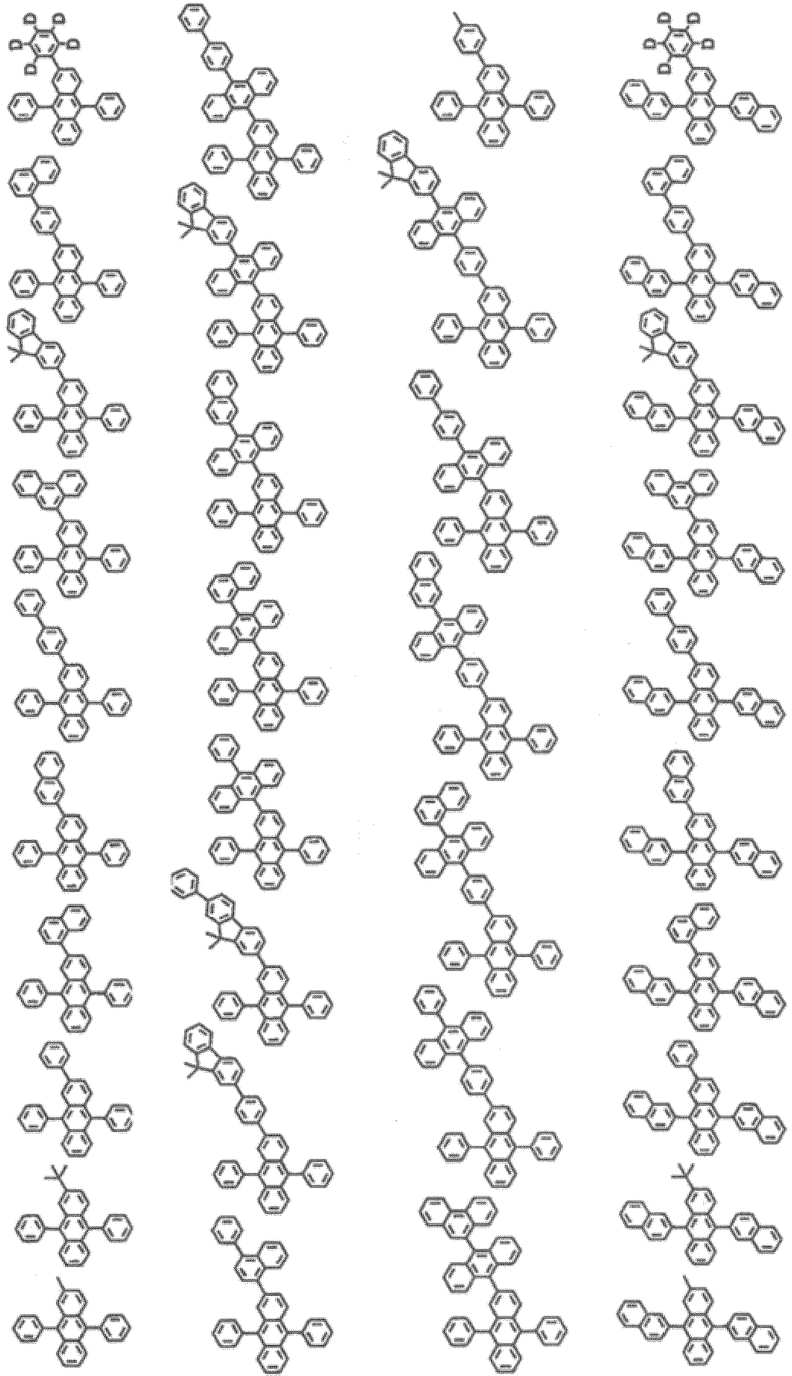

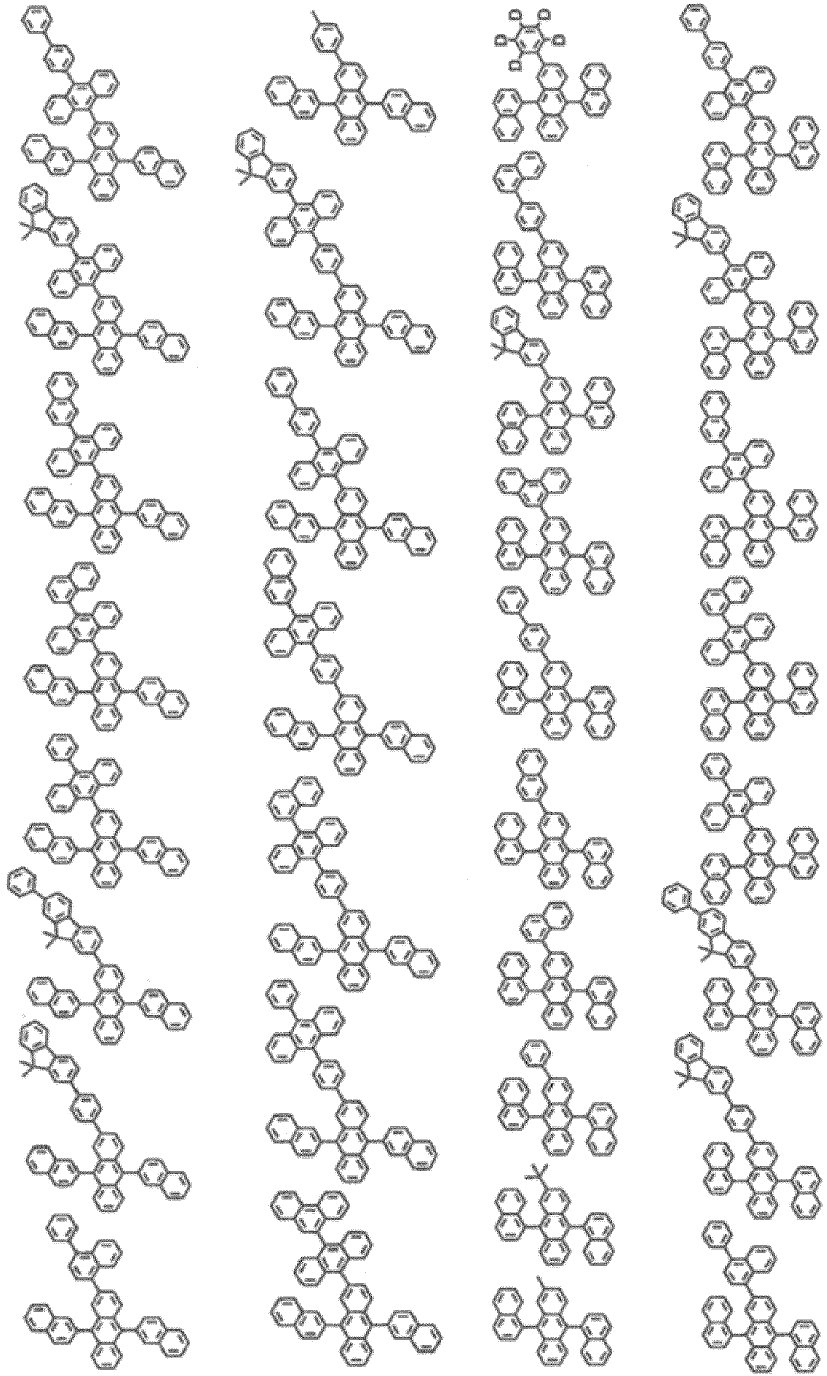

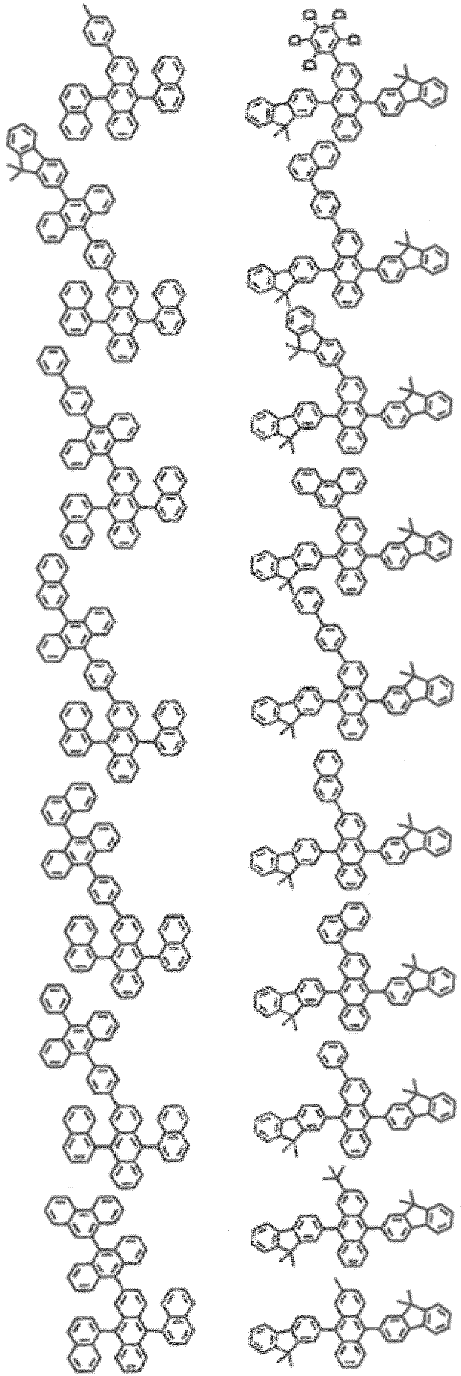

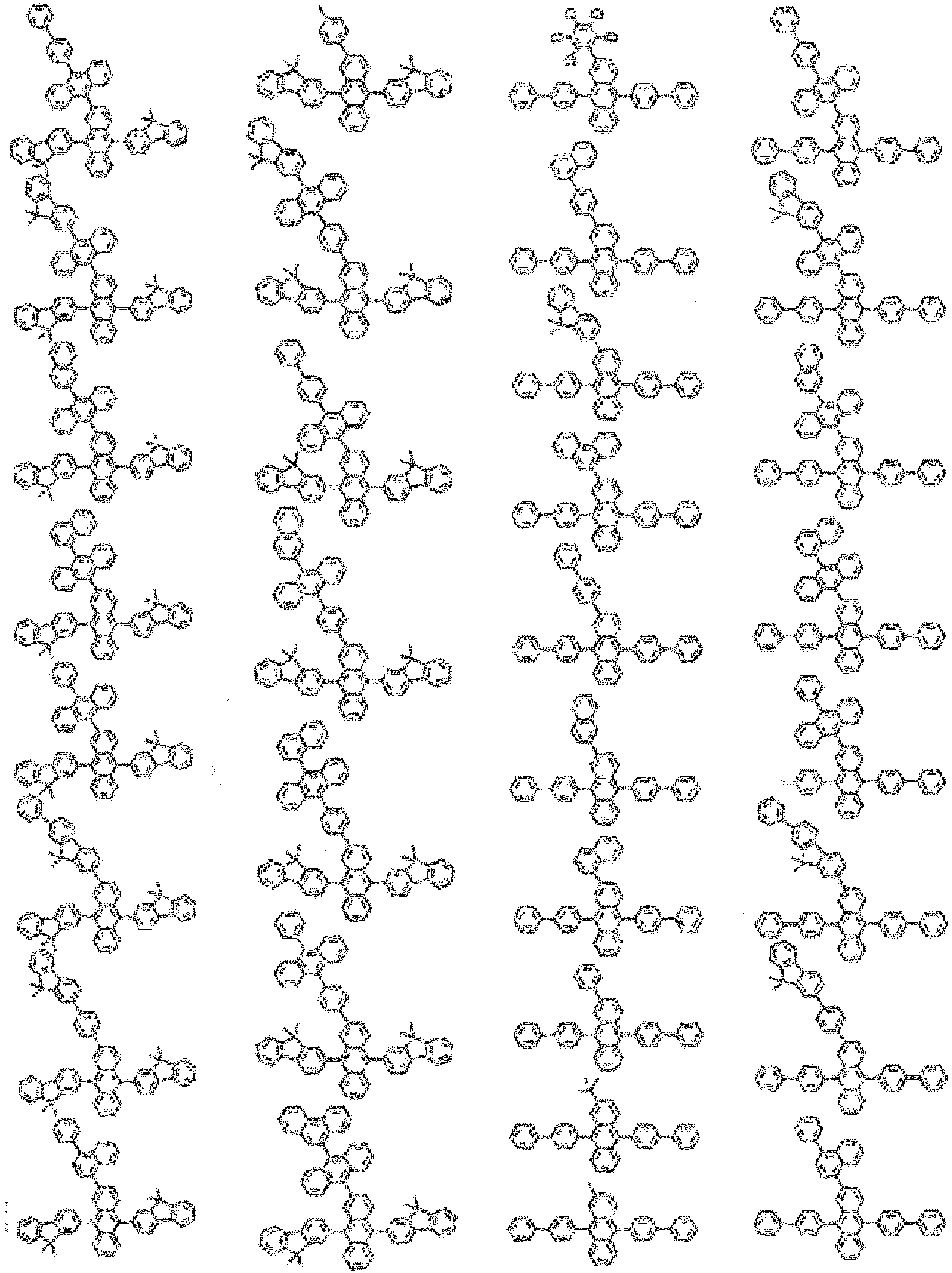

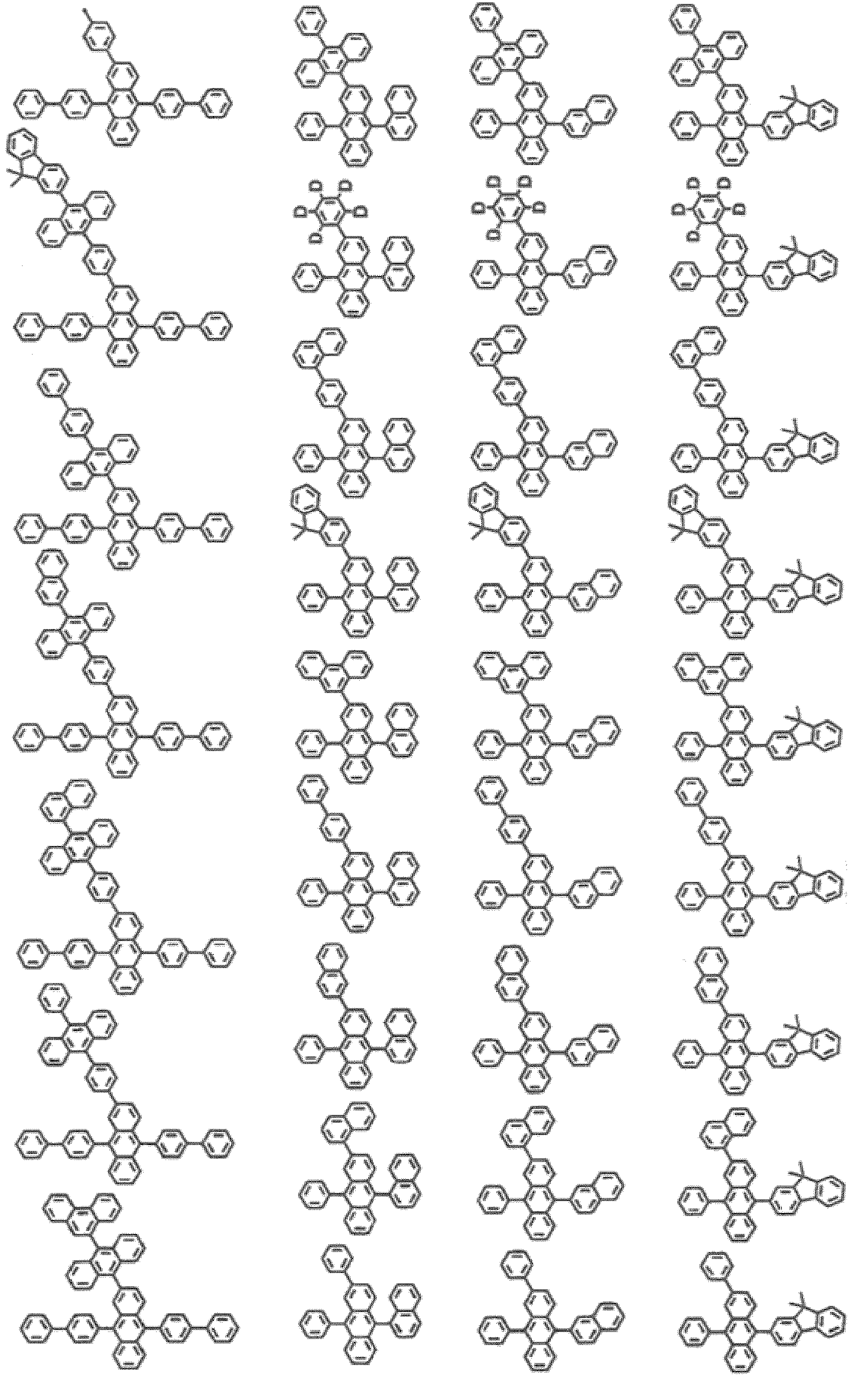

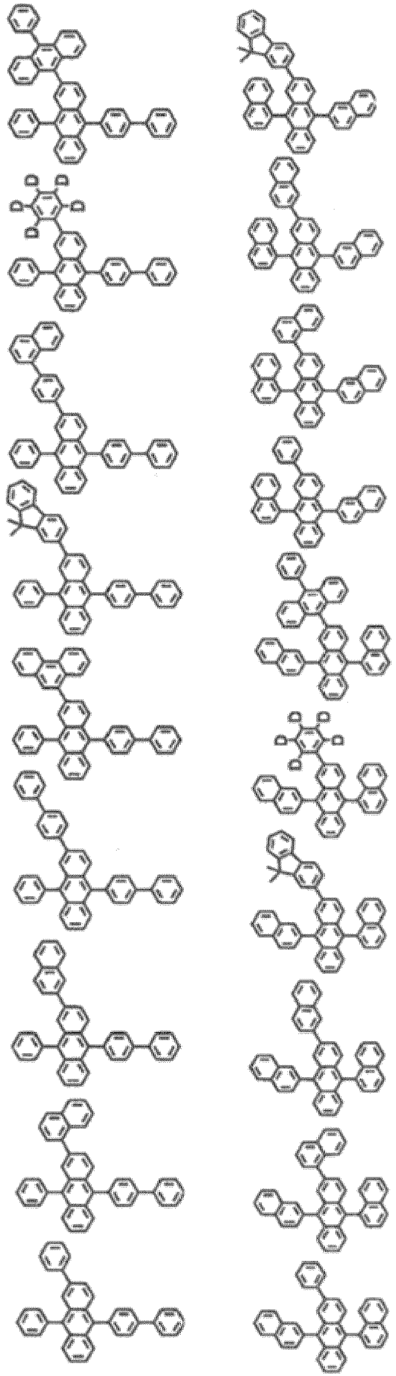

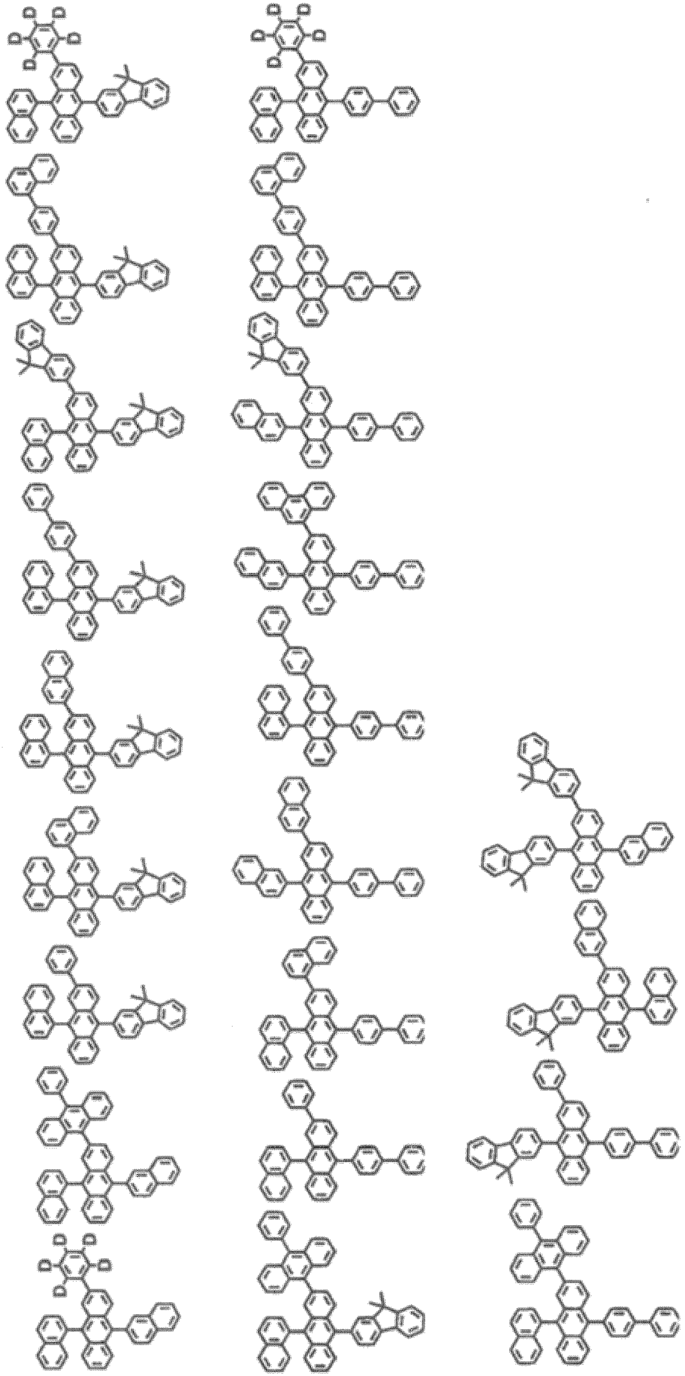

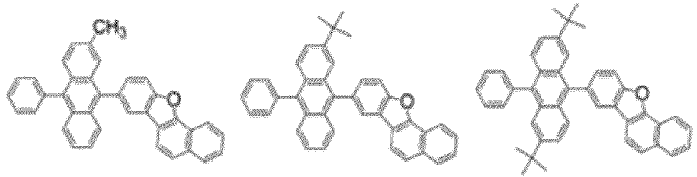

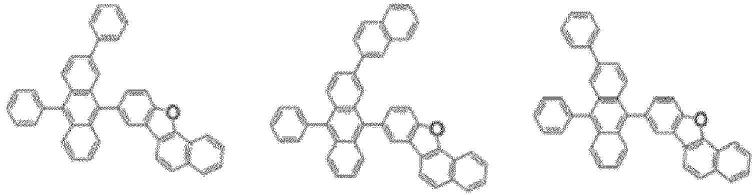

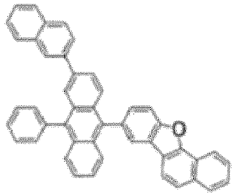

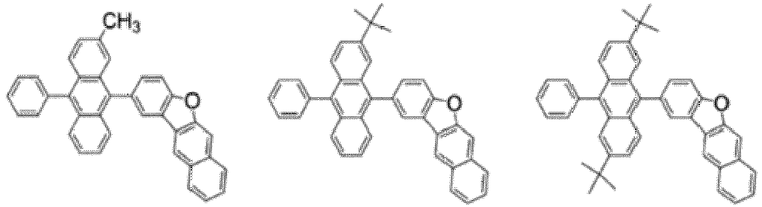

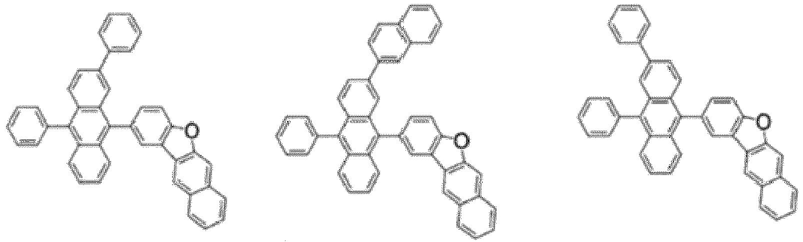

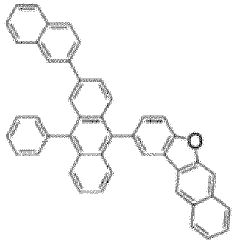

In the case that the emitting layer comprises the compound represented by formula (I) as a dopant and at least one host, wherein preferred hosts are mentioned above, and the host is more preferably at least one compound represented by formula (10), the content of the at least one compound represented by formula (I) is preferably 0.5 mass% to 70 mass%, more preferably 0.5 to 30 mass%, further preferably 1 to 30 mass%, still further preferably 1 to 20 mass%, and particularly preferably 1 to 10 mass%, further particularly preferably 1 to 5 mass%, relative to the entire mass of the emitting layer.
The content of the at least one host, wherein preferred hosts are mentioned above, preferably the at least one compound represented by formula (10) is preferably 30 mass% to 99.9 mass%, more preferably 70 to 99.5 mass%, further preferably 70 to 99 mass%, still further preferably 80 to 99 mass%, and particularly preferably 90 to 99 mass%, further particularly preferably 95 to 99 mass %, relative to the entire mass of the emitting layer.

**An explanation will be made on the layer configuration of the organic EL device according to one aspect of the invention.**

An organic EL device according to one aspect of the invention comprises a cathode, an anode, and one or more organic thin film layers comprising an emitting layer disposed between the cathode and the anode. The organic layer comprises at least one layer composed of an organic compound. Alternatively, the organic layer is formed by laminating a plurality of layers composed of an organic compound. The organic layer may further comprise an inorganic compound in addition to the organic compound.

At least one of the organic layers is an emitting layer. The organic layer may be constituted, for example, as a single emitting layer, or may comprise other layers which can be adopted in the layer structure of the organic EL device. The layer that can be adopted in the layer structure of the organic EL device is not particularly limited, but examples thereof include a hole-transporting zone (comprising at least one hole-transporting layer and preferably in addition at least one of a hole-injecting layer, an electron-blocking layer, an exciton-blocking layer, etc.), an emitting layer, a spacing layer, and an electron-transporting zone (comprising at least one electron-transporting layer and preferably in addition at least one of an electron-injecting layer, a hole-blocking layer, etc.) provided between the cathode and the emitting layer.

The organic EL device according to one aspect of the invention may be, for example, a fluorescent or phosphorescent monochromatic light emitting device or a fluorescent/phosphorescent hybrid white light emitting device. Preferably, the organic EL device is a fluorescent monochromatic light emitting device, more preferably a blue fluorescent monochromatic light emitting device or a fluorescent/phosphorescent hybrid white light emitting device. Blue fluorescence means a fluorescence at 400 to 500 nm (peak maximum), preferably at 430 nm to 490 nm (peak maximum).

Further, it may be a simple type device having a single emitting unit or a tandem type device having a plurality of emitting units.

The "emitting unit" in the specification is the smallest unit that comprises organic layers, in which at least one of the organic layers is an emitting layer and light is emitted by recombination of injected holes and electrons.

In addition, the "emitting layer" described in the present specification is an organic layer having an emitting function. The emitting layer is, for example, a phosphorescent emitting layer, a fluorescent emitting layer or the like, preferably a fluorescent emitting layer, more preferably a blue fluorescent emitting layer, and may be a single layer or a stack of a plurality of layers.

The emitting unit may be a stacked type unit having a plurality of phosphorescent emitting layers or fluorescent emitting layers. In this case, for example, a spacing layer for preventing excitons generated in the phosphorescent emitting layer from diffusing into the fluorescent emitting layer may be provided between the respective light-emitting layers.

As the simple type organic EL device, a device configuration such as anode/emitting unit/cathode can be given.

Examples for representative layer structures of the emitting unit are shown below. The layers in parentheses are provided arbitrarily.
(a) (Hole-injecting layer/) Hole-transporting layer/Fluorescent emitting layer (/Electron-transporting layer/Electron-injecting layer)
(b) (Hole-injecting layer/) Hole-transporting layer/Phosphorescent emitting layer (/Electron-transporting layer/Electron-injecting layer)
(c) (Hole-injecting layer/) Hole-transporting layer/First fluorescent emitting layer/Second fluorescent emitting layer (/Electron-transporting layer/Electron-injecting layer)
(d) (Hole-injecting layer/) Hole-transporting layer/First phosphorescent layer/Second phosphorescent layer (/Electron-transporting layer/Electron-injecting layer)
(e) (Hole-injecting layer/) Hole-transporting layer/Phosphorescent emitting layer/Spacing layer /Fluorescent emitting layer (/Electron-transporting layer/Electron-injecting layer)
(f) (Hole-injecting layer/) Hole-transporting layer/First phosphorescent emitting layer/Second phosphorescent emitting layer/Spacing layer/Fluorescent emitting layer (/Electron-transporting layer/Electron-injecting layer)
(g) (Hole-injecting layer/) Hole-transporting layer/First phosphorescent layer/Spacing layer/ Second phosphorescent emitting layer/Spacing layer/Fluorescent emitting layer (/Electron-transporting layer / Electron-injecting layer)
(h) (Hole-injecting layer/) Hole-transporting layer/Phosphorescent emitting layer/Spacing layer/First fluorescent emitting layer/Second fluorescent emitting layer (/Electron-transporting Layer/Electron-injecting Layer)
(i) (Hole-injecting layer/) Hole-transporting layer/Electron-blocking layer/Fluorescent emitting layer (/Electron-transporting layer/Electron-injecting layer)
(j) (Hole-injecting layer/) Hole-transporting layer/Electron-blocking layer/Phosphorescent emitting layer (/Electron-transporting layer /Electron-injecting layer)
(k) (Hole-injecting layer/) Hole-transporting layer/Exciton-blocking layer/Fluorescent emitting layer (/Electron-transporting layer/Electron-injecting layer)
(l) (Hole-injecting layer/) Hole-transporting layer/Exciton-blocking layer/Phosphorescent emitting layer (/Electron-transporting layer/Electron-injecting layer)
(m) (Hole-injecting layer/) First hole-transporting Layer/Second hole-transporting Layer/ Fluorescent emitting layer (/Electron-transporting layer/electron-injecting Layer)
(n) (Hole-injecting layer/) First hole-transporting layer/Second hole-transporting layer/ Fluorescent emitting layer (/First electron-transporting layer/Second electron-transporting layer /Electron-injection layer)
(o) (Hole-injecting layer/) First hole-transporting layer /Second hole-transporting layer/Phosphorescent emitting layer (/Electron-transporting layer /Electron-injecting Layer)
(p) (Hole-injecting layer/) First hole-transporting layer/Second hole-transporting layer /Phosphorescent emitting layer (/First electron-transporting Layer/Second electron-transporting layer /Electron-injecting layer)
(q) (Hole-injecting layer/) Hole-transporting layer/Fluorescent emitting layer/Hole-blocking layer (/Electron-transporting layer/Electron-injecting layer)
(r) (Hole-injecting layer /) Hole-transporting layer/Phosphorescent emitting layer/ Hole-blocking layer (/ Electron-transport layer/ Electron-injecting layer)
(s) (Hole-injecting layer/) Hole-transporting layer/Fluorescent emitting layer /Exciton-blocking layer (/Electron-transporting layer/Electron-injecting layer)
(t) (Hole-injecting layer/) Hole-transporting layer/Phosphorescent emitting layer /Exciton-blocking layer (/Electron-transporting layer/Electron-injecting layer)

The layer structure of the organic EL device according to one aspect of the invention is not limited to the examples mentioned above.

For example, when the organic EL device has a hole-injecting layer and a hole-transporting layer, it is preferred that a hole-injecting layer be provided between the hole-transporting layer and the anode. Further, when the organic EL device has an electron-injecting layer and an electron-transporting layer, it is preferred that an electron-injecting layer be provided between the electron-transporting layer and the cathode. Further, each of the hole-injecting layer, the hole-transporting layer, the electron-transporting layer and the electron-injecting layer may be formed of a single layer or be formed of a plurality of layers.

The plurality of phosphorescent emitting layer, and the plurality of the phosphorescent emitting layer and the fluorescent emitting layer may be emitting layers that emit mutually different colors. For example, the emitting unit (f) may include a hole-transporting layer/first phosphorescent layer (red light emission)/ second phosphorescent emitting layer (green light emission)/spacing layer/fluorescent emitting layer (blue light emission)/electron-transporting layer.

An electron-blocking layer may be provided between each light emitting layer and the hole-transporting layer or the spacing layer. Further, a hole-blocking layer may be provided between each emitting layer and the electron-transporting layer. By providing the electron-blocking layer or the hole-blocking layer, it is possible to confine electrons or holes in the emitting layer, thereby to improve the recombination probability of carriers in the emitting layer, and to improve light emitting efficiency.

As a representative device configuration of a tandem type organic EL device, for example, a device configuration such as anode/first emitting unit/intermediate layer/second emitting unit/cathode can be given.
The first emitting unit and the second emitting unit are independently selected from the above-mentioned emitting units, for example.
The intermediate layer is also generally referred to as an intermediate electrode, an intermediate conductive layer, a charge generating layer, an electron withdrawing layer, a connecting layer, a connector layer, or an intermediate insulating layer. The intermediate layer is a layer that supplies electrons to the first emitting unit and holes to the second emitting unit, and can be formed from known materials.

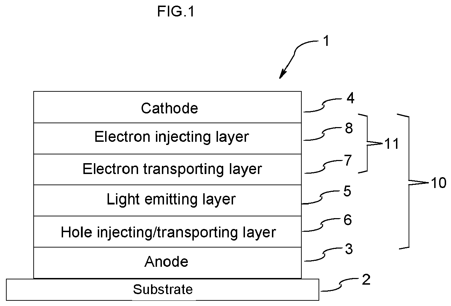

**FIG. 1** shows a schematic configuration of one example of the organic EL device of the invention. The organic EL device 1 comprises a substrate 2, an anode 3, a cathode 4 and an emitting unit 10 provided between the anode 3 and the cathode 4. The emitting unit 10 comprises an emitting layer 5 preferably comprising a host material and a dopant. A hole injecting and transporting layer 6 or the like may be provided between the emitting layer 5 and the anode 3 and an electron injecting layer 8 and an electron transporting layer 7 or the like (electron injecting and transporting unit 11) may be provided between the emitting layer 5 and the cathode 4. An electron-barrier layer may be provided on the anode 3 side of the emitting layer 5 and a hole-barrier layer may be provided on the cathode 4 side of the emitting layer 5. Due to such configuration, electrons or holes can be confined in the emitting layer 5, whereby possibility of generation of excitons in the emitting layer 5 can be improved.

**Hereinbelow, an explanation will be made on function, materials, etc. of each layer constituting the organic EL device described in the present specification.**

### (Substrate)

The substrate is used as a support of the organic EL device. The substrate preferably has a light transmittance of 50% or more in the visible light region with a wavelength of 400 to 700 nm, and a smooth substrate is preferable. Examples of the material of the substrate include soda-lime glass, aluminosilicate glass, quartz glass, plastic and the like. As a substrate, a flexible substrate can be used. The flexible substrate means a substrate that can be bent (flexible), and examples thereof include a plastic substrate and the like. Specific examples of the material for forming the plastic substrate include polycarbonate, polyallylate, polyether sulfone, polypropylene, polyester, polyvinyl fluoride, polyvinyl chloride, polyimide, polyethylene naphthalate and the like. Also, an inorganic vapor deposited film can be used.

### (Anode)

As the anode, for example, it is preferable to use a metal, an alloy, a conductive compound, a mixture thereof or the like and having a high work function (specifically, 4.0 eV or more). Specific examples of the material of the anode include indium oxide-tin oxide (ITO: Indium Tin Oxide), indium oxide-tin oxide containing silicon or silicon oxide, indium oxide-zinc oxide, indium oxide containing tungsten oxide or zinc oxide, graphene and the like. In addition, it is also possible to use gold, silver, platinum, nickel, tungsten, chromium, molybdenum, iron, cobalt, copper, palladium, titanium, and nitrides of these metals (e.g. titanium oxide).

The anode is normally formed by depositing these materials on the substrate by a sputtering method. For example, indium oxide-zinc oxide can be formed by a sputtering method by using a target in which 1 to 10 mass% zinc oxide is added relative to indium oxide. Further, indium oxide containing tungsten oxide or zinc oxide can be formed by a sputtering method by using a target in which 0.5 to 5 mass% of tungsten oxide or 0.1 to 1 mass% of zinc oxide is added relative to indium oxide.
As other methods for forming the anode, a vacuum deposition method, a coating method, an inkjet method, a spin coating method or the like can be given. When silver paste or the like is used, it is possible to use a coating method, an inkjet method or the like.

The hole-injecting layer formed in contact with the anode is formed by using a material that allows easy hole injection regardless of the work function of the anode. For this reason, in the anode, it is possible to use a common electrode material, e.g. a metal, an alloy, a conductive compound and a mixture thereof. Specifically, a material having a small work function such as alkaline metals such as lithium and cesium; alkaline earth metals such as calcium and strontium; alloys containing these metals (for example, magnesium-silver and aluminum-lithium); rare earth metals such as europium and ytterbium; and an alloy containing rare earth metals.

### (Hole-transporting layer) / (Hole-injecting layer)

The hole-transporting layer is an organic layer that is formed between the emitting layer and the anode, and has a function of transporting holes from the anode to the emitting layer. If the hole-transporting layer is composed of plural layers, an organic layer that is nearer to the anode may often be defined as the hole-injecting layer. The hole-injecting layer has a function of injecting holes efficiently to the organic layer unit from the anode. Said hole injection layer is generally used for stabilizing hole injection from anode to hole transporting layer which is generally consist of organic materials. Organic material having good contact with anode or organic material with p-type doping is preferably used for the hole injection layer.

p-doping usually consists of one or more p-dopant materials and one or more matrix materials. Matrix materials preferably have shallower HOMO level and p-dopant preferably have deeper LUMO level to enhance the carrier density of the layer. Specific examples for p-dopants are the below mentioned acceptor materials. Suitable matrix materials are the hole transport materials mentioned below, preferably aromatic or heterocyclic amine compounds.

Acceptor materials, or fused aromatic hydrocarbon materials or fused heterocycles which have high planarity, are preferably used as p-dopant materials for the hole injection layer. Specific examples for acceptor materials are, quinone compounds with one or more electron withdrawing groups, such as F₄TCNQ (2,3,5,6-tetrafluoro-7,7,8,8-tetracyanoquinodimethane), and 1,2,3-tris[(cyano)(4-cyano-2,3,5,6-tetrafluorophenyl)methylene]cyclopropane; hexa-azatriphenylene compounds with one or more electron withdrawing groups, such as hexa-azatriphenylene-hexanitrile; aromatic hydrocarbon compounds with one or more electron withdrawing groups; and aryl boron compounds with one or more electron withdrawing groups. Preferred p-dopants are quinone compounds with one or more electron withdrawing groups, such as F₄TCNQ, 1,2,3-Tris[(cyano)(4-cyano-2,3,5,6-tetrafluorophenyl)methylene]cyclopropane.

The ratio of the p-type dopant is preferably less than 20% of molar ratio, more preferably less than 10%, such as 1 %, 3%, or 5%, related to the matrix material.

The hole transporting layer is generally used for injecting and transporting holes efficiently, and aromatic or heterocyclic amine compounds are preferably used.

Specific examples for compounds for the hole transporting layer are represented by the general formula (H), 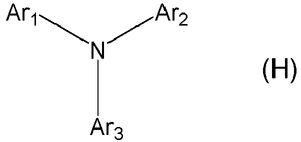 wherein
Ar₁ to Ar₃ each independently represents substituted or unsubstituted aryl group having 5 to 50 carbon atoms or substituted or unsubstituted heterocyclic group having 5 to 50 cyclic atoms, preferably phenyl group, biphenyl group, terphenyl group, naphthyl group, phenanthryl group, triphenylenyl group, fluorenyl group, spirobifluorenyl group, indenofluorenyl group, carbazolyl group, dibenzofuranyl group, dibenzothiophenyl group, carbazole substituted aryl group, dibenzofuran substituted aryl group or dibenzothiophene substituted aryl group; two or more substituents selected among Ar¹ to Ar³ may be bonded to each other to form a ring structure, such as a carbazole ring structure, or a acridane ring structure.

Preferably, at least one of Ar₁ to Ar₃ have additional one aryl or heterocyclic amine substituent, more preferably Ar₁ has an additional aryl amino substituent, at the case of that it is preferable that Ar₁ represents substituted or unsubstituted biphenylene group, substituted or unsubstituted fluorenylene group. Specific examples for the hole transport material are 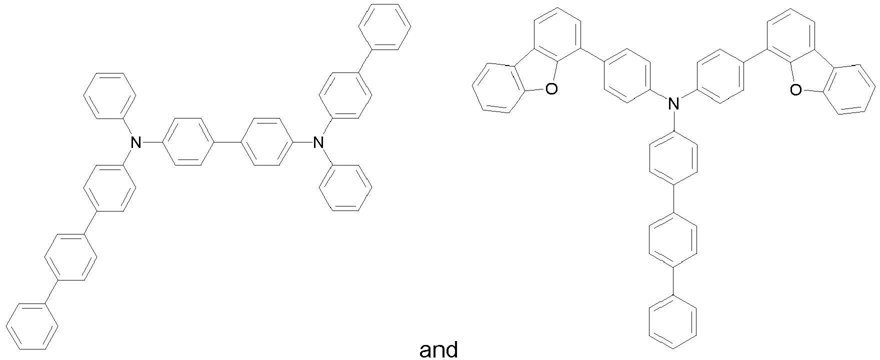 and the like.

A second hole transporting layer is preferably inserted between the first hole transporting layer and the emitting layer to enhance device performance by blocking excess electrons or excitons.
Specific examples for second hole transporting layer are the same as for the first hole transporting layer. It is preferred that second hole transporting layer has higher triplet energy to block triplet excitons, especially for phosphorescent devices, such as bicarbazole compounds, biphenylamine compounds, triphenylenyl amine compounds, fluorenyl amine compounds, carbazole substituted arylamine compounds, dibenzofuran substituted arylamine compounds, and dibenzothiophene substituted arylamine compounds.

### (Emitting layer)

The emitting layer is a layer containing a substance having a high emitting property (emitter material or dopant material). As the dopant material, various materials can be used. For example, a fluorescent emitting compound (fluorescent dopant), a phosphorescent emitting compound (phosphorescent dopant) or the like can be used. A fluorescent emitting compound is a compound capable of emitting light from the singlet excited state, and an emitting layer containing a fluorescent emitting compound is called a fluorescent emitting layer. Further, a phosphorescent emitting compound is a compound capable of emitting light from the triplet excited state, and an emitting layer containing a phosphorescent emitting compound is called a phosphorescent emitting layer.

Preferably, the emitting layer in the organic EL device of the present application comprises a compound of formula (I) as a dopant material.

The emitting layer preferably comprises at least one dopant material and at least one host material that allows it to emit light efficiently. In some literatures, a dopant material is called a guest material, an emitter or an emitting material. In some literatures, a host material is called a matrix material.
A single emitting layer may comprise plural dopant materials and plural host materials. Further, plural emitting layers may be present.

In the present specification, a host material combined with the fluorescent dopant is referred to as a "fluorescent host" and a host material combined with the phosphorescent dopant is referred to as the "phosphorescent host". Note that the fluorescent host and the phosphorescent host are not classified only by the molecular structure. The phosphorescent host is a material for forming a phosphorescent emitting layer containing a phosphorescent dopant, but does not mean that it cannot be used as a material for forming a fluorescent emitting layer. The same can be applied to the fluorescent host.

In one embodiment, it is preferred that the emitting layer comprises the compound represented by formula (I) according to the present invention (hereinafter, these compounds may be referred to as the "compound (I)"). More preferably, it is contained as a dopant material. Further, it is preferred that the compound (I) be contained in the emitting layer as a fluorescent dopant. Even further, it is preferred that the compound (I) be contained in the emitting layer as a blue fluorescent dopant.

In one embodiment, no specific restrictions are imposed on the content of the compound (I) as the dopant material in the emitting layer. In respect of sufficient emission and concentration quenching, the content is preferably 0.5 to 70 mass%, more preferably 0.8 to 30 mass%, further preferably 1 to 30 mass%, still further preferably 1 to 20 mass%, and particularly preferably 1 to 10 mass%, further particularly preferably 1 to 5 mass%, even further particularly preferably 2 to 4 mass%, related to the mass of the emitting layer.

### (Fluorescent dopant)

As a fluorescent dopant other than the compound (I), a fused polycyclic aromatic compound, a styrylamine compound, a fused ring amine compound, a boron-containing compound, a pyrrole compound, an indole compound, a carbazole compound can be given, for example. Among these, a fused ring amine compound, a boron-containing compound, carbazole compound is preferable.

As the fused ring amine compound, a diaminopyrene compound, a diaminochrysene compound, a diaminoanthracene compound, a diaminofluorene compound, a diaminofluorene compound with which one or more benzofuro skeletons are fused, or the like can be given.

As the boron-containing compound, a pyrromethene compound, a triphenylborane compound or the like can be given.

As a blue fluorescent dopant, pyrene compounds, styrylamine compounds, chrysene compounds, fluoranthene compounds, fluorene compounds, diamine compounds, triarylamine compounds and the like can be given, for example. Specifically, N,N'-bis[4-(9H-carbazol-9-yl)phenyl]-N,N'-diphenylstilbene-4,4'-diamine (abbreviation: YGA2S), 4-(9H-carbazol-9-yl)-4'-(10-phenyl-9-anthryl)triphenyamine (abbreviation: YGAPA), 4-(10-phenyl-9-anthryl)-4'-(9-phenyl-9H-carbazole-3-yl)triphenylamine (abbreviation: PCBAPA) or the like can be given.

As a green fluorescent dopant, an aromatic amine compound or the like can be given, for example. Specifically, N-(9,10-diphenyl-2-anthryl)-N,9-diphenyl-9H-carbazole-3-amine (abbreviation: 2PCAPA), N-[9,10-bis(1,1'-biphenyl-2-yl)-2-anthryl]-N,9-diphenyl-9H-carbazole-3-amine (abbreviation: 2PCABPhA), N-(9,10-diphenyl-2-anthryl)-N,N',N'-triphenyl-1,4-phenylenediamine (abbreviation: 2DPAPA), N-[9,10-bis(1,1'-biphenyl-2-yl)-2-anthryl]-N,N',N'-triphenyl-1,4-phenylenediamine (abbreviation: 2DPABPhA), N-[9,10-bis(1,1'-biphenyl-2-yl)]-N-[4-(9H-carbazole-9-yl)phenyl]-N-phenylanthracene-2-amine (abbreviation: 2YGABPhA), N,N,9-triphenylanthracene-9-amine (abbreviation: DPhAPhA) or the like can be given, for example.

As a red fluorescent dopant, a tetracene compound, a diamine compound or the like can be given. Specifically, N,N,N',N'-tetrakis(4-methylphenyl)tetracene-5,11-diamine (abbreviation: p-mPhTD), 7,14-diphenyl-N,N,N',N'-tetrakis(4-methylphenyl)acenaphtho[1,2-a]fluoranthene-3,10-diamine (abbreviation: p-mPhAFD) or the like can be given.

### (Phosphorescent dopant)

As a phosphorescent dopant, a phosphorescent emitting heavy metal complex and a phosphorescent emitting rare earth metal complex can be given.
As the heavy metal complex, an iridium complex, an osmium complex, a platinum complex or the like can be given. The heavy metal complex is for example an ortho-metalated complex of a metal selected from iridium, osmium and platinum.
Examples of rare earth metal complexes include terbium complexes, europium complexes and the like. Specifically, tris(acetylacetonate)(monophenanthroline)terbium(III) (abbreviation: Tb(acac)₃(Phen)), tris(1,3-diphenyl-1,3-propandionate)(monophenanthroline)europium(III)
(abbreviation: Eu(DBM)₃(Phen)), tris[1-(2-thenoyl)-3,3,3-trifluoroacetonate](monophenanthroline)europium(lll) (abbreviation: Eu(TTA)₃(Phen)) or the like can be given. These rare earth metal complexes are preferable as phosphorescent dopants since rare earth metal ions emit light due to electronic transition between different multiplicity.

As a blue phosphorescent dopant, an iridium complex, an osmium complex, a platinum complex, or the like can be given, for example. Specifically, bis[2-(4',6'-difluorophenyl)pyridinate-N,C2']iridium(III) tetrakis(1-pyrazolyl)borate (abbreviation: Flr6), bis[2-(4',6'-difluorophenyl) pyridinato-N,C2']iridium(III) picolinate (abbreviation: Ir(CF_{3ppy})₂(pic)), bis[2-(4',6'-difluorophenyl)pyridinato-N,C2']iridium(III) acetylacetonate (abbreviation: Flracac) or the like can be given.

As a green phosphorescent dopant, an iridium complex or the like can be given, for example. Specifically, tris(2-phenylpyridinato-N,C2') iridium(III) (abbreviation: Ir(ppy)₃), bis(1,2-diphenyl-1H-benzimidazolato)iridium(III) acetylacetonate (abbreviation: Ir(pbi)₂(acac)), bis(benzo[h]quinolinato)iridium(III) acetylacetonate (abbreviation: Ir(bzq)₂(acac)) or the like can be given.

As a red phosphorescent dopant, an iridium complex, a platinum complex, a terbium complex, an europium complex or the like can be given. Specifically, bis[2-(2'-benzo[4,5-α]thienyl)pyridinato-N,C3']iridium(III) acetylacetonate (abbreviation: Ir(btp)₂(acac)), bis(1-phenylisoquinolinato-N,C2')iridium(III) acetylacetonate (abbreviation: Ir(piq)₂(acac)), (acetylacetonato)bis[2,3-bis(4-fluorophenyl)quinoxalinato]iridium(III) (abbreviation: Ir(Fdpq)₂(acac)), 2,3,7,8,12,13,17,18-octaethyl-21H,23H-porphyrin platinum(II) (abbreviation PtOEP) or the like can be given.

As mentioned above, the emitting layer preferably comprises at least one compound (I) as a dopant.

### (Host material)

As host material, metal complexes such as aluminum complexes, beryllium complexes and zinc complexes; heterocyclic compounds such as indole compounds, pyridine compounds, pyrimidine compounds, triazine compounds, quinoline compounds, isoquinoline compounds, quinazoline compounds, dibenzofuran compounds, dibenzothiophene compounds, oxadiazole compounds, benzimidazole compounds, phenanthroline compounds; fused polyaromatic hydrocarbon (PAH) compounds such as a naphthalene compound, a triphenylene compound, a carbazole compound, an anthracene compound, a phenanthrene compound, a pyrene compound, a chrysene compound, a naphthacene compound, a fluoranthene compound; and aromatic amine compound such as triarylamine compounds and fused polycyclic aromatic amine compounds can be given, for example. Plural types of host materials can be used in combination.

As a **fluorescent** host, a compound having a higher singlet energy level than a fluorescent dopant is preferable. For example, a heterocyclic compound, a fused aromatic compound or the like can be given. As a fused aromatic compound, an anthracene compound, a pyrene compound, a chrysene compound, a naphthacene compound or the like are preferable. An anthracene compound is preferentially used as blue fluorescent host.

In the case that compound (I) is employed as at least one dopant material, preferred host materials are substituted or unsubstituted polyaromatic hydrocarbon (PAH) compounds, substituted or unsubstituted polyheteroaromatic compounds, substituted or unsubstituted anthracene compounds, or substituted or unsubstituted pyrene compounds, preferably substituted or unsubstituted anthracene compounds or substituted or unsubstituted pyrene compounds, more preferably substituted or unsubstituted anthracene compounds, most preferably anthracene compounds represented by formula (10), as mentioned above.

As a **phosphorescent host,** a compound having a higher triplet energy level as compared with a phosphorescent dopant is preferable. For example, a metal complex, a heterocyclic compound, a fused aromatic compound or the like can be given. Among these,
an indole compound, a carbazole compound, a pyridine compound, a pyrimidine compound, a triazine compound, a quinolone compound, an isoquinoline compound, a quinazoline compound, a dibenzofuran compound, a dibenzothiophene compound, a naphthalene compound, a triphenylene compound, a phenanthrene compound, a fluoranthene compound or the like can be given.

### (Electron-transporting layer) / (Electron-injecting layer)

The electron-transporting layer is an organic layer that is formed between the emitting layer and the cathode and has a function of transporting electrons from the cathode to the emitting layer. When the electron-transporting layer is formed of plural layers, an organic layer or an inorganic layer that is nearer to the cathode is often defined as the electron injecting layer (see for example layer 8 in FIG. 1, wherein an electron injecting layer 8 and an electron transporting layer 7 form an electron injecting and transporting unit 11). The electron injecting layer has a function of injecting electrons from the cathode efficiently to the organic layer unit. Preferred electron injection materials are alkali metal, alkali metal compounds, alkali metal complexes, the alkaline earth metal complexes and the rare earth metal complexes. According to one embodiment, it is preferred that the electron-transporting layer further comprises one or more layer(s) like a second electron-transporting layer, an electron injection layer to enhance efficiency and lifetime of the device, a hole blocking layer, an exciton blocking layer or a triplet blocking layer.

According to one embodiment, it is preferred that an electron-donating dopant be contained in the interfacial region between the cathode and the emitting unit. Due to such a configuration, the organic EL device can have an increased luminance or a long life. Here, the electron-donating dopant means one having a metal with a work function of 3.8 eV or less. As specific examples thereof, at least one selected from an alkali metal, an alkali metal complex, an alkali metal compound, an alkaline earth metal, an alkaline earth metal complex, an alkaline earth metal compound, a rare earth metal, a rare earth metal complex and a rare earth metal compound or the like can be mentioned.

As the alkali metal, Li (work function: 2.9 eV), Na (work function: 2.36 eV), K (work function: 2.28 eV), Rb (work function: 2.16 eV), Cs (work function: 1.95 eV) and the like can be given. One having a work function of 2.9 eV or less is particularly preferable. Among them, K, Rb and Cs are preferable. Rb or Cs is further preferable. Cs is most preferable. As the alkaline earth metal, Ca (work function: 2.9 eV), Sr (work function: 2.0 eV to 2.5 eV), Ba (work function: 2.52 eV) and the like can be given. One having a work function of 2.9 eV or less is particularly preferable. As the rare-earth metal, Sc, Y, Ce, Tb, Yb and the like can be given. One having a work function of 2.9 eV or less is particularly preferable.

Examples of the alkali metal compound include an alkali oxide such as Li₂O, Cs₂O or K₂O, and an alkali halide such as LiF, NaF, CsF and KF. Among them, LiF, Li₂O and NaF are preferable. Examples of the alkaline earth metal compound include BaO, SrO, CaO, and mixtures thereof such as BaₓSr₁₋ₓO (0<x<1) and BaₓCa₁₋ₓO (0<x<1). Among them, BaO, SrO and CaO are preferable. Examples of the rare earth metal compound include YbF₃, ScF₃, ScO₃, Y₂O₃, Ce₂O₃, GdF₃ and TbF₃. Among these, YbF₃, SCF₃ and TbF₃ are preferable.

The alkali metal complexes, the alkaline earth metal complexes and the rare earth metal complexes are not particularly limited as long as they contain, as a metal ion, at least one of alkali metal ions, alkaline earth metal ions, and rare earth metal ions. Meanwhile, preferred examples of the ligand include, but are not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzimidazole, hydroxybenzotriazole, hydroxyfluborane, bipyridyl, phenanthroline, phthalocyanine, porphyrin, cyclopentadiene, β-diketones, and azomethines.

Regarding the addition form of the electron-donating dopant, it is preferred that the electron-donating dopant be formed in a shape of a layer or an island in the interfacial region. A preferred method for the formation is a method in which an organic compound (a light emitting material or an electron-injecting material) for forming the interfacial region is deposited simultaneously with deposition of the electron-donating dopant by a resistant heating deposition method, thereby dispersing the electron-donating dopant in the organic compound.
In a case where the electron-donating dopant is formed into the shape of a layer, the light-emitting material or electron-injecting material which serves as an organic layer in the interface is formed into the shape of a layer. After that, a reductive dopant is solely deposited by the resistant heating deposition method to form a layer preferably having a thickness of from 0.1 nm to 15 nm. In a case where the electron-donating dopant is formed into the shape of an island, the emitting material or the electron-injecting material which serves as an organic layer in the interface is formed into the shape of an island. After that, the electron-donating dopant is solely deposited by the resistant heating deposition method to form an island preferably having a thickness of from 0.05 nm to 1 nm. As the electron-transporting material used in the electron-transporting layer other than a compound of the formula (I), an aromatic heterocyclic compound having one or more hetero atoms in the molecule may preferably be used. In particular, a nitrogen-containing heterocyclic compound is preferable.

According to one embodiment, it is preferable that the electron-transporting layer comprises a nitrogen-containing heterocyclic metal chelate.

According to the other embodiment, it is preferable that the electron-transporting layer comprises a substituted or unsubstituted nitrogen containing heterocyclic compound. Specific examples of preferred heterocyclic compounds for the electron-transporting layer are, 6-membered azine compounds; such as pyridine compounds, pyrimidine compounds, triazine compounds, pyrazine compounds, preferably pyrimidine compounds or triazine compounds; 6-membered fused azine compounds, such as quinolone compounds, isoquinoline compounds, quinoxaline compounds, quinazoline compounds, phenanthroline compounds, benzoquinoline compounds, benzoisoquinoline compounds, dibenzoquinoxaline compounds, preferably quinolone compounds, isoquinoline compounds, phenanthroline compounds; 5-membered heterocyclic compounds, such as imidazole compounds, oxazole compounds, oxadiazole compounds, triazole compounds, thiazole compounds, thiadiazole compounds; fused imidazole compounds, such as benzimidazole compounds, imidazopyridine compounds, naphthoimidazole compounds, benzimidazophenanthridine compounds, benzimidzobenzimidazole compounds, preferably benzimidazole compounds, imidazopyridine compounds or benzimidazophenanthridine compounds.

According to another embodiment, it is preferable the electron-transporting layer comprises a phosphine oxide compound represented as Arₚ₁Arₚ₂Ar_{P3}P=O.
Arₚ₁ to Arₚ₃ are the substituents of phosphor atom and each independently represent substituted or unsubstituted above mentioned aryl group or substituted or unsubstituted above mentioned heterocyclic group.

According to another embodiment, it is preferable that the electron-transporting layer comprises aromatic hydrocarbon compounds. Specific examples of preferred aromatic hydrocarbon compounds for the electron-transporting layer are, oligo-phenylene compounds, naphthalene compounds, fluorene compounds, fluoranthenyl group, anthracene compounds, phenanthrene compounds, pyrene compounds, triphenylene compounds, benzanthracene compounds, chrysene compounds, benzphenanthrene compounds, naphthacene compounds, and benzochrysene compounds, preferably anthracene compounds, pyrene compounds and fluoranthene compounds.

### (Cathode)

For the cathode, a metal, an alloy, an electrically conductive compound, and a mixture thereof, each having a small work function (specifically, a work function of 3.8 eV or less) are preferably used. Specific examples of a material for the cathode include an alkali metal such as lithium and cesium; an alkaline earth metal such as magnesium, calcium, and strontium; aluminum, an alloy containing these metals (for example, magnesium-silver, aluminum-lithium); a rare earth metal such as europium and ytterbium; and an alloy containing a rare earth metal.

The cathode is usually formed by a vacuum vapor deposition or a sputtering method. Further, in the case of using a silver paste or the like, a coating method, an inkjet method, or the like can be employed.

Moreover, various electrically conductive materials such as silver, ITO, graphene, indium oxide-tin oxide containing silicon or silicon oxide, selected independently from the work function, can be used to form a cathode. These electrically conductive materials are made into films using a sputtering method, an inkjet method, a spin coating method, or the like.

### (Insulating layer)

In the organic EL device, pixel defects based on leakage or a short circuit are easily generated since an electric field is applied to a thin film. In order to prevent this, it is preferred to insert an insulating thin layer between a pair of electrodes. Examples of materials used in the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. A mixture thereof may be used in the insulating layer, and a laminate of a plurality of layers that include these materials can be also used for the insulating layer.

### (Spacing layer)

A spacing layer is a layer provided between a fluorescent emitting layer and a phosphorescent emitting layer when a fluorescent emitting layer and a phosphorescent emitting layer are stacked in order to prevent diffusion of excitons generated in the phosphorescent emitting layer to the fluorescent emitting layer or in order to adjust the carrier balance. Further, the spacing layer can be provided between the plural phosphorescent emitting layers.

Since the spacing layer is provided between the emitting layers, the material used for the spacing layer is preferably a material having both electron-transporting capability and hole-transporting capability. In order to prevent diffusion of the triplet energy in adjacent phosphorescent emitting layers, it is preferred that the spacing layer have a triplet energy of 2.6 eV or more. As the material used for the spacing layer, the same materials as those used in the above-mentioned hole-transporting layer can be given.

### (Electron-blocking layer, hole-blocking layer, exciton-blocking layer)

An electron-blocking layer, a hole-blocking layer, an exciton (triplet)-blocking layer, and the like may be provided in adjacent to the emitting layer.

The electron-blocking layer has a function of preventing leakage of electrons from the emitting layer to the hole-transporting layer. The hole-blocking layer has a function of preventing leakage of holes from the emitting layer to the electron-transporting layer. In order to improve hole blocking capability, a material having a deep HOMO level is preferably used. The exciton-blocking layer has a function of preventing diffusion of excitons generated in the emitting layer to the adjacent layers and confining the excitons within the emitting layer. In order to improve triplet block capability, a material having a high triplet level is preferably used.

### (Method for forming a layer)

The method for forming each layer of the organic EL device of the invention is not particularly limited unless otherwise specified. A known film-forming method such as a dry film-forming method, a wet film-forming method or the like can be used. Specific examples of the dry film-forming method include a vacuum deposition method, a sputtering method, a plasma method, an ion plating method, and the like. Specific examples of the wet film-forming method include various coating methods such as a spin coating method, a dipping method, a flow coating method, an inkjet method, and the like.

### (Film thickness)

The film thickness of each layer of the organic EL device of the invention is not particularly limited unless otherwise specified. If the film thickness is too small, defects such as pinholes are likely to occur to make it difficult to obtain a sufficient luminance. If the film thickness is too large, a high driving voltage is required to be applied, leading to a lowering in efficiency. In this respect, the film thickness is preferably 0.1 nm to 10 µm, and more preferably 5 nm to 0.2 µm.

### (Electronic apparatus (electronic equipment))

The present invention further relates to an electronic equipment (electronic apparatus) comprising the organic electroluminescence device according to the present application. Examples of the electronic apparatus include display parts such as an organic EL panel module; display devices of television sets, mobile phones, smart phones, and personal computer, and the like; and emitting devices of a lighting device and a vehicle lighting device.

### EXAMPLES

Next, the invention will be explained in more detail in accordance with the following synthesis examples, examples, and comparative examples, which should not be construed as limiting the scope of the invention.

The percentages and ratios mentioned in the examples below - unless stated otherwise - are % by weight and weight ratios.

### I Synthesis Examples

All experiments are carried out in protective gas atmosphere.

### Compound 1

### Step 1

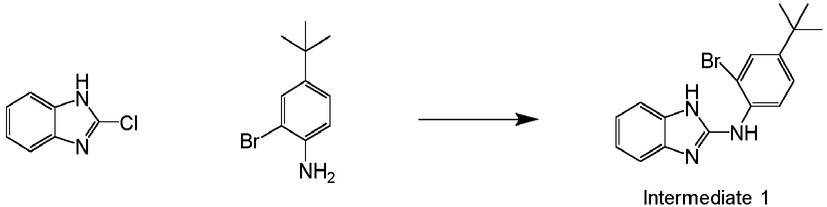

To 15.3 g (0.100 mol) of 2-chlorbenzimidazole, 25.1 g (0.110 mol) of 2-bromo-4-tert-butylaniline in 100 ml of N-methyl-2-pyrrolidone were added. 10.6 g (0.110 mol) of methane sulfonic acid were added and the reaction mixture was stirred at 100 °C for 18 hours under nitrogen. The reaction mixture was diluted with ethyl acetate and neutralized with a sodium hydrogen carbonate solution. The ethyl acetate phase was washed 2 times with water and was dried with magnesium sulfate. The solvent was removed in vacuum.
The product was refluxed in 100 ml c-hexane to give 31.7 g (92.5% yield) of Intermediate 1, after filtration.
**¹H NMR** (400 MHz, CDCl₃) δ : 7.93 (d, 1H), 7.55 (d, 1H), 7.38 (m, 2H), 7.31 (dd, 1H), 7.16 (m, 2H), 6.00-3.50 (broad signal, 2H), 1.28 (s, 9H).

### Step 2

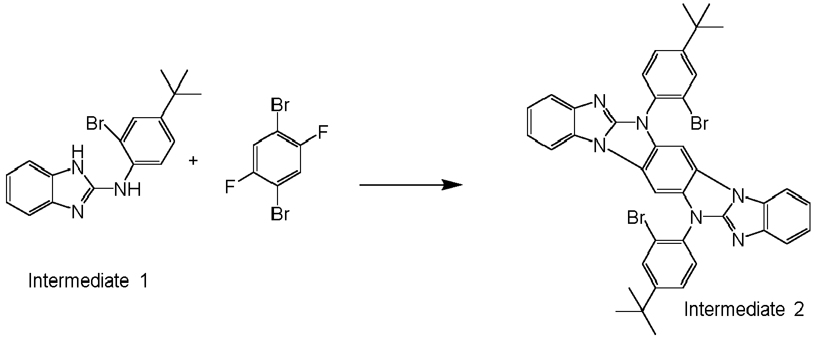

To 15.1 g (44.0 mmol) of Intermediate 1 and 5.44 g (20.0 mmol) of 1,4-di-bromo-2,5-di-fluoro benzene in abs. dimethylformamide, 93.4 g (440 mmol) potassium phosphate tribasic were added. The reaction mixture was stirred at 130 °C for 4 hours and at 160 °C for 16 hours under nitrogen.
The reaction mixture was poured on water and the product was filtered off.
The product was first refluxed in methanol, filtered off and then refluxed in methyl ethyl ketone to give after filtration 7.83 g (52% yield) of Intermediate 2.
**¹H NMR** (400 MHz, TFA-d₁) δ : 8.29 (s, 2H), 8.25 (d, 2H), 8.13 (m, 2H), 7.98 (m, 4H), 7.88 (m, 2H), 7.79 (m, 4H), 1.63 (s, 18H).

### Step 3

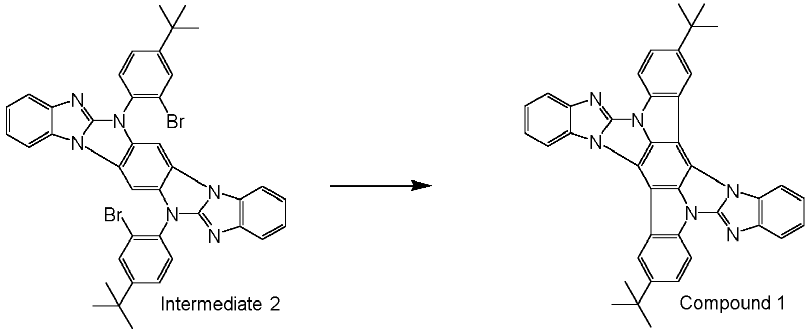

3.00 g (3.95 mmol) of the Intermediate 2, 2.73 g (19.8 mmol) of potassium carbonate in 300 ml abs. dimethylformamide were degassed with argon. 12 mg (0.050 mmol) of palladium acetate and 42 mg (0.10 mmol) of 1,3-bis(2,6-diisopropylphenyl) imidazolium chloride were added and the reaction mixture was degassed with argon. The reaction mixture was stirred at 160 °C for 23 hours under argon. 5 ml of a 1% solution of sodium cyanide in water were added and the reaction mixture was stirred at 100 °C for 1 hour. The reaction mixture was poured on methanol and the product was filtered off. The product was refluxed in N,N-dimethylacetamide and was washed with water and methanol to give 1.70 g (72% yield) of Compound 1 as yellow solid.
**¹H NMR** (400 MHz, TFA-d₁) δ : 9.05 (d, 2H), 8.88 (d, 2H), 8.46 (d, 2H), 8.20 (d, 2H), 8.13 (d, 2H), 8.04 (m, 2H), 7.97 (m, 2H), 1.76 (s, 18H).

### Synthesis Comparative compound 1

### Step 1

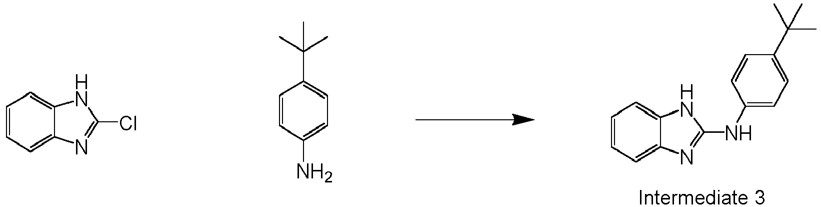

To 10.0 g (70.0 mmol) of 2-chlorbenzimidazole, 11.5 g (77.0 mmol) of 2-bromo-4-tert-butylaniline in 100 ml of N-methyl-2-pyrrolidone were added. 10.6 g (0.110 mmol) of methane sulfonic acid were added and the reaction mixture was stirred at 100 °C for 4 hours under nitrogen.

The reaction mixture was poured on water and was neutralized with sodium hydrogen carbonate. The product was filtered off and was washed with water. The product was refluxed in heptane to give 18.4 g (99% yield) of Intermediate 3, after filtration.
**¹H NMR** (400 MHz, DMSO-d₆) δ : 7.51 (m, 2H), 7.44 (m, 2H), 7.37 (m, 2H), 7.15 (m, 2H), 1.30 (s, 9H). NH protons not visible
**¹H NMR** (400 MHz, CDCl₃) δ : 7.80 (s, 3H), 7.25 (m, 2H), 7.08 (m, 5H), 1.30 (s, 9H).

### Step 2

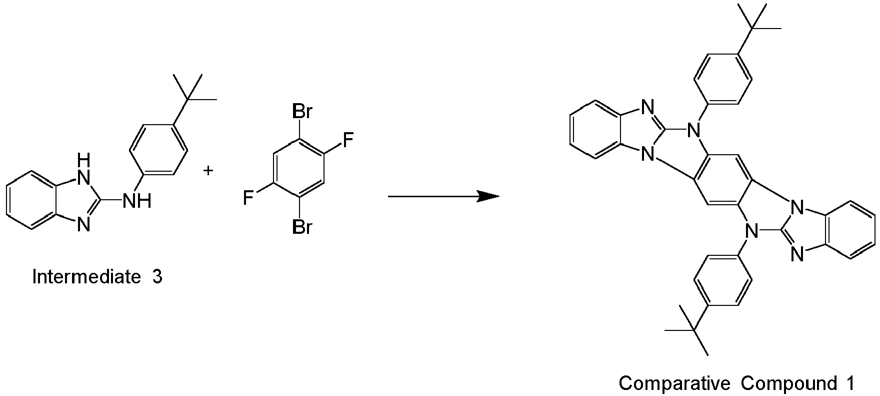

To 12.8 g (48.4 mmol) of Intermediate 3 and 5.98 g (22.0 mmol) of 1,4-di-bromo 2,5-di-fluoro benzene in abs. dimethylformamide, 46.7 g (220 mmol) of potassium phosphate tribasic were added. The reaction mixture was stirred at 130 °C for 3 hours and at 150 °C for 18 hours under nitrogen. 14.0 g (6.60 mmol) of potassium phosphate tribasic were added and the reaction mixture was stirred for 5 h at 150 °C under nitrogen.
The reaction mixture was poured on water and the product was filtered off. The product was washed with ethanol and acetone.
The product was refluxed in N,N-dimethylacetamide. The product was filtered off and was washed with ethanol and acetone to give 9.90 g (74% yield) of Comparative compound 1 as white solid.
**¹H NMR** (400 MHz, TFA-d₁) δ : 8.46 (s, 2H), 8.14 (m, 2H), 8.04 (m, 4H), 7.87 (m, 6H), 7.78 (m, 4H), 1.61 (s, 18H).

### II Evaluation of Compounds

Next, the properties of the compounds used in the examples were measured. Measurement and calculation methods are shown below.

### 1.1 Device Application Data (invented compound as emitter dopant)

### Preparation and Evaluation of Organic EL Devices

The organic EL devices were prepared and evaluated as follows:

### Application Example 1

A glass substrate with 130 nm-thick indium-tin-oxide (ITO) transparent electrode (manufactured by Geomatec Co., Ltd.) used as an anode was first treated with N2 plasma for 100 sec. This treatment also improved the hole injection properties of the ITO. The cleaned substrate was mounted on a substrate holder and loaded into a vacuum chamber. Thereafter, the organic materials specified below were applied by vapor deposition to the ITO substrate at a rate of approx. 0.2-1 Å/sec at about 10⁻⁶-10⁻⁸ mbar. As a hole injection layer, 10 nm-thick mixture of Compound HT-1 and 3% by weight of compound HI were applied. Then 80 nm-thick of Compound HT1 and 10 nm of Compound HT-2 were applied as hole transporting layer 1 and hole transporting layer 2, respectively. Subsequently, a mixture of 2% by weight of an emitter **Compound 1** and 98% by weight of host Compound BH-1 were applied to form a 25 nm-thick fluorescent-emitting layer. On the emitting layer, 10 nm-thick Compound ET-1 was applied as an electron transporting layer 1 and 15 nm of Compound ET-2 as an electron transporting layer 2. Finally, 1 nm-thick LiF was deposited as an electron injection layer and 80 nm-thick Al was then deposited as a cathode to complete the device. The device was sealed with a glass lid and a getter in an inert nitrogen atmosphere with less than 1 ppm of water and oxygen. To characterize the OLED, electroluminescence (EL) spectra were recorded at various currents and voltages. EL peak maximum and Full Width at Half Maximum (FWHM) were recorded at 10 mA/cm². In addition, the current-voltage characteristic were measured in combination with the luminance to determine luminous efficiency and external quantum efficiency (EQE). Driving voltage (Voltage) was given at a current density of 10 mA/cm². Lifetime of OLED device was measured as a decay of the luminance at constant current density of 50 mA/cm² to 95% of its initial value. The device results are shown in Table 1.

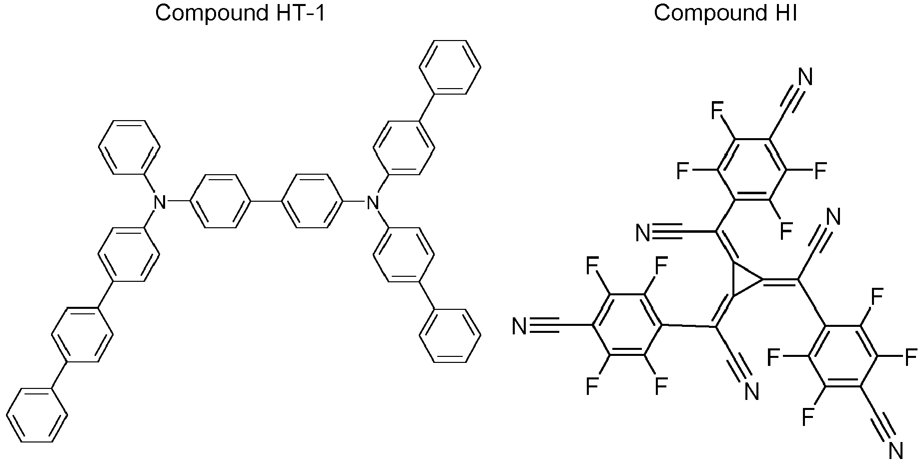

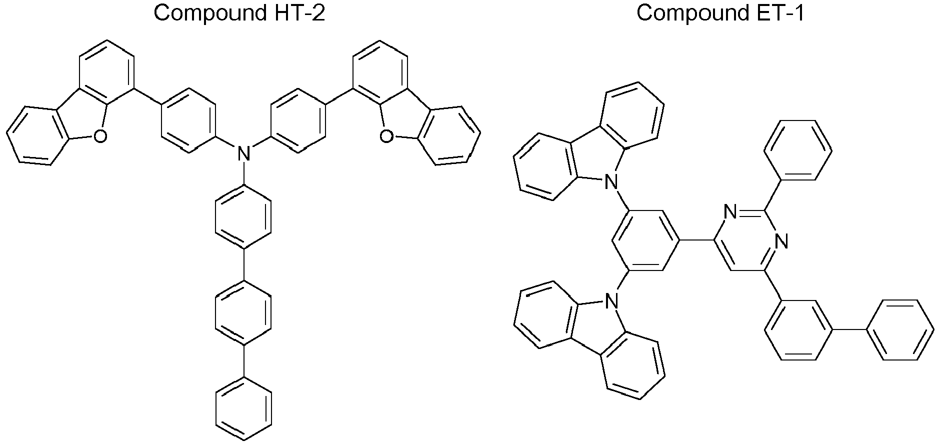

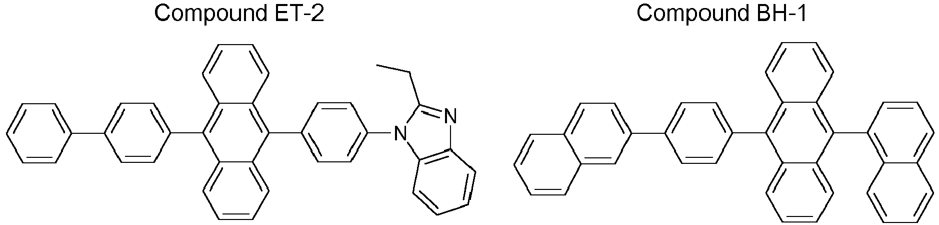

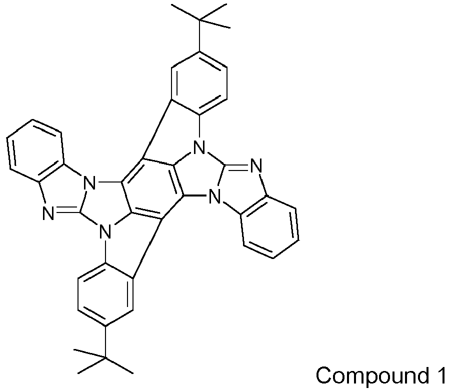

### Comparative application Example 1

Application Example 1 was repeated except for using **Comparative compound 1** instead of the Compound 1.

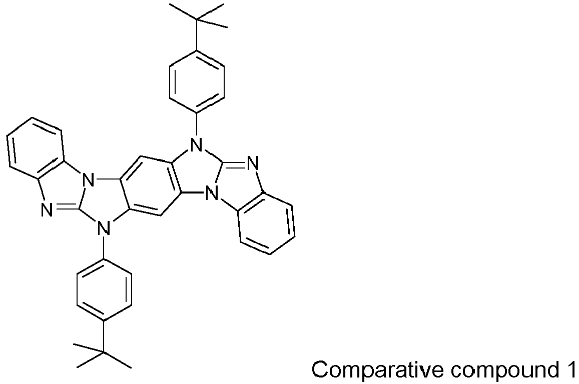

The device results are shown in Table 1.

**Table 1**

| Appl. Ex. | Voltage, V | EQE, % | LT95 at 50 mA/cm², h | EL max, nm | FWHM, nm |
|---|---|---|---|---|---|
| Appl. Ex. 1 | 3.63 | 7.82 | 45 | 450 | 30 |
| Comp. Appl. Ex. 1 | 3.64 | 6.29 | 28 | 443 | 54 |

These results demonstrated that the Compound 1 gives better EQE, lifetime and a more narrow spectrum (smaller FWHM), i.e. better color purity than Comparative Compound 1 when used as fluorescent emitting material in OLED devices.

## Claims

1. A heterocyclic compound represented by formula (I): 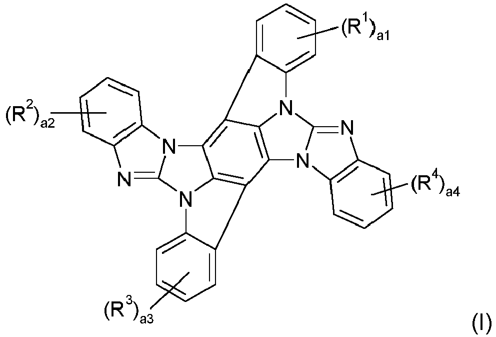 wherein
R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; a cycloalkyl group having from ring 3 to 20 carbon atoms which is unsubstituted or substituted; an alkenyl group having from 2 to 20 carbon atoms which is unsubstituted or substituted; an alkynyl group having from 2 to 20 carbon atoms which is unsubstituted or substituted; CN; N(R⁵)₂; OR⁶; SR⁷; Si(R⁸)₃; or halogen;
or
two adjacent residues R¹, R², R³ and/or R⁴ together form a ring structure which is unsubstituted or substituted;
R⁵, R⁶, R⁷ and R⁸ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; or a cycloalkyl group having from 3 to 20 ring carbon atoms which is unsubstituted or substituted;
a1, a2, a3 and a4 each independently represents 0, 1, 2, 3 or 4;
when a1, a2, a3 and a4 are 2 to 4, each of the residues R¹, R², R³ as well as R⁴ are allowed to be the same or different.

2. The heterocyclic compound according to claim 1, wherein
R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; a cycloalkyl group having from 3 to 20 ring carbon atoms which is unsubstituted or substituted; an alkenyl group having from 2 to 20 carbon atoms which is unsubstituted or substituted; an alkynyl group having from 2 to 20 carbon atoms which is unsubstituted or substituted; CN; N(R⁵)₂; OR⁶; SR⁷; Si(R⁸)₃; or halogen.

3. The heterocyclic compound according to claim 2, wherein
R¹, R², R³ and R⁴ each independently represents an aryl group having from 6 to 18 ring carbon atoms which is unsubstituted or substituted; a heteroaryl group having from 5 to 18 ring atoms which is unsubstituted or substituted; an alkyl group having from 1 to 20 carbon atoms which is unsubstituted or substituted; CN; N(R⁵)₂, Si(R⁸)₃; or halogen; or a cycloalkyl group having from 3 to 20 ring carbon atoms which is unsubstituted or substituted.

4. The heterocyclic compound according to claim 3, wherein
R¹, R², R³ and R⁴ each independently represents an alkyl group having 1 to 4 carbon atoms which is unsubstituted or substituted; an aryl group having 6 to 13 ring carbon atoms is unsubstituted or substituted; a heteroaryl group having from 5 to 13 ring atoms which is unsubstituted or substituted.

5. The heterocyclic compound according to any one of claims 1 to 4, wherein the sum of a1, a2, a3 and a4 is at least 1, preferably at least 2.

6. The compound according to claim 5, wherein
a1 and a3 each independently represents 1; and
a2 and a4 each independently represents 0, 1 or 2.

7. A material, preferably an emitter material, for an organic electroluminescence device, comprising at least one compound according to any one of claims 1 to 6.

8. An organic electroluminescence device comprising at least one compound according to any one of claims 1 to 6.

9. The organic electroluminescence device according to claim 8, comprising a cathode, an anode and one or more organic thin film layers comprising an emitting layer disposed between the cathode and the anode, wherein at least one layer of the organic thin film layers comprises at least one compound according to any one of claims 1 to 6.

10. The organic electroluminescence device according to claim 9, wherein the light emitting layer comprises at least one compound according to any one of claims 1 to 6.

11. The organic electroluminescence device according to claim 10, wherein the light emitting layer comprises at least one host and at least one dopant, wherein the dopant comprises at least one compound according to any one of claims 1 to 6.

12. The organic electroluminescence device according to claim 11, wherein the host comprises at least one substituted or unsubstituted fused aromatic hydrocarbon compound and/or at least one substituted or unsubstituted anthracene compound.

13. An electronic equipment comprising the organic electroluminescence device according to any one of claims 8 to 12.

14. A light emitting layer comprising at least one host and at least one dopant, wherein the dopant comprises at least one compound according to any one of claims 1 to 6.

15. Use of a compound of formula (I) according to any one of claims 1 to 6 in an organic electroluminescence device.
